# EUROPEAN PATENT SPECIFICATION

(11) **EP 2 402 327 B1**
(45) Date of publication and mention of the grant of the patent: **07.03.2018**
(21) Application number: 10172616.4
(22) Date of filing: 12.08.2010
(51) Int. Cl.: C07D 277/54, C07D 417/12, C07D 417/14, C07D 513/04, A61K 31/426, A61P 3/00

(54) **Acetamide compounds as glucokinase activators, their process and medicinal applications**
Acetamidverbindungen als Glucokinase-Aktivatoren, Verfahren derselben und medizinische Anwendungen
Composés d'acétamide en tant qu'activateurs de la glucokinase, leur procédé et applications médicales

(30) Priority: 29.06.2010 IN CH18372010
(43) Date of publication of application: 04.01.2012
(73) Proprietor: Impetis Biosciences Ltd., Mumbai, Maharashtra 400001 (IN)
(72) Inventor: Deshpande, Anil, 411057, Pune (IN); Dave, Bhavesh, 411057, Pune (IN); Kurhade, Santosh, 411057, Pune (IN); Kobal, Balasaheb, 411057, Pune (IN); Naik, Keshav, 411057, Pune (IN); Kandalkar, Sachin, 411057, Pune (IN); Bhuniya, Debnath, 411057, Pune (IN); Palle, Venkata Poornapragnacharyulu, 411057, Pure (IN)
(74) Representative: Vossius & Partner Patentanwälte Rechtsanwälte mbB

(56) References cited:
- WO-A1-03/055482
- WO-A2-2008/104994
- WO-A2-2009/047798

## Description

### Field of the invention

This disclosure relates to a series of acetamide derivatives, their stereoisomers, tautomers, pharmaceutically acceptable salts, polymorphs, solvates and formulations thereof. The disclosure also relates to the process of preparation of acetamide derivatives along with their glucokinase activating effects, which are beneficial for the prophylaxis, management, treatment, control of progression, or adjunct treatment of diseases and/or medical conditions where the activation of glucokinase would be beneficial, such as diabetes, metabolic syndrome, and/or diabetes-related complications including retinopathy, nephropathy, neuropathy, ischemic heart disease, arteriosclerosis, β-cell dysfunction, and as therapeutic and/or prophylactic agents for obesity.

It also relates to compounds with liver selective Glucokinase activation, useful for the treatment of hyperglycemia, diabetes, obesity, dyslipidemia, metabolic syndrome and the like, in mammals and have minimum hypoglycemic potential.

### Background

Diabetes mellitus is a metabolic disorder characterized by recurrent or persistent hyperglycemia (high blood glucose) and other signs, as distinct from a single disease or condition. Glucose level abnormalities can result in serious long-term complications, which include cardiovascular disease, chronic renal failure, retinal damage, nerve damage (of several kinds), microvascular damage and obesity.

Type 1 diabetes, also known as Insulin Dependent Diabetes Mellitus (IDDM), is characterized by loss of the insulin-producing β-cells of the islets of Langerhans of the pancreas leading to a deficiency of insulin. Type-2 diabetes previously known as adult-onset diabetes, maturity-onset diabetes, or Non-Insulin Dependent Diabetes Mellitus (NIDDM) - is due to a combination of increased hepatic glucose output, defective insulin secretion, and insulin resistance or reduced insulin sensitivity (defective responsiveness of tissues to insulin).

Chronic elevation of blood glucose level leads to damage of blood vessels. In diabetes, the resultant problems are grouped under "microvascular disease" (due to damage of small blood vessels) and "macrovascular disease" (due to damage of the arteries). Examples of microvascular disease include diabetic retinopathy, neuropathy and nephropathy, while examples of macrovascular disease include coronary artery disease, stroke, peripheral vascular disease, and diabetic myonecrosis.

Diabetic retinopathy, characterized by the growth of weakened blood vessels in the retina as well as macular edema (swelling of the macula), can lead to severe vision loss or blindness. Retinal damage (from microangiopathy) makes it the most common cause of blindness among non-elderly adults in the US. Diabetic neuropathy is characterized by compromised nerve function in the lower extremities. When combined with damaged blood vessels, diabetic neuropathy can lead to diabetic foot. Other forms of diabetic neuropathy may present as mononeuritis or autonomic neuropathy. Diabetic nephropathy is characterized by damage to the kidney, which can lead to chronic renal failure, eventually requiring dialysis. Diabetes mellitus is the most common cause of adult kidney failure worldwide. A high glycemic diet (i.e., a diet that consists of meals that give high postprandial blood sugar) is known to be one of the causative factors contributing to the development of obesity.

Glucokinase (GK), also known as hexokinase IV or D, is one of four glucose-phosphorylating enzymes called hexokinases that catalyze the first step of glycolysis, the conversion of glucose to glucose 6-phosphate (G6P), in vertebrate tissues. GK functions in a dual role, with distinct functions in the pancreas and liver; (a) as a molecular glucose sensor in the insulin-producing pancreatic β-cells, and (b) as the high-capacity enzymatic step initiating the storage of glucose in the form of glycogen in the liver and uptake of glucose during hyperglycemia. Therefore, GK plays a central role in glucose homeostasis, through the phosphorylation of glucose in the liver, and the modulation of insulin secretion in the pancreas (Postic, C. et al (1999) J. Biol. Chem. 274: 305-315). GK also functions as a sensor in other neuroendocrine cells of the gastrointestinal tract and in various brain cells including specific cells in the hypothalamus (Jetton, T. A. et al (1994) J. Biol. Chem. 269: 3641-3654).

The physiological concentration of glucose in human plasma is approximately 5.5 mM under fasting conditions, and increases to about 12 mM in the fed state. This concentration is dependent on and maintained by the activity of GK, which senses glucose and controls metabolic flux in key cell types. The glucose concentration, at which GK activity is at half of its maximal velocity or Vₘₐₓ, is defined as its S_{0.5}. The S_{0.5} of GK for glucose lies in the middle of the physiological glucose concentration range at approximately 8 mM, allowing this enzyme to act as a molecular glucose sensor crucial for glucose homeostasis. The limited tissue distribution and unique kinetic properties of GK allow it to play a critical role in pancreatic β-cell insulin secretion and hepatic glucose utilization. GK differs from the other members of the mammalian hexokinase family in its unique sigmoidal kinetics with respect to glucose, a high S_{0.5} that lies in the physiological glucose concentration range (the other three mammalian hexokinases have S_{0.5} values less than 0.5 mM), the lack of product inhibition by G6P, and its tissue distribution in cell types that are thought to be responsive to changing plasma glucose levels.

Tissue-specific differences have been observed between the regulation of GK in the liver and the pancreas. In the liver, GK is allosterically inhibited by the glucokinase regulatory protein (GKRP), which results in its sequestration in the nucleus and subsequent protection from proteolytic degradation. This inhibition is reversed by high concentrations of glucose and by fructose 1-phosphate, and is potentiated by fructose 6-phosphate. In the pancreatic β-cells, GK expression is believed to be constitutive. GK is also known to be expressed in the hypothalamus, where it may exert effects on feeding behavior, and in the intestine K and L cells, where it may contribute to the secretion of enteroincretins such as glucagon-like peptide-1 (GLP-1), glucose dependent insulinotropic peptide (GIP) (Matschinsky F. M. et al (2006) Diabetes 55: 1-12; Theodorakis M. J. et al (2006) Am. J. Physiol. Endocrinol. Metab. 290: E550-E559). Given the role of GK as a molecular glucose sensor, it is not surprising that GK mutations have a profound influence on glucose homeostasis. About 2000 GK mutations that have been identified in humans result in impaired glucose-mediated insulin secretion and maturity-onset diabetes of the young type 2 (MODY-2). Some of these mutations result in decreased accumulation of hepatic glycogen, while others decrease GK activity by reducing the stability of the enzyme or by decreasing its Vₘₐₓ. Mutations that result in activation of GK are implicated in the onset of persistent hyperinsulinemic hypoglycemia of infancy (PHHI). Single point mutations (e.g. V62M, D158A, Y214A, V455M, and F456V) in regions distinct from the substrate binding site of the enzyme lead to modulation of GK activity (Glaser, B. et al (1998) N. Engl. J. Med. 338: 226-230; Gloyn, A. L. (2003) Hum. Mutat. 22: 353-362; Gloyn, A. L. et al (2003) Diabetes 52: 2433-2440). These observations highlight that GK activity can be regulated through allosteric modulation.

Homozygous knock out of GK in mice results in severe diabetes and death, while heterozygous disruption results in a milder diabetic phenotype, decreased hepatic glucose uptake and impaired insulin secretion in response to glucose. Conversely, over-expression of GK in fat-induced diabetic as well as non-diabetic mice results in improved glucose tolerance. Transgenic mice over-expressing GK in the liver show a modest (20%) increase in fasting GK activity, which correlates with lower fasting plasma glucose and insulin, and improved glucose tolerance (Hariharan, N. et al (1997) Diabetes 46: 11-16).

The enzymatic properties of GK can be described in terms of its velocity (i.e. its rate of converting glucose to G6P) and its S_{0.5} for glucose (i.e. the apparent glucose concentration at which GK converts glucose to G6P at half of its maximal velocity). The S_{0.5} of human GK for glucose is approximately 8mM in enzyme based assay. GKAs induce increased conversion by GK of glucose to G6P by either decreasing the S_{0.5} of GK for glucose, increasing its Vₘₐₓ, or by a combination of both, and can potentially lower blood glucose concentrations to hypoglycemic levels.

Several patent applications and publications describe the discovery of small-molecule glucokinase activators (GKAs) that allosterically modulate or activate the activity of GK (Kamata, K. et al (2004) Structure 12: 429-438; WO 2003/055482 A1; WO 2005/123132 A2; WO 2004/002481 A2; US 6,486,184 B2; WO 2006/040528 A1; Fyfe, M. C. T. (2007) Diabetologia, 50: 1277-1287; McKerrecher, D. et al Bioorg. Med. Chem. Lett. 15 (2005) 2103-2106; Efanov, A. M. et al (2005) Endocrinology 146: 3696-3701; Printz, R. L. and Granner, D. K. (2005) Endocrinology 146: 3693-3695; Brocklehurst, K. J. et al (2004) Diabetes, 53: 535-541; Grimsby, J. et al (2003) Science 301: 370-373). These GKAs increase GK activity by decreasing its S_{0.5} for glucose, and, in some cases, also increasing its Vₘₐₓ. However, for many of these compounds, hypoglycemia has been reported in animal studies which may be a consequence of excessive GK activation. For example, GK activators like Ro-28-1675 cause hypoglycemia in animal efficacy models (Kamata, K. et al (2004) Structure 12: 429-438). Similar hypoglycemic potential is seen in another GK activator, PSN-GK1, at higher dose (Fyfe, M. C. T. (2007) Diabetologia, 50: 1277-1287).

Moreover, document WO 2009/047798 A2 discloses acetamide derivatives as glucokinase activators.

Document WO 2008/104994 A2 relates to 2,2,2-tri-substituted acetamide derivatives as glucokinase activators.

Rat liver glucokinase is inhibited by long chain acyl-CoA. Deinhibition of such inhibition may also result into glucokinase activation (Tippett P. S. et. al (1982) J. Biol. Chem. 25712839-12845, Tippett P. S. et. al (1982) J. Biol. Chem. 257,12846-12852.

A concept of minimizing hypoglycemic potential by liver selective glucokinase activation has been mentioned in patent application no. WO 2005/123123 wherein, compounds described in WO 2004/002481 are identified as liver selective glucokinase activators which increase glucose utilization in the liver without inducing an increase in insulin secretion in response to glucose.

The present disclosure provides a novel class of compounds characterized as glucokinase activators or modulators, and their potential use as medicament for the prophylactic or therapeutic treatment of hyperglycemia, diabetes, obesity, dyslipidemia, metabolic syndrome and the like.

### Summary of the invention

The present disclosure relates to a series of acetamide derivatives described by formula (I), their stereoisomers, tautomers, pharmaceutically acceptable salts, polymorphs, solvates and formulations thereof as glucokinase activators (GKAs); 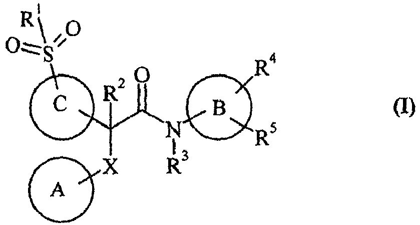 wherein;
R¹ is a cycloalkyl; R² and R³ are hydrogen;
R⁴ and R⁵ are independently selected from a group consisting of hydrogen, halogen, alkyl, cycloalkyl, heterocyclyl, aryl, heteroaryl, mono, di or tri substituted haloalkyl, nitrile, -NR⁶C(O)OR⁷, -(CR⁸R⁹)ₙC(O)OR⁶, -(CR⁸R⁹)ₙC(O)NR⁶R⁷, - (CR⁸R⁹)ₙS(O)ₚNR⁶R⁷, -(CR⁸R⁹)ₙN(R⁶)C(O)R⁷, -(CR⁸R⁹)ₙOR⁶, -C(R⁸R⁹)ₙNR⁶R⁷, - C(R⁸R⁹)ₙCO(R⁶) and -S(O)ₚC(R⁸R⁹)ₙC(O)OR⁶; wherein each of R⁴ and R⁵ is optionally substituted with one or more substituents selected from halo, alkyl, alkenyl, alkynyl, cycloalkyl, aryl, heteroaryl, heterocyclyl, alkylsulfonyl, oxo, nitro, cyano, -(CR⁸R⁹)ₙR⁶, -(CR⁸R⁹)ₙCOOR⁶, -(CR⁸R⁹)ₙC(O)NR⁶R⁷, -(CR⁸R⁹)ₙOR⁶, -SR⁶ or -NR⁶R⁷; wherein n = 0-4;
X represents O;
Ring A represents a monocyclic heterocyclyl;
Ring B is heteroaryl and Ring C is phenyl;
Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino NR⁶R⁷, -OR⁶, -S(O)ₚR⁶, -S(O)ₚNR⁶R⁷, -NR⁶S(O)ₚR⁷, -NR⁶C(O)R⁷, - OS(O)ₚR⁷, -NR⁶C(O)OR⁷, -NR⁶C(O)NR⁶R⁷, -(CR⁸R⁹)ₙC(O)OR⁶, - (CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
   p = 0-2; n= 0-4;
R⁶ and R⁷ are independently selected from a group consisting of hydrogen, alkyl, alkenyl, alkynyl, aryl, arylalkyl, heteroaryl, heteroarylalkyl, cycloalkyl, cycloalkylalkyl, heterocyclyl and heterocyclylalkyl wherein each of R⁶ and R⁷ is optionally substituted with one or more substituents selected from halo, alkyl, cycloalkyl, aryl, heteroaryl, heterocyclyl, alkylsulfonyl, cyano, (CR⁸R⁹)ₙOR⁶, -(CR⁸R⁹)ₙCOOR⁶ or - (CR⁸R⁹)ₙC(O)NR⁶R⁷; wherein n = 0-4;
   or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, - OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl
   wherein R⁶ and R⁷ are as described above;
R⁸ and R⁹ are independently selected from a group consisting of hydrogen, fluorine, OR⁶, alkyl, and perfluoroalkyl,
   or
R⁸ and R⁹ taken together form a monocyclic or a bicyclic ring system which is saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, oxo, -OR⁶, -SR⁶, - NR⁶R⁷, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl.

The present disclosure relates to a series of acetamide derivatives being not part of the present invention described by formula (I), their stereoisomers, tautomers, prodrugs, pharmaceutically acceptable salts, polymorphs, solvates and formulations thereof as glucokinase activators (GKAs); 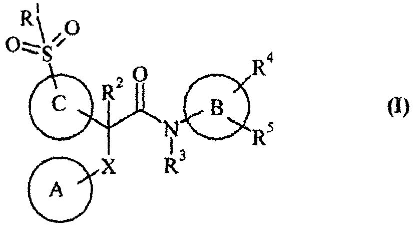 wherein;
R¹ is a heterocycle; R² and R³ are hydrogen;
R⁴ and R⁵ are independently selected from a group consisting of hydrogen, halogen, alkyl, cycloalkyl, heterocyclyl, aryl, heteroaryl, mono, di or tri substituted haloalkyl, nitrile, -NR⁶C(O)OR⁷, -(CR⁸R⁹)ₙC(O)OR⁶, -(CR⁸R⁹)ₙC(O)NR⁶R⁷, - (CR⁸R⁹)ₙS(O)ₚNR⁶R⁷, -(CR⁸R⁹)ₙN(R⁶)C(O)R⁷, -(CR⁸R⁹)ₙOR⁶, -C(R⁸R⁹)ₙNR⁶R⁷, - C(R⁸R⁹)ₙCO(R⁶) and -S(O)ₚC(R⁸R⁹)ₙC(O)OR⁶; wherein each of R⁴ and R⁵ is optionally substituted with one or more substituents selected from halo, alkyl, alkenyl, alkynyl, cycloalkyl, aryl, heteroaryl, heterocyclyl, alkylsulfonyl, oxo, nitro, cyano, -(CR⁸R⁹)ₙR⁶, -(CR⁸R⁹)ₙCOOR^{b}, -(CR⁸R⁹)ₙC(O)NR⁶R⁷, -(CR⁸R⁹)ₙOR⁶, -SR⁶ or -NR⁶R⁷; wherein n = 0-4;
X represents O;
Ring A is selected from aryl or heterocyclyl;
Ring B is heteroaryl and Ring C is phenyl
   or
R¹ is a cycloalkyl; R², R³ and R⁵ are hydrogen;
R⁴ is selected from a group consisting of hydrogen and -OR⁶;
X represents O;
Ring A is selected from aryl or heterocyclyl;
Ring B is fused bicyclic heteroaryl and Ring C is phenyl
   or
R¹ is a cycloalkyl; R² and R³ are hydrogen;
R⁴ is OR⁶; wherein R^{6'} is aryl or heteroaryl; wherein R^{6'} is optionally substituted with one or more substituents selected from halo, alkyl, cyano, (CR⁸R⁹)ₙOR⁶, - (CR⁸R⁹)ₙCOOR⁶ or -(CR⁸R⁹)ₙC(O)NR⁶R⁷; wherein n = 0-4;
R⁵ is selected from hydrogen or alkyl;
Ring A and C are aryl and ring B is heteroaryl
   and
Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino -NR⁶R⁷, -OR⁶, -S(O)ₚR⁶, -S(O)ₚNR⁶R⁷, -NR⁶S(O)ₚR⁷, -NR⁶C(O)R⁷, - OS(O)ₚR⁷, -NR⁶C(O)OR⁷, -NR⁶C(O)NR⁶R⁷, -(CR⁸R⁹)ₙC(O)OR⁶, - (CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
   p = 0-2; n= 0-4;
R⁶ and R⁷ are independently selected from a group consisting of hydrogen, alkyl, alkenyl, alkynyl, aryl, arylalkyl, heteroaryl, heteroarylalkyl, cycloalkyl, cycloalkylalkyl, heterocyclyl and heterocyclylalkyl wherein each of R⁶ and R⁷ is optionally substituted with one or more substituents selected from halo, alkyl, cycloalkyl, aryl, heteroaryl, heterocyclyl, alkylsulfonyl, cyano, (CR⁸R⁹)ₙOR⁶, -(CR⁸R⁹)ₙCOOR⁶ or - (CR⁸R⁹)ₙC(O)NR⁶R⁷; wherein n = 0-4;
   wherein n = 0-4:
   or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, - OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl
   wherein R⁶ and R⁷ are as described above;
R⁸ and R⁹ are independently selected from a group consisting of hydrogen, fluorine, OR⁶, alkyl, and perfluoroalkyl,
   or
R⁸ and R⁹ taken together form a monocyclic or a bicyclic ring system which is saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, oxo, -OR⁶, -SR⁶, - NR⁶R⁷, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl.

The disclosure also relates to the process of preparation of acetamide derivatives of formula-I.

These GKAs are beneficial for the prophylaxis, management, treatment, control of progression, or adjunct treatment of diseases and/or medical conditions such as Type-I and Type-II diabetes, obesity, dyslipidemia, metabolic syndrome and/or diabetes-related complications including retinopathy, nephropathy, neuropathy, ischemic heart disease, arteriosclerosis, β-cell dysfunction, and as therapeutic and/or prophylactic agents for obesity where the activation of glucokinase would be beneficial.

The present disclosure also relates to the compounds of formula (I) that are liver selective GK activators. Such liver selective GK activators may be useful for the treatment of hyperglycemia, diabetes, obesity, dyslipidemia, metabolic syndrome and the like, in mammals and have minimum hypoglycemic potential.

Surprisingly, compounds of the present invention were found to have superior glucokinase activating properties over the compounds disclosed in co-pending Indian patent application 409/CHE/2007.

These and other features, aspects, and advantages of the present subject matter will become better understood with reference to the following description and appended claims. This Summary is provided to introduce a selection of concepts in a simplified form. This Summary is not intended to identify key features or essential features of the claimed subject matter, nor is it intended to be used to limit the scope of the claimed subject matter.

### Detailed description of the invention

### Definitions

In the structural formulae given herein and throughout the present disclosure, the following terms have the indicated meaning:
The term "optionally substituted" as used herein means that the group in question is either unsubstituted or substituted with one or more of the substituents specified. When the group in question is substituted with more than one substituent, the substituent may be same or different.

The term "mono or bicyclic ring" refers to a carbocycle, an aryl, a heterocycle or a heteroaryl which can be aromatic or non-aromatic, saturated or unsaturated, 3 to 18 ring atoms system including 0 to 5 heteroatoms independently selected from S, N, O; the said rings can be optionally substituted with common substituents.

The term "aryl ", alone or in combination with any other term, refers to a monocyclic or a polycyclic aromatic ring system containing carbon-ring atoms, such as phenyl, biphenyl, naphthyl or anthryl which optionally carries one or more substituents, preferably one to three, each independently selected from halogen, trifluoromethyl, trifluoromethoxy, amino, alkyl, alkenyl, alkynyl, alkoxy, alkylcarbonyl, alkoxycarbamoyl, aminocarbonyl, cycloalkyl, cycloalkenyl, acyl, cyano, carbamoyl, methylendioxy, carboxy, alkoxycarbonyl, aryloxy, alkylaminocarbonyl, dialkylaminocarbonyl, hydroxy, heteroaryl, heterocyclyl, nitro, SO₂alkyl, SO₂cycloalkyl and the like.

"Heteroaryl", alone or in combination with any other term, refers to a monocyclic aromatic ring structure containing 5 or 6 ring atoms, or a bicyclic aromatic group having 8 to 12 atoms, containing one or more heteroatoms independently selected from O, S, and N, and optionally substituted with 1 to 3 groups or substituents such as halo, hydroxy, alkoxy, alkylthio, alkylsulfinyl, alkylsulfonyl, acyloxy, aryloxy, heteroaryloxy, amino optionally mono- or di-substituted with alkyl, aryl or heteroaryl groups, amidino, urea optionally substituted with alkyl, aryl, heteroaryl or heterocyclyl groups, aminosulfonyl optionally N-mono- or N,N-di-substituted with alkyl, aryl or heteroaryl groups, alkylsulfonylamino, arylsulfonylamino, heteroarylsulfonylamino, alkylcarbonylamino, arylcarbonylamino, heteroarylcarbonylamino, or the like. "Heteroaryl" is also intended to include oxidized S or N, such as sulfinyl, sulfonyl and N-oxide of tertiary ring nitrogen. A carbon or hetero-atom is the point of attachment of the heteroaryl ring structure such that a stable aromatic ring is retained. Examples of heteroaryl groups are azepinyl, benzimidazolyl, benzisoxazolyl, benzofurazanyl, benzopyranyl, benzothiazolyl, benzothienyl, benzoxazolyl, cinnolinyl, pyridinyl, pyridazinyl, pyrazinyl, quinazolinyl, purinyl, indolyl, quinolinyl, pyrimidinyl, pyrrolyl, oxazolyl, oxadiazolyl, thiazolyl, thienyl, isooxazolyl, oxathiadiazolyl, isothiazolyl, tetrazolyl, imidazolyl, triazinyl, furanyl, benzofuryl, naphthyridinyl, thiadiazolyl, triazolyl, oxazolopyridinyl, imidazopyridinyl, thiazolopyridinyl, thiazolotraizinyl, thiazolopyrazinyl, quinoxalinyl and the like. A substituted heteroaryl contains a substituent attached to an available carbon or heteroatom to produce a stable compound. "Heteroaryl" is also intended to encompass compounds where a heteroaryl is attached to another non-aromatic cyclyl or heterocyclyl rings. Non-limiting examples include chromanyl, dihydrobenzofuranyl, indalinyl, dihydrobenzothienyl, benzodioxolyl dihydrobenzothienyl, dihydrobenzothiopyranyl, isochromanyl, dihydrobenzothiopyranyl sulfone, 1,3-dioxolanyl, benzofuryl, and the like.

As used herein, "heterocycle" or "heterocyclyl" refers to a stable 4 to 7-membered monocyclic or stable 8 to 12 membered bicyclic heterocyclic non-aromatic ring which is either saturated or unsaturated, and which consists of carbon atoms and from one to five heteroatoms selected from the group consisting of N, O, and S. "Heterocyclyl" is also intended to include oxidized S or N, such as sulfinyl, sulfonyl and N-oxide of tertiary ring nitrogen. The heterocyclic ring may be attached at any heteroatom or carbon atom which results in the creation of a stable structure. Non-limiting examples include imidazolidinyl, imidazolinyl, indolinyl, isoindolinyl, isoquinolinyl, isothiazolidinyl, isothiazolidinyl, morpholinyl, 2-oxopiperazinyl, 2-oxopiperdinyl, 2-oxopyrrolidinyl, piperidyl, piperazinyl, pyrazolidinyl, pyrrolidinyl, quinoxalinyl, dihydroimidazole-one, tetrahydrofuryl, tetrahydroisoquinolinyl, tetrahydroquinolinyl, tetrahydroquinoxalinyl, thiamorpholinyl sulfoxide, thiazolinyl, thiazolidine, benzooxazinone, benzothiazinone, isoxazoline, oxazolidin, dihydropyrazinyl, dihydrobenzoxazinyl, dihydrobenzothiazinyl, benzodioxolyl, dihydrobenzodioxolyl, dihydropyridyl and dihydrobenzodiazepinone.

"Alkyl" refers to straight or branched chain having 1 to 10 carbon atoms which is/are further substituted with one or more common substituents. Examples of alkyl groups include, but are not limited to methyl, ethyl, propyl, isopropyl, butyl, t-butyl and the like.

"Cycloalkyl" refers to a cyclic or polycyclic alkyl group containing 3 to 15 carbon atoms which are further substituted with one or more common substituents. Examples of cycloalkyl groups include, but are not limited to cyclopropyl, cyclobutyl, cyclopentyl, bicyclo[4.4.0]decane, adamantanyl, and the like. "Cycloalkyl" is also intended to encompass cyclic alkyl group attached to an aryl group such as 1,2,3,4-tetrahydronaphthalenyl, indanyl and the like.

"Alkenyl", alone or in combination refers to a straight, branched, mono cyclic or polycyclic unsaturated hydrocarbon preferably containing 2 to 10 carbon atoms, and having 1 to 5 double bonds and preferably 1 double bond. Examples of alkenyl groups include, but are not limited to are ethenyl, propenyl, isopropenyl, butenyl, bicycle[2.2.1]heptene and the like.

"Alkynyl", alone or in combination with any other term means a straight or branched hydrocarbon containing 2 to 10 carbon atoms containing 1 to 3 carbon to carbon triple bonds and at least one carbon to carbon triple bond. Examples of alkynyl groups include but are not limited to ethynyl, propynyl, butynyl and the like.

"Halo" or "Halogen", alone or in combination with any other term means halogens such as chloro (Cl), fluoro (F), bromo (Br) and iodo (I).

Common substitution or common substituents are defined as halo, straight chain or branched chain alkyl, alkenyl, alkynyl, cycloalkyl, aryl, heteroaryl, heterocycle, alkylsulfonyl, nitro, cyano, -COOR⁶, -C(O)NR⁶R⁷, -OR⁶, -SR⁶, -NR⁶R⁷, oxo.

The compounds of the present disclosure may have the ability to crystallize in more than one form, a characteristic known as polymorphism, and all such polymorphic forms ("polymorphs") are encompassed within the scope of the disclosure. Polymorphism generally can occur as a response to changes in temperature or pressure or both, and can also result from variations in the crystallization process. Polymorphs can be distinguished by various physical characteristics, and typically the x-ray diffraction patterns, solubility behavior, and melting point of the compound are used to distinguish polymorphs.

The compounds described herein may contain one or more chiral centers and/or double bonds and therefore, may exist as stereoisomers, such as geometric isomers, enantiomers or diastereomers. Accordingly, the chemical structures depicted herein encompass all possible enantiomers and stereoisomers of the illustrated or identified compounds including the stereoisomerically pure form (e.g., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the person skilled in the art. The compounds may also exist in several tautomeric forms including the enol form, the keto form and mixtures thereof. Accordingly, the chemical structures depicted herein encompass all possible tautomeric forms of the illustrated or identified compounds.

Compounds may exist in unsolvated forms as well as solvated forms, including hydrated forms. In general, compounds may be hydrated, solvated or N-oxides. Certain compounds may exist in multiple crystalline or amorphous forms. Also contemplated within the scope of the disclosure are congeners, analogs, hydrolysis products, metabolites and precursor of the compound. In general, unless otherwise indicated, all physical forms are equivalent for the uses contemplated herein and are intended to be within the scope of the present disclosure.

"Prodrug" refers to a derivative of a drug molecule as, for example, esters, carbonates, carbamates, ureas, amides or phosphates that requires a transformation within the body to release the active drug. Prodrugs are frequently, although not necessarily, pharmacologically inactive until converted to the parent drug. Prodrugs may be obtained by bonding a promoiety (defined herein) typically via a functional group, to a drug.

"Promoiety" refers to a group bonded to a drug, typically to a functional group of the drug, via bond(s) that are cleavable under specified conditions of use. The bond(s) between the drug and promoiety may be cleaved by enzymatic or non-enzymatic means. Under the conditions of use, for example following administration to a patient, the bond(s) between the drug and promoiety may be cleaved to release the parent drug. The cleavage of the promoiety may proceed spontaneously, such as via a hydrolysis reaction, or it may be catalyzed or induced by another agent, such as by an enzyme, by light, by acid, or by a change of or exposure to a physical or environmental parameter, such as a change of temperature, pH, etc. The agent may be endogenous to the conditions of use, such as an enzyme present in the systemic circulation to which the prodrug is administered or the acidic conditions of the stomach or the agent may be supplied exogenously.

A "Cocrystal" is a crystalline entity in which more than one molecular substance is incorporated into the unit cell
Herein disclosed are compounds of formula (I) being not part of the present invention wherein R¹ is selected from 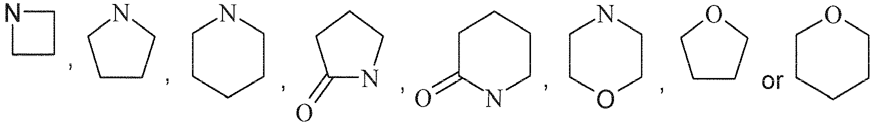
Ring A is selected from 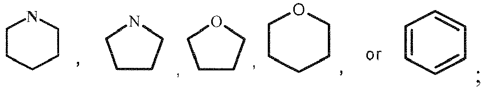
Ring B is selected from 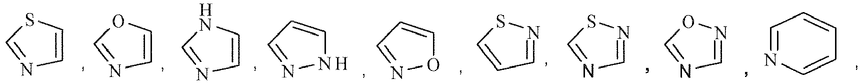 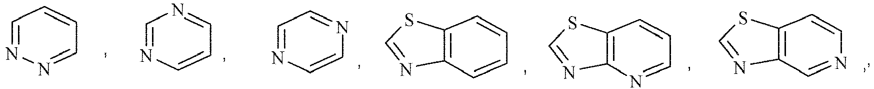 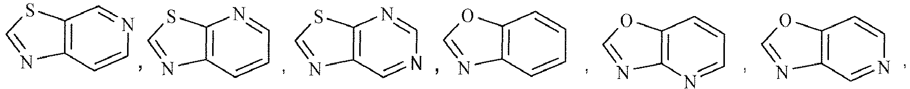 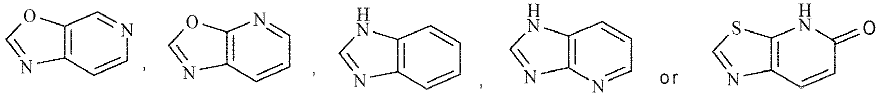
Ring C is 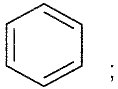 Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino -NR⁶R⁷, -OR⁶, -S(O)ₚR⁶, -S(O)ₚNR⁶R⁷, - NR⁶S(O)ₚR⁷, -NR⁶C(O)R⁷, -OS(O)ₚR⁷, -NR⁶C(O)OR⁷, -NR⁶C(O)NR⁶R⁷, - (CR⁸R⁹)ₙC(C)OR⁶, -(CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
   p = 0-2; n= 0-4;
X is O;
R² and R³ are hydrogen;
R⁴ and R³ are independently selected from a group consisting of hydrogen, halogen, alkyl, cycloalkyl, heterocyclyl, aryl, heteroaryl, mono, di or tri substituted haloalkyl, nitrile, -(CR⁸R⁹)ₙC(O)OR⁶, -(CR⁸R⁹)ₙ(CO)NR⁶R⁷, -(CR⁸R⁹)ₙN(R⁶)C(O)R⁶, - (CR⁸R⁹)ₙOR⁶, -C(R⁸R⁹)ₙNR⁶R⁷, -C(R⁸R⁹)ₙCO(R⁶); wherein each of R⁴ and R⁵ is optionally substituted with one or more substituents selected from halo, alkyl, cycloalkyl, aryl, heteroaryl, heterocycle, alkylsulfonyl, cyano, -(CR⁸R⁹)ₙOR⁶, - (CR⁸R⁹)ₙCOOR⁶ or -(CR⁸R⁹)ₙC(O)NR⁶R⁷;
   wherein n = 0-4;
R⁶ and R⁷ are independently selected from a group consisting of hydrogen, alkyl, aryl, heteroaryl, cycloalkyl, heterocyclyl; wherein each of R⁶ and R⁷ is optionally substituted with one or more substituents selected from halo, alkyl, cycloalkyl, aryl, heteroaryl, heterocyclyl, alkylsulfonyl, cyano, -(CR⁸R⁹)ₙOR⁶, -(CR⁸R⁹)ₙCOOR⁶ or - (CR⁸R⁹)ₙC(O)NR⁶R⁷;
   wherein n = 0-4;
   or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, - OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl
   wherein R⁶ and R⁷ are as described above;
R⁸ and R⁹ are independently selected from a group consisting of hydrogen, fluorine, alkyl, and perfluoroalkyl.

According to one embodiment, the present disclosure relates to compounds of formula (I), or its stereoisomers, tautomers, pharmaceutically acceptable salts, polymorphs, solvates and formulations thereof wherein ring-A is selected from 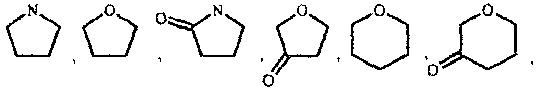 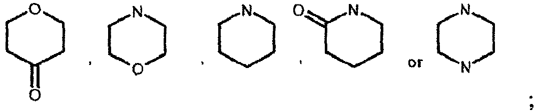
Ring-B is selected from 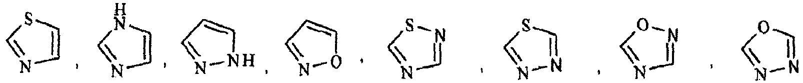 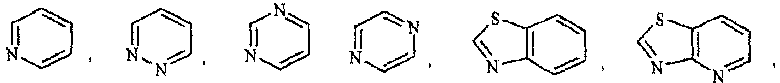 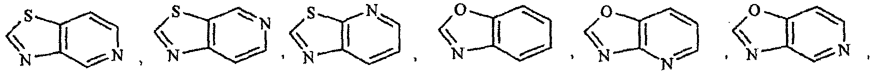 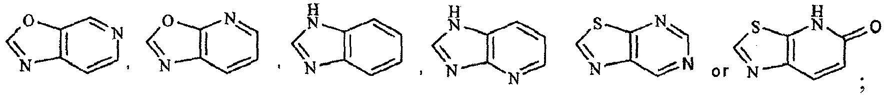 Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino -NR⁶R⁷, -OR⁶, -S(O)ₚR⁶, -S(O)ₚNR⁶R⁷, - NR⁶S(O)ₚR⁷, -NR⁶C(O)R⁷, -OS(O)ₚR⁷, -NR⁶C(O)OR⁷, -NR⁶C(O)NR⁶R⁷, - (CR⁸R⁹)ₙC(O)OR⁶, -(CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
   p = 0-2; n= 0-4;
R¹ is selected from C₃-C₆ cycloalkyl;
R⁴ and R⁵ are independently selected from a group consisting of hydrogen, halogen, alkyl, cycloalkyl, heterocyclyl, aryl, heteroaryl, mono, di or tri substituted haloalkyl, nitrile, nitro, -NR⁶R⁷, -OR⁶, -NR⁶C(O)R⁷, -NR⁶C(O)OR⁷, -(CR⁸R⁹)ₙC(O)OR⁶, - (CR⁸R⁹)ₙ(CO)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -C(R⁸R⁹)ₙNR⁶R⁷, -C(R⁸R⁹)ₙCO(R⁶) and -S(O)ₚC(R⁸R⁹)ₙC(O)OR⁶; wherein each of R⁴ and R⁵ is optionally substituted with one or more substituents selected from halo, straight chain or branched chain alkyl, alkenyl, alkynyl, cycloalkyl, aryl, heteroaryl, heterocyclyl, alkylsulfonyl, oxo, nitro, cyano, -(CR⁸R⁹)ₙCOOR⁶, -C(O)NR⁶R⁷,-OR⁶, - SR⁶ or -NR⁶R⁷;
   wherein n = 0-4;
R⁶ and R⁷ are independently selected from a group consisting of hydrogen, alkyl, aryl, arylalkyl, heteroaryl, heteroarylalkyl, cycloalkyl, cycloalkylalkyl, heterocyclyl and heterocyclylalkyl; wherein each of R⁶ and R⁷ is optionally substituted with one or more substituents selected from halo, alkyl, cycloalkyl, aryl, heteroaryl, heterocyclyl, alkylsulfonyl, cyano, -(CR⁸R⁹)ₙOR⁶, -(CR⁸R⁹)ₙCOOR⁶ or -(CR⁸R⁹)ₙC(O)NR⁶R⁷;
   wherein n = 0-4;
   or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, - OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl
   wherein R⁶ and R⁷ are as described above;
R⁸ and R⁹ are independently selected from a group consisting of hydrogen, fluorine, alkyl, and perfluoroalkyl;

According to a second embodiment, the present disclosure relates to compounds of formula (I), or its stereoisomers, tautomers, pharmaceutically acceptable salts, polymorphs, solvates and formulations thereof wherein ring-B is selected from 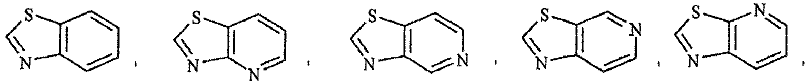 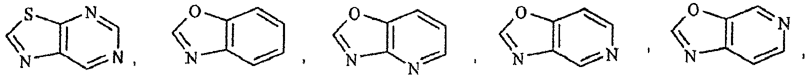 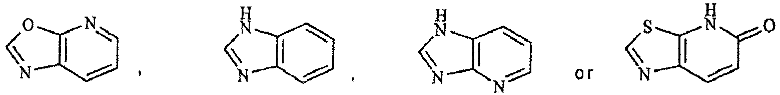
Ring A is selected from 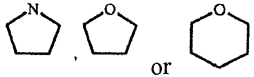 Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino -NR⁶R⁷, -OR⁶, -S(O)ₚR⁶, -S(O)ₚNR⁶R⁷, - NR⁶S(O)ₚR⁷, -NR⁶C(O)R⁷, -OS(O)ₚR⁷, -NR⁶C(O)OR⁷, -NR⁶C(O)NR⁶R⁷, - (CR⁸R⁹)ₙC(O)OR⁶, -(CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
   p = 0-2; n= 0-4;
R¹ is selected from C₃-C₆ cycloalkyl;
R², R³ and R⁵ are hydrogen;
R⁴ is selected from a group consisting of hydrogen, halogen, alkyl, cycloalkyl, heterocyclyl, aryl, heteroaryl, mono, di or tri substituted haloalkyl, nitrile, nitro, -NR⁶R⁷, -OR⁶, - NR⁶C(O)R⁷, -NR⁶C(O)OR⁷, -(CR⁸R⁹)ₙC(O)OR⁶, -(CR⁸R⁹)ₙ(CO)NR⁶R⁷, - (CR⁸R⁹)ₙS(O)ₚNR⁶R⁷, -(CR⁸R⁹)ₙN(R⁶)C(O)R'⁷, -(CR⁸R⁹)ₙOR⁶, -C(R⁸R⁹)ₙNR⁶R⁷, - C(R⁸R⁹)ₙCO(R⁶) and -S(O)ₚC(R⁸R⁹)ₙC(O)OR⁶; wherein R⁴ is optionally substituted with one or more substituents selected from halo, straight chain or branched chain alkyl, alkenyl, alkynyl, cycloalkyl, aryl, heteroaryl, heterocyclyl, alkylsulfonyl, oxo, nitro, cyano, -(CR⁸R⁹)ₙCOOR⁶, -C(O)NR⁶R⁷, -OR⁶, -SR⁶ or NR⁶R⁷;
   wherein n = 0-4;
R⁶ and R⁷ are independently selected from a group consisting of hydrogen, alkyl, aryl, heteroaryl, cycloalkyl, heterocyclyl; wherein each of R⁶ and R⁷ is optionally substituted with one or more substituents selected from halo, alkyl, cycloalkyl, aryl, heteroaryl, heterocyclyl, alkylsulfonyl, cyano, -(CR⁸R⁹)ₙOR⁶, -(CR⁸R⁹)ₙCOOR⁶ or - (CR⁸R⁹)ₙC(O)NR⁶R⁷;
   wherein n = 0-4;
   or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, - OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl
   wherein R⁶ and R⁷ are as described above;
R⁸ and R⁹ are independently selected from a group consisting of hydrogen, fluorine, alkyl, and perfluoroalkyl;

According to a third embodiment, the present disclosure relates to compounds of formula (I), or its stereoisomers, tautomers, pharmaceutically acceptable salts, polymorphs, solvates and formulations thereof wherein ring-B is selected from 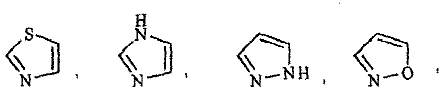 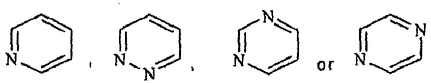 Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino -NR⁶R⁷, -OR⁶, -S(O)ₚR⁶, -S(O)ₚNR⁶R⁷, - NR⁶S(O)ₚR⁷, -NR⁶C(O)R⁷, -OS(O)ₚR⁷, -NR⁶C(O)OR⁷, -NR⁶C(O)NR⁶R⁷, - (CR⁸R⁹)ₙC(O)OR⁶, -(CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups; p = 0-2; n= 0-4;
R¹ is selected from C₃-C₆ cycloalkyl;
R⁴ is -OR^{6'}, wherein R^{6'} is phenyl or pyridyl which is optionally substituted with one or more substituents selected from halo, alkyl, cycloalkyl, alkylsulfonyl, cyano, - (CR⁸R⁹)ₙOR⁶, - (CR⁸R⁹)ₙCOOR⁶, -(CR⁸R⁹)ₙC(O)NR⁶R⁷;
   wherein n = 0-4;
R⁶ and R⁷ are independently selected from a group consisting of hydrogen, alkyl, aryl, arylalkyl, heteroaryl, heteroarylalkyl, cycloalkyl, cycloalkylalkyl, heterocyclyl and heterocyclylalkyl; wherein each of R⁶ and R⁷ is optionally substituted with one or more substituents selected from halo, alkyl, cycloalkyl, aryl, heteroaryl, heterocyclyl, alkylsulfonyl, cyano, -(CR⁸R⁹)ₙOR⁶, -(CR⁸R⁹)ₙCOOR⁶ or -(CR⁸R⁹)ₙC(O)NR⁶R⁷:
   wherein n = 0-4;
   or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nito, cyano, - OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl
   wherein R⁶ and R⁷ are as described above;
R⁸ and R⁹ are independently selected from a group consisting of hydrogen, fluorine, alkyl, and perfluoroalkyl.

In an embodiment of the present invention, compound of formula (I), or its stereoisomers, tautomers, pharmaceutically acceptable salts, polymorphs, solvates and formulations thereof are for use in treating a disease through Glucokinase activation.

In another embodiment, compound of formula (I), or its stereoisomers, tautomers, pharmaceutically acceptable salts, polymorphs, solvates and formulations thereof, are for use in treating a disease through Glucokinase deinhibition.

In yet another embodiment, compound of formula (I), or its stereoisomers, tautomers, pharmaceutically acceptable salts, polymorphs, solvates and formulations thereof, are for use in prophylactic or therapeutic treatment of hyperglycemia or diabetes, particularly type II diabetes.

In another embodiment, compound of formula (I), or its stereoisomers, tautomers, pharmaceutically acceptable salts, polymorphs, solvates and formulations thereof, are for use in preventing diabetes, particularly type II diabetes, in a human demonstrating pre-diabetic hyperglycemia or impaired glucose tolerance.

In yet another embodiment, compound of formula (I), or its stereoisomers, tautomers, pharmaceutically acceptable salts, polymorphs, solvates and formulations thereof, are for use in combined treatment or prevention of diabetes and obesity.

In another embodiment, compound of formula (I), or its stereoisomers, tautomers, pharmaceutically acceptable salts, polymorphs, solvates and formulations thereof, are for use in treating or preventing obesity.

In another embodiment, compound of formula (I), or its stereoisomers, tautomers, pharmaceutically acceptable salts, polymorphs, solvates and formulations thereof, are for use in treatment or prevention of dyslipidemia.

In an embodiment of the present invention, compound of formula (I), or its stereoisomers, tautomers, pharmaceutically acceptable salts, polymorphs, solvates formulations thereof, are for use in treating hyperglycemia, IGT, Syndrome X, type 2 diabetes, type 1 diabetes, dyslipidemia or hyperlipidemia, hypertension, for use in the treatment or prophylaxis of obesity, for use in lowering of food intake, for use in appetite regulation; and for use in regulating feeding behaviour.

In another embodiment, compound of formula (I), or its stereoisomers, tautomers, pharmaceutically acceptable salts, polymorphs, solvates and formulations thereof, are for use in enhancing the secretion of enteroincretins, like GLP-1 and GIP, thereby managing diseases or disorders associated with modulation of secretions of enteroincretins, like hyperglycemia, insulin resistance, impaired glucose tolerance, obesity, gastric emptying, gastroparesis, satiety, leptin resistance, dyslipidemia, wound healing, diabetic complications, such as nephropathy, retinopathy, neuropathy and cataracts.

In an embodiment, it is provided a pharmaceutical composition comprising, as an active ingredient, at least one compound of formula (I), or its stereoisomers, tautomers, pharmaceutically acceptable salts, polymorphs, and solvates thereof, together with one or more pharmaceutically acceptable carriers or excipients.

An embodiment of the invention also provides a pharmaceutical composition comprising, as an active ingredient, at least one compound of formula (I), or its stereoisomers, tautomers, pharmaceutically acceptable salts, polymorphs, and solvates thereof, in combination with one or more pharmaceutically acceptable therapeutically active agents.

Another embodiment of the present invention provide, a pharmaceutical composition wherein the pharmaceutically acceptable therapeutically active agent is selected from anti-diabetic agents, anti-hyperglycemic agents, anti-obesity agents, anti-hypertensive agents or anti-dyslipidemic agents. The pharmaceutical acceptable therapeutically active agent is selected from insulin secretagogues like sulfonylureas selected from amaryl, glyburide, glimepiride, glipyride, glipizide; insulinotropic sulfonyl urea receptor ligands like meglitinides selected from nateglinide, rapaglinide; biguanides like metformin, phenformin, buformin; glucagon antagonists like a peptide or non-peptide glucagon antagonist; glucosidase inhibitors like acarbose, miglitol; glucose sensitive insulinotropic agents like GLP-1, GLP-1 mimetics like exendin-4; insulin sensitizers like troglitazone, rosiglitazone, pioglitazone; dipeptidyl peptidase-IV inhibitors like sitagliptin, vildagliptin; sibutramine, orlistat, rimonabant; fibrates like gemfibrozil, fenofibrate; niacin; statins like rosuvatatin, atorvastatin, simvastatin; cholesterol absorption inhibitors like ezetimibe; bile acid sequestrants like cholestyramine; diuretics like hydrochlorothiazides, mannitol, indapamide, furosemide; angiotensin converting enzyme (ACE) inhibitors like captopril, enalapril; angiotensin-II receptor type-I blockers (ARB) like losartan, irbesartan; rennin inhibitors like aliskerin; β-adrenergic receptor blockers like atenolol, metoprolol; calcium channel blockers like amlodipine, nifedipine; aldosterone receptor antagonist like spironolactone, aldosterone synthase inhibitors like FAD286.

In an embodiment of the present invention it provides use of a compound of formula (I) its polymorphs, stereoisomers, pharmaceutically acceptable salt, or solvate thereof, for the manufacture of a medicament for the activation of Glucokinase.

Further, another embodiment provides a compound of Formula I, or pharmaceutically acceptable salt, or solvate thereof, for use as a Glucokinase activator.

Another embodiment provides a compound of Formula I, or pharmaceutically acceptable salt, or solvate thereof, for use in the prophylactic or therapeutic treatment of hyperglycemia or diabetes, particularly type II diabetes.

Yet another embodiment provides a compound of Formula I, or pharmaceutically acceptable salt, or solvate thereof, for use in the prevention of diabetes, particularly type II diabetes, in a human demonstrating pre-diabetic hyperglycemia or impaired glucose tolerance.

Another embodiment provides a compound of Formula I, or pharmaceutically acceptable salt, or solvate thereof, for use in the prophylactic or or therapeutic treatment of obesity.

Another embodiment provides use of a compound of Formula I, or pharmaceutically acceptable salt, or solvate thereof, for the manufacture of a medicament prophylactic or therapeutic treatment of hyperglycemia or diabetes, particularly type II diabetes.

Yet another embodiment provides use of a compound of Formula I, or pharmaceutically acceptable salt, or solvate thereof, for the manufacture of a medicament for the prophylactic or therapeutic treatment of obesity.

Another embodiment provides the use of a compound of Formula I, or pharmaceutically acceptable salt, or solvate thereof, for the manufacture of a medicament for the prevention of diabetes, particularly type II diabetes, in a human demonstrating pre-diabetic hyperglycemia or impaired glucose tolerance. The compounds and compositions of the present invention may be optionally employed in combination with one or more other anti-diabetic agents or anti-hyperglycemic agents, which include, for example, sulfonylureas (e.g. glyburide, glimepiride, glipyride, glipizide, chlorpropamide, gliclazide, glisoxepid, acetohexamide, glibornuride, tolbutamide, tolazamide, carbutamide, gliquidone, glyhexamide, phenbutamide, tolcyclamide, etc.), biguanides (e.g. metformin, phenformin, buformin, etc.), glucagon antagonists (e.g. a peptide or non-peptide glucagon antagonist), glucosidase inhibitors (e.g. acarbose, miglitol, etc.), insulin secetagogues, insulin sensitizers (e.g. troglitazone, rosiglitazone, pioglitazone, etc.) and the like; or anti-obesity agents (e.g. sibutramine, orlistat, etc.) and the like. The compounds and compositions of the present invention and the other anti-diabetic agents or anti-hyperglycemic agents may be administered simultaneously, sequentially or separately.

According to an embodiment, the present disclosure relates to a process for the preparation of a compound of formula (I), their stereoisomers, co-crystal, tautomers, pharmaceutically acceptable salts, polymorphs, solvates, said process comprising: 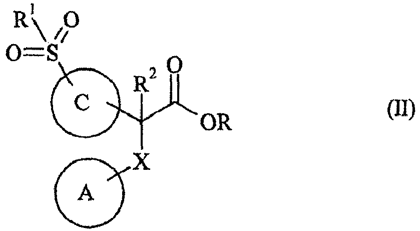 wherein R is hydrogen, alkyl or arylalkyl, with a compound of formula (III) 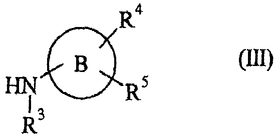 in presence of a suitable amide coupling reagent, optionally hydrolyzing and optionally further coupling with an amine of formula NHR⁶R⁷ to obtain compound of formula (I).

Compounds of formula I may be prepared as shown in the following reaction schemes and the description thereof, as well as relevant literature procedures that may be used by one skilled in the art. Exemplary reagents and procedures for these reactions appear hereinafter and in the working examples. Protection and deprotection in the schemes below may be carried out by procedures generally known in the art (see, for example, Greene, T. W. and Wuts, P.G.M., Protecting Groups in Organic Synthesis, 3rd Edition, 1999 [Wiley]).

The compounds of formula (I) may be prepared following independent general synthetic routes as outlined in the Schemes 1-6:

### Scheme 1: General route for the synthesis of compounds of formula (I).

Compounds of formula (II) wherein R is hydrogen, alkyl or arylalkyl, may be reacted with compounds of formula (III) following amide coupling reaction conditions to obtain compounds of formula (I), as shown herein below.

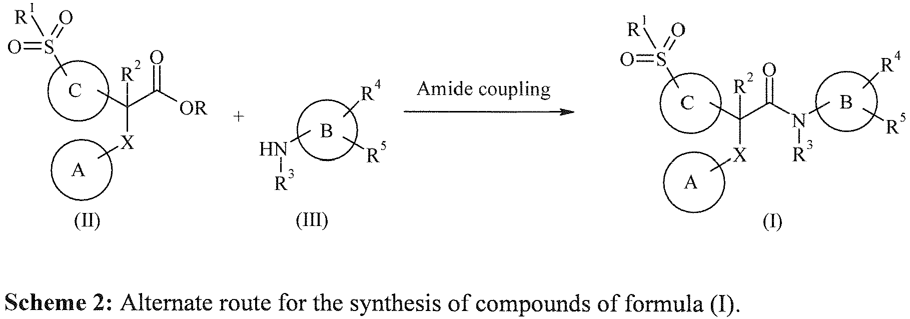

Compounds of formula (IV) wherein R is hydrogen, alkyl or arylalkyl, obtained according to WO2004072031, may be reduced to compounds of formula (V) which may be reacted with compounds of formula (III) under amide coupling conditions to provide compounds of formula (VI). Compounds of formula (VI) may be reacted with compounds of formula (VIIa) under Mitsunobu conditions to provide compounds of formula (I).

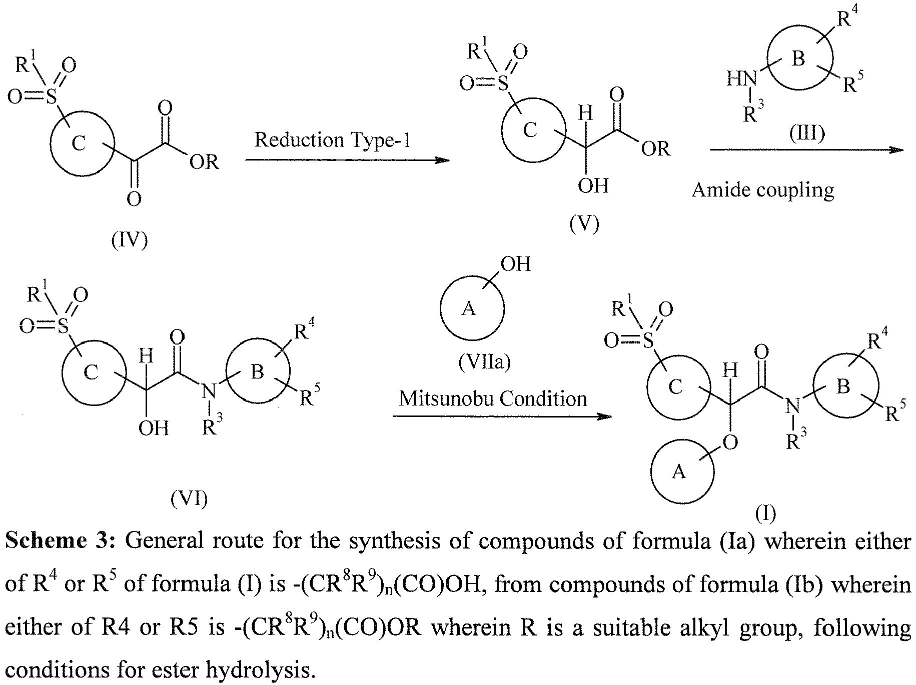

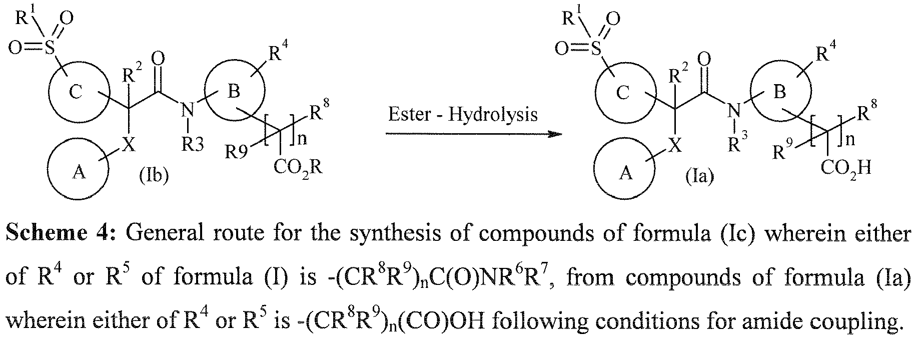

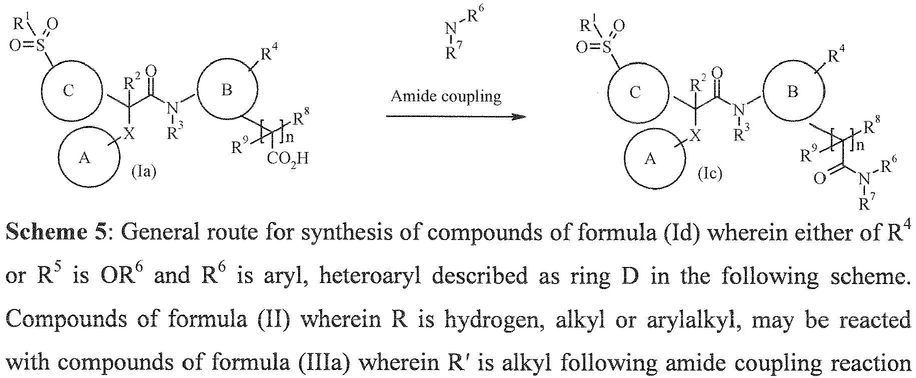

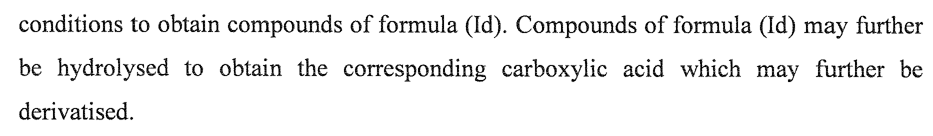

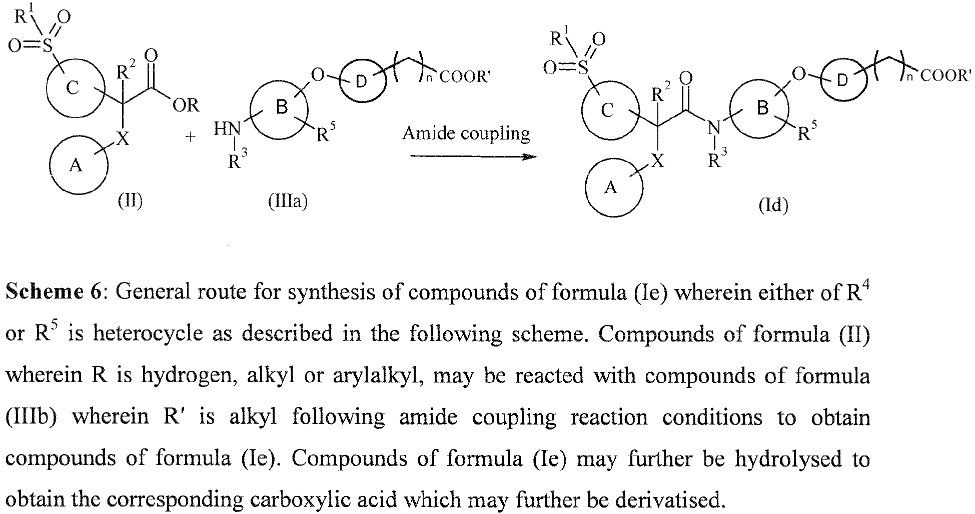

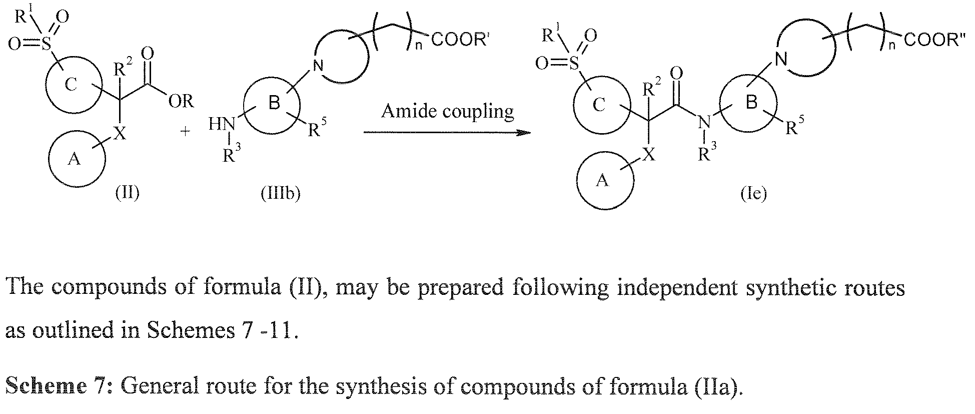

Compounds of formula (IVa) may be reduced to compounds of formula (VIIIa) followed by esterification to obtain compounds of formula (VIII), which may be halogenated to obtain compounds of formula (IX). Compounds of formula (IX) may be reacted with compounds of formula (VII) under nucleophilic substitution conditions to obtain compounds of formula (IIb) wherein R is alkyl or arylalkyl, which may be hydrolysed to obtain intermediates of formula (IIA).

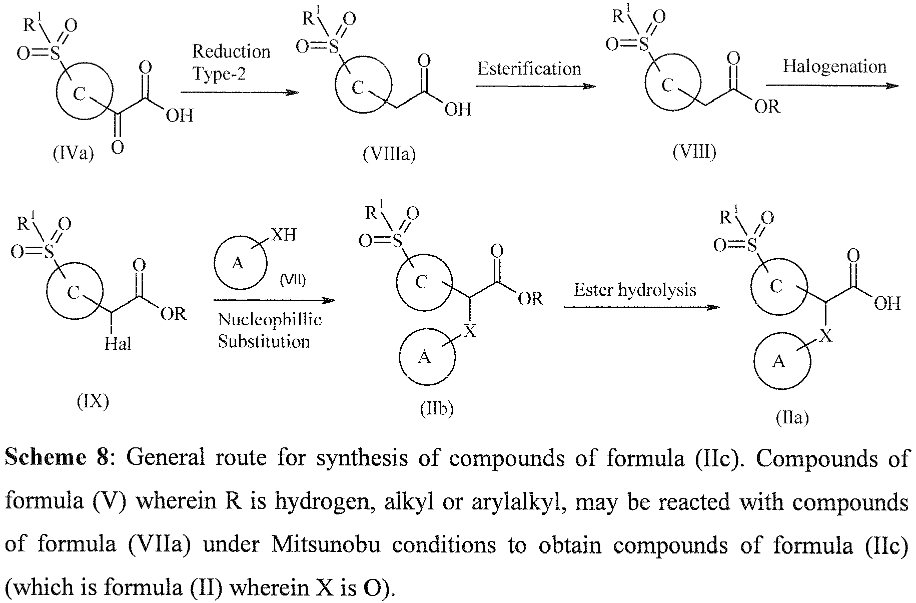

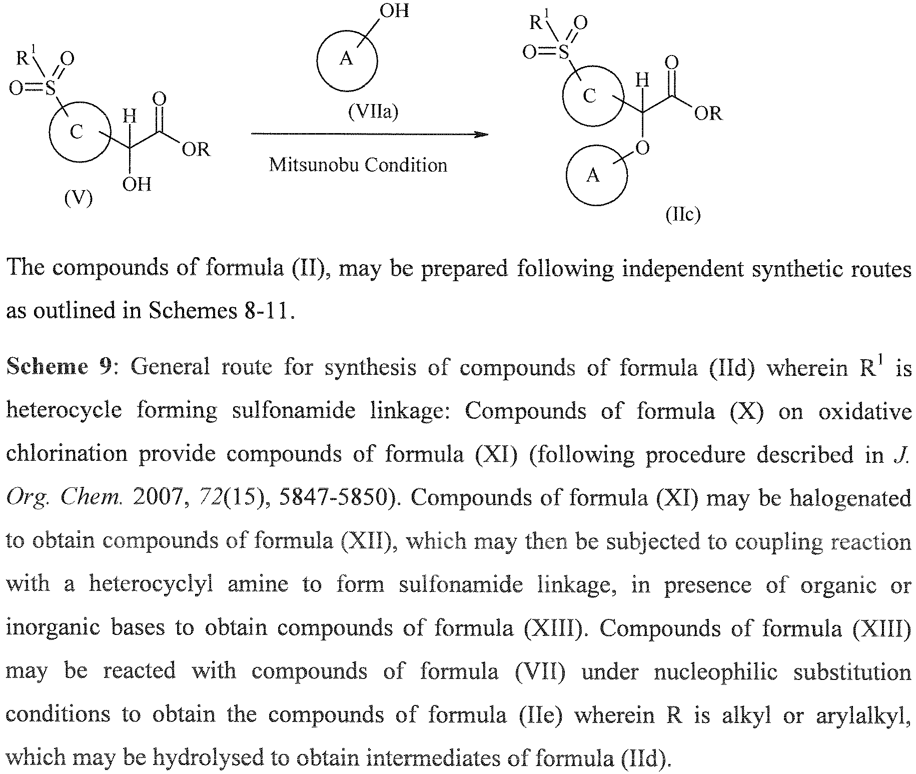

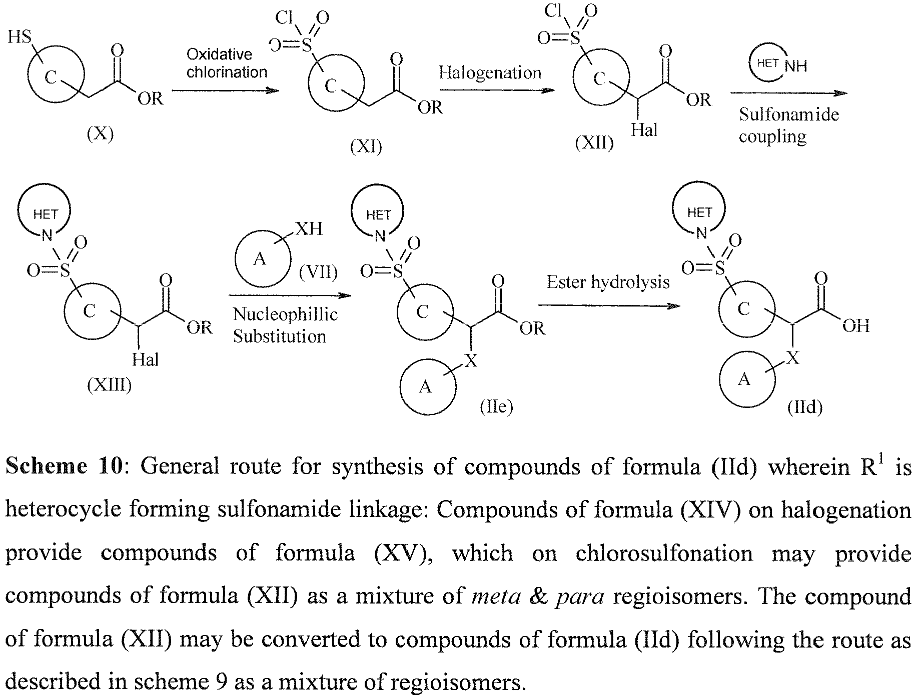

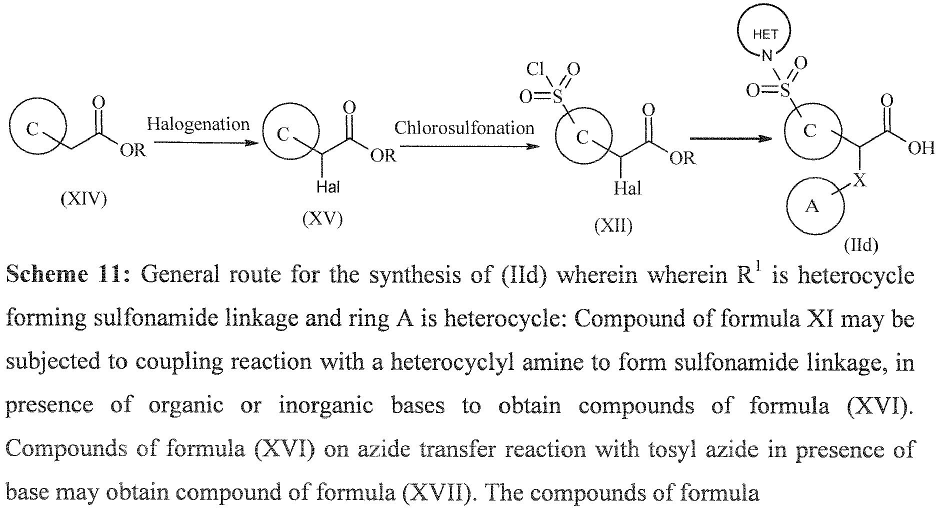

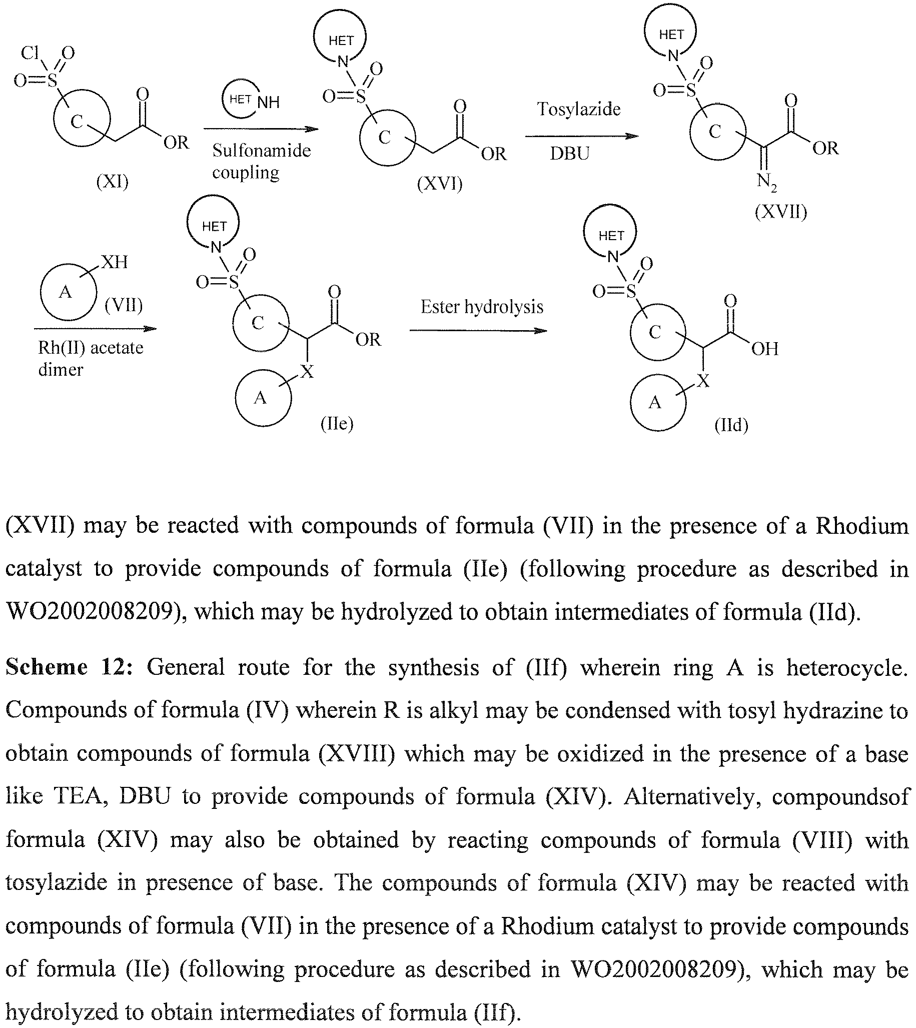

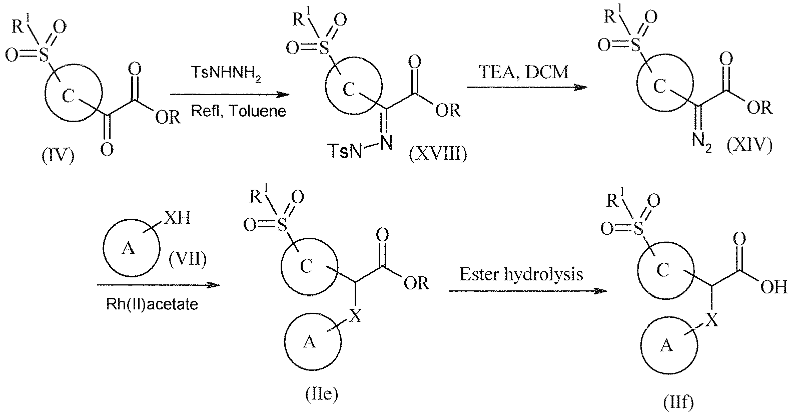

The compounds of formula (VIII) may be prepared following independent synthetic routes as outlined in Schemes 12 -13.

**Scheme 13:** General route for the synthesis of compounds of formula (VIII): Compounds of formula (X), which are available commercially, may be reacted with R¹-LG, where LG is a suitable leaving group, using reaction conditions for nucleophilic substitution to obtain S-alkylated compounds of formula (XI). The compounds of formula (XI) may be oxidized (sulfur to sulfone) to obtain compounds of formula (VIII).

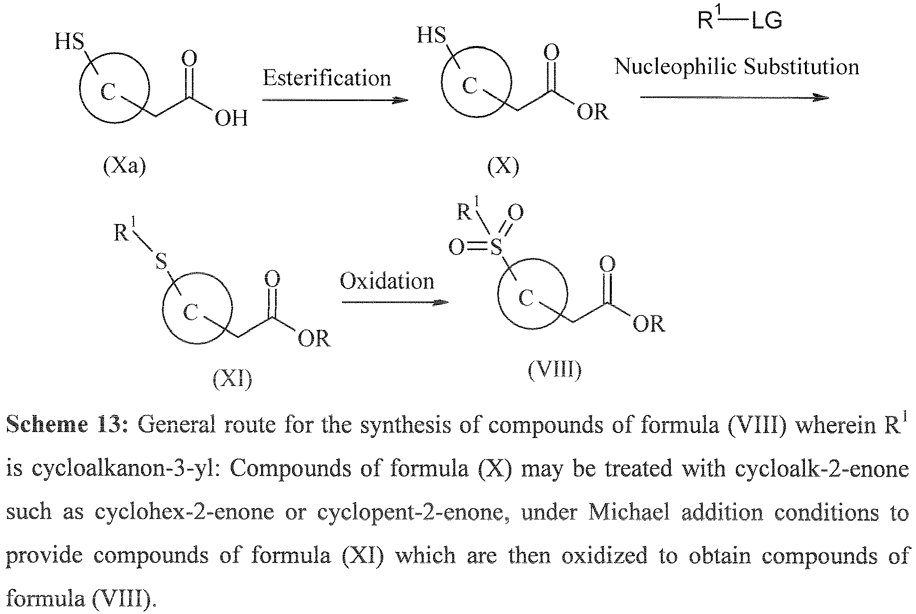

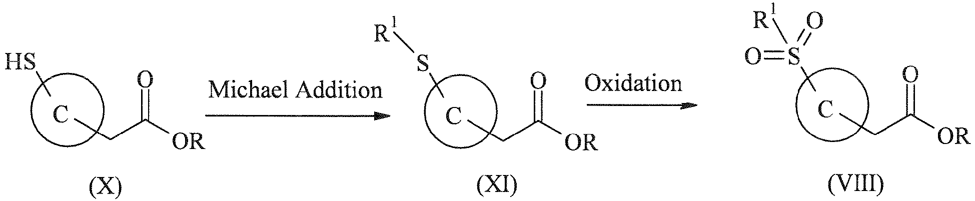

**Amide Coupling Conditions: Condition-I:** When R = H, the amide coupling may be carried out using any suitable amide coupling regents such as oxallyl chloride, thionyl chloride, BOP-Cl, DCC, HOBt, HOAt, HATU, EDCI, alkylchloroformate and the like in the presence of organic non-nucleophillic bases such as triethyl amine, diisopropylethyl amine, pyridine, N-methyl pyrrolidine, N,N-dimethylaminopyridine, DBU, DABCO, other hindered amines and pyridines. The amide coupling reaction may be carried out in the presence of solvents such as dichloromethane, dichloroethane, DMF, dimethylacetamide, THF, acetonitrile or mixture of them may be used at a temperature ranging from -5 to 150 °C. The reaction may be carried out optionally in presence of catalytic amount of DMF. **Condition-II:** When R is not H, the amide coupling may be carried out by heating ester and amine either in the absence of solvent or in presence of high boiling solvent like toluene, xylene, DMSO. Amide coupling may be carried out in presence of trialkyl aluminium (Chem. Commun., 2008, 1100-1102).

**Halogenation Conditions:** Halogenation reaction may be carried out using reagents such as N-halosuccinimide, dihalogens and the like, in presence of radical generating reagents like peroxides such as benzoylperoxide. Solvents used for this reaction include, but are not limited to, carbontetrachloride and ethers or mixtures thereof. The reaction may be carried out at a temperature ranging from -5 to 80 °C.

**Conditions for Nucleophilic Substitution:** Nucleophilic substitution reaction, may be carried out using any suitable organic or inorganic bases. Organic bases may be selected from a group consisting of mono, di or trialkyl amines particularly methylamine, ethylamine, dimethylamine, diethylamine or triethylamine. Inorganic bases may be selected from a group consisting of alkali and alkaline earth metal hydrides, hyroxides, carbonates and bicarbonates or mixtures thereof. Solvents used for this reaction may be selected from a group consisting of lower alcohols, acetone, acetonitrile, DMSO, DMF, dimethylacetamide, THF and toluene, or mixtures thereof. The reaction may be carried out at a temperature in the range of 0 to 150 °C .

**Conditions for Ester Hydrolysis:** Ester hydrolysis of carboxylic acids may be carried out using general saponification conditions employing inorganic bases such as alkali and alkaline earth metal hyroxides, carbonates and bicarbonates, for example lithium hydroxide, sodium hydride, sodium carbonate, potassium carbonate, cesium carbonate and the like; in the presence of solvents such as water, methanol, ethanol, THF and diethyl ether or mixtures thereof. These reactions may be done at 0 °C to refluxing temperature.

**Conditions for Esterification:** Ester formation, from the above mentioned carboxylic acids, may be carried out using general esterification conditions employing appropriate alcohol like methanol, ethanol and a suitable inorganic acid selected from HCl, H₂SO₄, or thionyl chloride, or base catalysed ester formation using alkyl halide and suitable base like sodium hydride, sodium hydroxide, sodium carbonate, potassium carbonate, sodium bicarbonate, potassium bicarbonate, cesium carbonate and the like in presence of solvents such as acetone, acetonitrile, DMF, DMSO, THF and diethyl ether or mixtures thereof.

**Conditions for Oxidation:** Oxidation of sulfanyls to sulfonyls, may be carried out using appropriate oxidizing reagent such as H₂O₂, perbenzoic acid, mCPBA, Oxone, dioxirane and the like in the presence of a solvent such as DCM, DCE, DMF, DMSO, THF and diethyl ether or mixtures thereof. Reagents like OsO₄, KMnO₄, PCC can also be used for such oxidation process.

**Conditions for Reduction Type-1:** Reduction Type-1, may be carried out using appropriate reduction conditions for transforming carbonyls to *sec*-alcohols employing reducing agents like hydrogenation in presence of catalysts such as Pd/C, Pt/C, PtO₂ and the like. Such reduction by hydrogenation can also be done using organo-metallic complexes as catalyst from metals like Iron, Rhodium, Ruthenium, and phosphorus-based organic ligands like triphenylphosphine, bidentate phosphine ligands such as bis(diphenylphosphino)ethane. Such hydrogenation based reductions can also be done under asymmetric reduction conditions to yield chiral products (in individual enantiomers and in enantiometically enriched form) if employed appropriate chiral phosphine ligands such as chiral 2,2'-bis(diphenylphosphino)-1,1'-binaphthyl (BINAP) to form the organometallic complex. Such reductions can also be done using metal hydrides such as sodium borohydride, lithium aluminiumhydride, borane reagents and the like. Such metal hydride or borane reagent based reductions can also be done in asymmetric way to yield chiral products (in individual enantiomers and in enantiometically enriched form) by using appropriate chiral ligands like tartarate (EP 0320096), chiral 1,1'-bi-2-napthol (BINOL), oxazaborolidines.

**Conditions for Reduction Type-2:** Reduction Type-2, may be carried out using specific conditions known for transformation of arylic carbonyl group to corresponding arylalkyl functionality. Such reductions may be done using known Wolff-Kishner (KOH, NH₂-NH₂) or Clemmensen (Zn/HCl) reduction conditions.

**Conditions for Mitsunobu reaction:** The Mitsunobu reaction between alcohol and phenol, to obtain the corresponding ether, may be carried out in THF using triphenylphosphine (TPP) and diethyl azodicarboxylate (DEAD) or diisopropyl azodicarboxylate (DIAD) as reagents.

**Sulfonamide Coupling Condition:** Sulfonamide may be prepared by reacting any appropriate amine with sulfonylhalide in the presence of base such as pyridine, triethylamine & diisopropylethylamine. The reaction may be carried out in suitable solvent like pyridine, dichloromethane or tetrahydrofuran.

**Oxidative Chlorination:** Thiols can be converted to sulfonyl chlorides under mild condition of oxidative chlorination. Here thiols are treated with combination of oxidant and chlorinating agent such as KNO₃-TMSCl, H₂O₂-SOCl₂, Oxone-SOCl₂ in appropriate solvent such as DCM, acetonitrile, DMF or combination of acetonitrile-AcOH. The reaction may be carried out at a temperature in the range of 5 to 100 °C.

**Chlorosulfonation:** Aryl or hetroaryl sulfonyl chloride synthesis may be carried out by elecrophilic substitution reaction using reagent like chlorosulfonic acid, SO₂Cl₂ in appropriate solvent which are not limited to halogenated like DCM, DCE, CHCl₃, CCl₄, but also nonpolar solvents like Benzene, Tolune, Dioxane or mixture thereof. The reaction may be carried out at a temperature in the range of 0 °C to 60 °C.

### Abbreviations

The following abbreviations are employed in the examples and elsewhere herein:
DMF: Dimethyl formamide
DMSO: Dimethyl sulfoxide
DCM: Dichloromethane
DCE: Dichloroethane
THF: Tetrahydrofuran
mCPBA: meta chloro perbenzoic acid
BOP-Cl: Bis(2-oxo-3-oxazolidinyl)phosphinic chloride
DABCO: 1,4-Diazabicyclo[2.2.2]octane
DBU: 1,8-Diazabicyclo[5.4.0]undec-7-ene
DCC: N,N-Dicyclohexyl carbodiimide
EDCI: 1-Ethyl-3-(3-dimetylaminopropyl)carbodiimide
HOBT: 1-Hydroxybenzotriazole
HOAT: 1-Hydroxy-7-azabenzotriazole
HBTU: O-(benzotriazol-1-yl)-tetramethyluronium hexafluorophosphate
HATU: O-(7-azabenzotriazol-1-yl)-tetramethyluronium hexafluorophosphate
TPP: triphenylphosphine
DEAD: diethyl azodicarboxylate
DIAD: diisopropyl azodicarboxylate
NBS : N-Bromosuccinamide
KNO₃ : Potassium nitrate
AlCl₃: Aluminium chloride
TMSCl : Trimethylsilyl chloride
H₂O₂ : Hydrogen peroxide
LiOH : Lithium hydroxide

### Examples

All stereoisomers of the compounds of the instant disclosure are contemplated, either in admixture or in pure or substantially pure form. The compounds of the present disclosure can have asymmetric centers at any of the carbon atoms, consequently, compounds of formula (I) can exist in enantiomeric, or diastereomeric forms, or in mixtures thereof. The processes for preparation can utilize racemates, enantiomers, or diastereomers as starting materials. When diastereomeric or enantiomeric products are obtained as mixtures, they can be separated by conventional methods for example, chromatographic separation or fractional crystallization or through diasteriomeric salt formation. When intended, a desired enantiomer or diasteriomer can also be obtained by following appropriate enantioselective or diastereoselective reactions.

Structures of the intermediates as well as the final compounds were confirmed by nuclear magnetic resonance spectra for proton (¹H NMR) and LCMS.

### Preparation 1: (2,4-Difluoro-phenoxy)-[4-(tetrahydro-pyran-4-sulfonyl)-phenyl]-acetic acid:

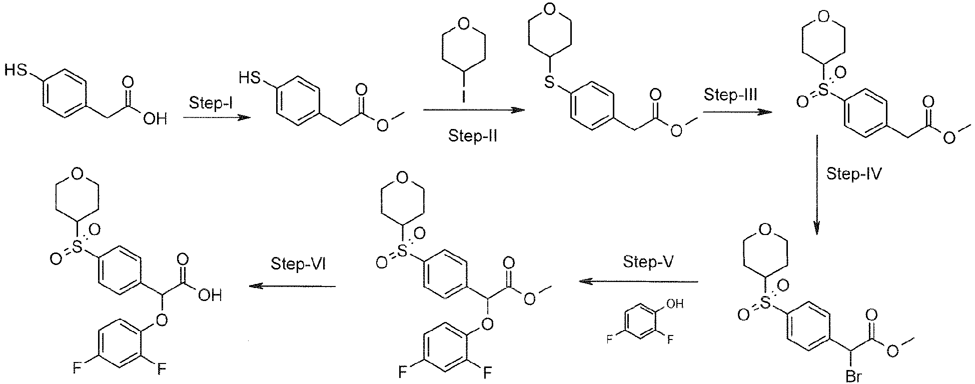

### Step-I :-(4-Mercapto-phenyl)-acetic acid methyl ester:

4-Mercapto-phenyl acetic acid (10 gm, 59.5 mmol) was dissolved in methanol (300 ml). To this solution, sulfuric acid (5.8 gm, 59.5 mmol) was added and mixture was refluxed for 2 hrs. Solvent was removed under reduced pressure. The resulting residue was dissolved in ethyl acetate (200 ml), and was washed with water, sodium bicarbonate solution and brine (100 ml each), dried over anhydrous sodium sulfate, and filtered and solvent was removed under reduced pressure to provide (4-mercapto-phenyl)-acetic acid methyl ester (10 gm).
¹HNMR (CDCl₃, 400 MHz):- δ 3.44 (s, 1H), 3.58 (s, 2H), 3.70 (s, 3H), 7.16 (d, J = 8.0 Hz, 2H), 7.25 (d, J = 8.4 Hz, 2H).

### Step-II: [4-(Tetrahydro-pyran-4-ylsulfanyl)-phenyl)-acetic acid methyl ester:

(4-Mercapto-phenyl)-acetic acid methyl ester (1.8 gm, 9.89 mmol) was dissolved in DMF (20 ml) at 0 °C. Triethylamine (1.5 gm, 14.83 mmol) was added and stirred for 15 minutes followed by addition of 4-iodo-tetrahydropyran (3.14 gm, 14.83 mmol). The reaction mixture was stirred overnight at room temperature. Solvent was removed under reduced pressure, the resulting residue was taken into water and extracted with ethyl acetate (50 ml x 2) washed with water and brine (50 ml each) dried over anhydrous sodium sulfate, fliltered and concentrated to obtain a crude product which was purified by column chromatography over silica gel using 15 % ethyl acetate in hexanes as eluent to provide [4-(Tetrahydro-pyran-4-ylsulfanyl)-phenyl]-acetic acid methyl ester (2.1 gm).
¹HNMR (CDCl₃, 400 MHz):- δ 1.65-1.72 (m, 2H), 1.89-1.92 (m, 2H), 3.25-3.26(m, 1H), 3.41-3.46 (m, 2H), 3.62 (s, 2H), 3.71 (s, 3 H), 3.86-4.00 (m, 2 H), 7.39 (d, J = 8.0 Hz, 2H), 7.45 (d, J = 8.4 Hz, 2H). MS (EI) m/z: 267.20 (M+1).

### Step-III: -[4-(Tetrahydro-pyran-4-sulfonyl)-phenyl]-acetic acid methyl ester:

[4-(Tetrahydro-pyran-4-ylsulfanyl)-phenyl]-acetic acid methyl ester (2.1 gm, 7.89 mmol) was taken in dichloromethane (100 ml) at 0 °C. mCPBA (8.00 gm, 47.36 mmol) was added portion wise and stirred for 5 hrs at room temperature. Reaction mixture was diluted with dichloromethane (100 ml), solid was filtered, filtrate was washed with saturated solution of sodium thiosulphate (150 ml), organic layer was washed with saturated solution of sodium bicarbonate, water and brine (100 ml each), dried over anhydrous sodium sulfate, filtered and concentrated. Crude compound was purified by column chromatography over silica gel using 35 % ethyl acetate hexane as eluent to provide pure [4-(tetrahydro-pyran-4-sulfonyl)-phenyl]-acetic acid methyl ester (1.69 gm).
¹HNMR (CDCl₃, 400 MHz):- δ 1.80-1.87 (m, 2H), 1.92-1.95 (m, 2H), 3.12-3.18 (m, 1H), 3.31-3.38 (m, 2H), 3.76 (s, 5H), 4.06 - 4.10 (m, 2H),), 7.53 (d, J = 8.0 Hz, 2H), 7.86 (d, J = 8.0 Hz, 2H).

### Step-IV: Bromo-[4-(tetrahydro-pyran-4-sulfonyl)-phenyl]-acetic acid methyl ester:

[4-(Tetrahydro-pyran-4-sulfonyl)-phenyl]-acetic acid methyl ester (1.60 gm, 5.36 mmol), NBS (1.04 g, 5.90 mmol) and benzoyl peroxide (0.130 gm, 0.53 mmol) in carbon tetrachloride (50 ml) was refluxed for 7 hrs. Reaction mixture was diluted with DCM (50 ml), washed with water and brine (100 ml each), dried over anhydrous sodium sulfate, fliltered and concentrated. Crude compound was purified by column chromatography over silica gel using 30% ethyl acetate hexane as eluent to give the pure bromo-[4-(tetrahydro-pyran-4-sulfonyl)-phenyl]-acetic acid methyl ester (0.75 gm).
¹HNMR (CDCl₃, 400 MHz) :- δ 1.75-1.87 (m, 2H), 1.93-1.96 (m, 2H), 3.15-3.22 (m, 1H)3.33-3.38 (m,2 H), 3.85 (s, 3H), 4.07-4.11 (m, 2H), 5.41 (s,1H), 7.79 (d, J= 8.4 Hz, 2H), 7.90 (d, J = 8.4 Hz, 2H).

### Step-V:(2, 4-Difluoro-phenoxy)-[4-(tetrahydro-pyran-4-sulfonyi)-phenyl]-acetic acid methyl ester:

A mixture of 2,4- difluorophenol (0.28 gm, 2.18 mmol) and cesium carbonate (0.39 gm, 1.19 mmol) in acetonitrilie (20 ml) stirred for 30 mins. A solution of bromo-[4-(tetrahydro-pyran-4-sulfonyl)-phenyl]-acetic acid methyl ester (0.75 gm, 1.98 mmol) in acetonitrile (5 ml) was added drop wise at room temperature and stirred further for 2 hrs. Reaction mixture was diluted with ethyl acetate (50 ml), washed with water, 1N NaOH (cold, 50 ml), and brine (50 ml) dried over anhydrous sodium sulfate, filtered and concentrated to provide (2, 4-difluoro-phenoxy)-[4-(tetrahydro-pyran-4-sulfonyl)-phenyl]-acetic acid methyl ester (0.35 gm).
¹HNMR (CDCl₃, 400 MHz) :- δ 1.80-1.87 (m, 2H), 1.93-1.96 (m, 2H), 3.15-3.22 (m,1H), 3.33-3.38 (m, 2H), 3.80 (s, 3H), 4.07-4.11 (m, 2H), 5.70 (s, 1H), 6.78-6.83 (m, 1H), 6.89-6.95 (m, 1H), 6.98-7.04 (m,1H), 7.83 (d, J = 8.0 Hz, 2H), 7.95 (d, J = 8.4 Hz, 2H).MS (EI) m/z:444.00 (M+18)

### Step VI: (2, 4-Difluoro-phenoxy)-[4-(tetrahydro-pyran-4-sulfonyl)-phenyl]-acetic acid:

To a solution of (2, 4-difluoro-phenoxy)-[4-(tetrahydro-pyran-4-sulfonyl)-phenyl]-acetic acid methyl ester (0.35 gm, 0.82 mmol) in THF (3 ml), was added lithium hydroxide monohydrate (0.035 gm, 2.05 mmol) in 1 ml water and stirred overnight at room temperature. Solvent was removed under reduced pressure, residue obtained was taken into water (5 ml), extracted with diethyl ether (10 ml x 2). Aqueous layer was acidified with 1 N HCl solution, and extracted with ethyl acetate (10 ml x 3), washed with water and brine (20ml, each) dried over anhydrous sodium sulfate, filtered and solvent was concentrated to provide (2, 4-difluoro-phenoxy)-[4-(tetrahydro-pyran-4-sulfonyl)-phenyl]-acetic acid (0.28 gm).
¹HNMR (CDCl₃, 400 MHz) :- δ 1.79-1.87 (m, 2H), 1.92-1.95 (m, 2H), 3.15-3.21 (m, 1H), 3.32-3.38 (m,2H), 4.07-4.11 (m, 2H), 5.71 (s, 1H), 6.78-6.83 (m, 1H), 6.89-6.95 (m, 1H), 6.98-7.04 (m,1H), 7.85 (d, J = 8.4 Hz, 2H), 7.95 (d, J = 8.0 Hz, 2H).

### Preparation 2: (2,4-Difluoro-phenoxy)-[4-(piperidine-1-sulfonyl)-phenyl]-acetic acid:

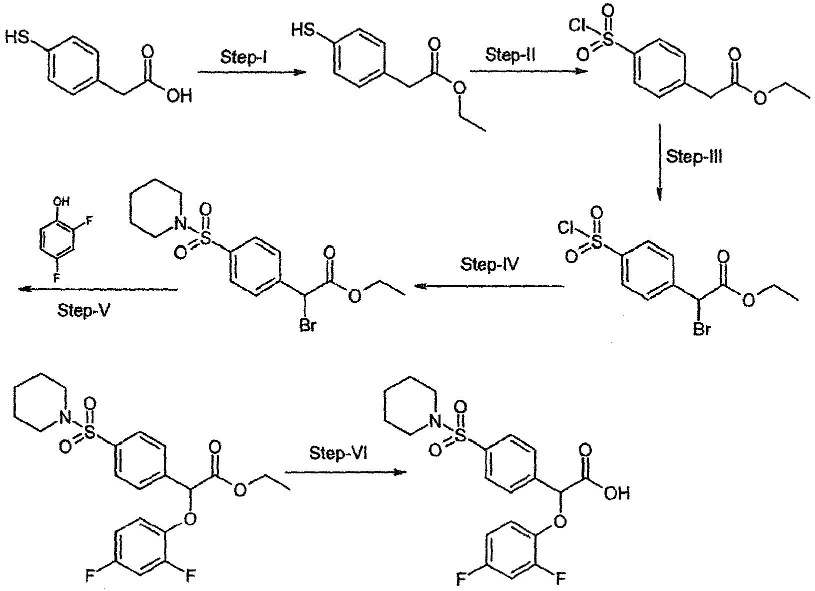

### Step I: (4-Mercapto-phenyl)-acetic acid ethyl ester:

To a solution of 4-mercapto-phenyl acetic acid (5 gm, 29.72 mmol) in ethanol (60 ml) was added sulfuric acid (1.58 ml, 29.72 mmol) dropwise. Reaction mixture was then heated at 60 °C for 3 hr, concentrated to remove ethanol. The residue was neutralized with satd. aq. NaHCO₃ solution and extracted with ethyl acetate (3X30 ml) washed with brine, dried over anhydrous sodium sulfate, filtered and concentrated under reduced pressure to provide (4-mercapto-phenyl)-acetic acid ethyl ester (5.5 gm).
¹H NMR (400 MHz, CDCl₃): δ 1.22 (t, J=6.8 Hz, 3H), 3.55 (s, 2H), 4.11 (q, J=7.2 Hz, 2H), 7.14 (d, J=8.0 Hz, 2H), 7.22 (d, J=8.0 Hz, 2H).

### Step II: (4-Chlorosulfonyl-phenyl)-acetic acid ethyl ester:

(4-Mercapto-phenyl)-acetic acid ethyl ester (2 gm, 10.2 mmole) was taken in a seal tube. To it DCM (30 ml) was added followed by KNO₃ (2.42 gm, 22.4 mmole) and TMSCl (2.79 ml, 22.4 mmole). Mixture was heated at 50 °C for 24 hr, cooled to room temperature and filtered to remove solids, residue was washed with DCM (10X2 ml), combined filtrate was washed with water followed by brine solution, dried over anhydrous sodium sulfate, filtered and concentrated under reduced pressure to provide (4-chlorosulfonyl-phenyl)-acetic acid ethyl ester (1.6 gm).
¹H NMR (400 MHz, DMSO-d₆): δ 1.17 (t, J=6.8 Hz, 3H), 3.66 (s, 2H), 4.05-4.11 (m, 2H), 7.21 (d, J=8.0 Hz, 2H), 7.54 (d, J=8.0 Hz, 2H).

### Step III: Bromo-(4-chlorosulfonyl-phenyl)-acetic acid ethyl ester:

(4-Chlorosulfonyl-phenyl)-acetic acid ethyl ester (1.6 gm, 6.45 mmol) was taken in carbon tetrachloride (13 ml). N-Bromosuccinimide (1.26 gm, 7.09 mmol) was added in one lot followed by benzoyl peroxide (171 mg, 0.7 mmol). The reaction mixture was refluxed for 2 days. The reaction mixture was cooled to room temperature and filtered; filtrate was washed with water followed by brine solution, dried over anhydrous sodium sulfate and concentrated under reduced pressure to provide bromo-(4-chlorosulfonyl-phenyl)-acetic acid ethyl ester (2.08 gm) as a gummy mass. The crude material was used as such for further reaction.

### Step IV: Bromo-[4-(piperidme-1-sulfonyl)-phenyl]-acetic acid ethyl ester:

Bromo-(4-chlorosulfonyl-phenyl)-acetic acid ethyl ester (2.08 gm, 6.38 mmol) was taken in DCM (63 ml) under argon atmosphere and cooled to 0-5 °C, piperidine (0.56 ml, 5.74 mmol) was added dropwise to the mixture at 0-5 °C. Reaction mixture was then stirred at room temperature for 30 min. Reaction mixture was washed with water followed by brine solution, dried over anhydrous sodium sulfate and concentrated under reduced pressure to give the crude product, which was purified by flash column chromatography over silica gel using 30-50% ethyl acetate in hexane as eluent to give bromo-[4-(piperidine-1-sulfonyl)-phenyl]-acetic acid ethyl ester (1.2 gm).
¹H NMR (400 MHz, CDCl₃): 8 1.31 (t, J=7.2 Hz, 3H), 1.46-1.49 (m, 2H), 1.65-1.71 (m, 4H), 2.99-3.04 (m, 4H), 4.21-4.31 (m, 2H), 5.38 (s, 1H), 7.72 (d, J=8.4 Hz, 2H), 7.76 (d, J=8.4 Hz, 2H).

### Step V: (2,4-Difluoro-phenoxy)-14-(piperidine-1-sulfonyl)-phenyl]-acetic acid ethyl ester:

2,4-Difluorophenol (0.38 ml, 3.89 mmol) and cesium carbonate (0.76 gm, 2.33 mmol) were taken in acetonitrile (30 ml) under argon atmosphere and was stirred for 30 minutes. Bromo-[4-(piperidine-1-sulfonyl)-phenyl]-acetic acid ethyl ester (1.4 gm, 3.86 mmol) in acetonitrile (15 ml) was added to the mixture and stirred at 25 °C for 3 hour. Reaction mixture was diluted with water (30 ml), extracted with ethyl acetate (3X20 ml), organic layer was washed with brine solution, dried over anhydrous sodium sulfate and concentrated under reduced pressure to give a gummy mass which was purified by flash column chromatography using over silica gel using 40-60% ethyl acetate in hexane as eluent to give (2,4-difluoro-phenoxy)-[4-(piperidine-1-sulfonyl)-phenyl]-acetic acid ethyl ester (1.31 gm).
¹H NMR (400 MHz, CDCl₃): δ 1.22 (t, J=7.2 Hz, 3H), 1.44-1.46 (m, 2H), 1.66-1.67 (m, 4H), 3.0-3.04 (m, 4H), 4.21-4.27 (m, 2H), 5.66 (s, 1H), 6.80-6.82 (m, 1H), 6.89-6.95 (m, 1H), 6.95-7.29 (m, 1H), 7.76 (d, J=8.4 Hz, 2H), 7.81 (d, J=8.4 Hz, 2H).

### Step VI: (2,4-Difluoro-phenoxy)-[4-(piperidine-1-sulfonyl)-phenyl]-acetic acid:

(2,4-Difluoro-phenoxy)-[4-(piperidine-1-sulfonyl)-phenyl]-acetic acid ethyl ester (1.3 gm, 2.98 mmol) was dissolved in THF (5 ml) and methanol (0.5 ml). To this was added a solution of lithium hydroxide (0.62 gm, 14.90 mmol) in water (5 ml) and stirred for 18 hours at 25⁰C. Organic solvents were evaporated from the reaction mixture and aqueous layer was acidified with 1N HCl, precipitated product was filtered off and residue was washed with water. Solid product was dried under vacuum to provide (2,4-difluoro-phenoxy)-[4-(piperidine-1-sulfonyl)-phenyl]-acetic acid (1gm).
¹H NMR (400 MHz, DMSO-d₆): δ 1.35-1.42 (m, 2H), 1.51-1.61 (m, 4H), 2.86-2.91 (m, 4H), 6.12 (s, 1H), 7.02-7.06 (m, 1H), 7.18-7.24 (m, 1H), 7.33-7.39 (m, 1H), 7.79-7.84 (brs, 4H). MS (EI) m/z: 412.0 (M+1).

### Preparations 3 to 7 were prepared in analogous manner of preparation 2.

| **Preparation No.** | **IUPAC Name** |
|---|---|
| 3 | (2,4-Difluoro-phenoxy)-[4-(morpholine-4-sulfonyl)-phenyl]-acetic acid |
| 4 | (4-Chloro-phenoxy)-[4-(morpholine-4-sulfonyl)-phenyl]-acetic acid |
| 5 | (4-Chloro-phenoxy)-[4-(piperidine-1-sulfonyl)-phenyl]-acetic acid |
| 6 | (4-Chloro-2-fluoro-phenoxy)-[4-(piperidine-1-sulfonyl)-phenyl]-aceticacid |
| 7 | (4-Chloro-2-fluoro-phenoxy)-[4-(morpholine-4-sulfonyl)-phenyl]-aceticacid |

### Preparation 8: (2,4-Difluoro-phenoxy)-[4-(pyrrolidine-1-sulfonyl)-phenyl]-acetic acid Preparation 9: (2,4-Difluoro-phenoxy)-[3-(pyrrolidine-1-sulfonyl)-phenyl]-acetic acid

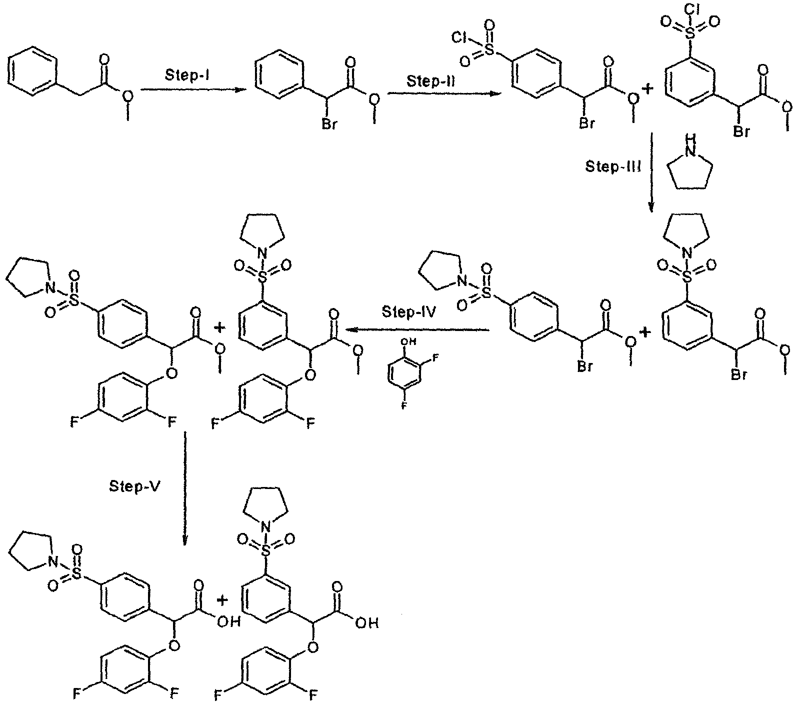

### Step I: Bromo-phenyl-acetic acid methyl ester:

Phenyl-acetic acid methyl ester (10 gm, 66.6 mmol) was taken in carbon tetrachloride (100 ml). N-Bromosuccinimide (13.05 gm, 73.33 mmol) was added in one lot followed by benzoyl peroxide (1.77 gm, 7.3 mmol) and refluxed for 3 hours. The reaction mixture was cooled to room temperature and filtered; filtrate was washed with water followed by brine solution, dried over anhydrous sodium sulfate and concentrated under reduced pressure to provide bromo-phenyl-acetic acid methyl ester (16.4 gm) as a gummy mass.
¹H NMR (400 MHz, CDCl₃): δ 3.82 (s, 3H), 5.39 (s, 1H), 7.39-7.41 (m, 3H), 7.56-7.58 (m, 2H).

### Step II: Bromo-(4-chlorosulfonyl-phenyl)-acetic acid methyl ester and Bromo-(3-chlorosulfonyl-phenyl)-acetic acid methyl ester:

Chlorosulphonic acid (1.5 ml, 21.86 mmol) was dissolved in DCM (20 ml) and cooled to 0-5 °C under argon atmosphere. Bromo-phenyl-acetic acid methyl ester (1.0 gm, 4.36 mmol) was added to cooled mixture in drop wise manner and continued to stirr at 0-5⁰C. for 1 hour then at room temperature for 1 hour. Reaction mixture was diluted with water (10 ml), organic layer was washed with brine solution, dried over anhydrous sodium sulfate, filtered and concentrated under reduced pressure to give the product as mixture of para and meta regioisomers as gummy mass. The crude material was used as such for further reaction.

### Step III: Bromo-[4-(pyrrolidine-1-sulfonyl)-phenyl]-acetic acid methyl ester and Bromo-[3-(pyrrolidine-1-sulfonyl)-phenyl]-acetic acid methyl ester:

A solution of pyrrolidine (0.14 gm, 2 mmol) in DCM (10 ml) was cooled to 0-5 °C under argon atmosphere. To this was added a solution of bromo-(4-chlorosulfonyl-phenyl)-acetic acid methyl ester and bromo-(3-chlorosulfonyl-phenyl)-acetic acid methyl ester (0.207 gm, 2.04 mmol) in DCM (10 ml) drop wise at 0-5 °C The reaction mixture was then stirred at room temperature for 18 hours, diluted with water (10 ml), organic layer was washed with brine solution, dried over anhydrous sodium sulfate, filtered and concentrated under reduced pressure to give the product as a mixture of para and meta regioisomers.
MS (EI) *m*/*z*: 363.9 (M+1)

### Step IV: (2,4-Difluoro-phenoxy)-(4-(pyrrolidine-1-sulfonyl)-phenyl]-acetic acid methyl ester and (2,4-Difluoro-phenoxy)-[3-(pyrrolidine-1-sulfonyl)-phenyl]-acetic acid methyl ester:

2,4-Difluorophenol (0.5 gm, 3.86 mmol) and cesium carbonate (0.75 gm, 2.32 mmol) were taken in acetonitrile (10 ml). Mixture was stirred for 30 minutes under argon atmosphere, followed by addition of regioisomers obtained in step III (1.4 gm, 3.86 mmol) in acetonitrile (15 ml). Mixture was then stirred at 25 °C 18 hours, diluted with water (30 ml) and extracted with ethyl acetate (3X20 ml). The organic layer was washed with brine solution, dried over anhydrous sodium sulfate, filtered and concentrated under reduced pressure to give the product as a gummy mass which was purified by column chromatography(30-40% ethyl acetate in hexane) to give 0.720 gm of mixture of products as meta and para regioisomers.
MS (EI) *m*/*z:* 412.2 (M+1).

### Step V: (2,4-Difluoro-phenoxy)-[4-(pyrrolidine-1-sulfonyl)-phenyl]-acetic acid and (2,4-Difluoro-phenoxy)-[3-(pyrrolidine-1-sulfonyl)-phenyl]-acetic acid:

Mixture of regioisomers obtained in step IV (0.6 gm, 1.45 mmol) was dissolved in THF (5 ml) and methanol (5 ml). A solution of lithium hydroxide (0.15 gm, 3.64 mmol) in water (5 ml) was added stirred for 18 hours at 25 °C. Organic solvents were evaporated from the reaction mixture and aqueous layer was acidified with 1N HCl and extracted with ethylacetate (3X10 ml), organic layer was washed with brine solution, dried over anhydrous sodium sulfate, and concentrated under reduced pressure to give 0.56 gm of products as mixture of para and meta regioisomers.
MS (EI) *m*/*z*: 398.2.

### Preparations 10 to 12 were prepared in analogous manner of preparations 8 and 9.

| Preparation No. | IUPAC Name |
|---|---|
| 10 | [4-(Azetidine-1-sulfonyl)-phenyl]-(2,4-difluoro-phenoxy)-acetic acid |
| 11 | [3-(Azetidine-1-sulfonyl)-phenyl]-(2,4-difluoro-phenoxy)-acetic acid |
| 12 | (2,4-Difluoro-phenoxy)-[3-(piperidine-1-sulfonyl)-phenyl]-acetic acid |

### Preparation 13: [4-(Piperidine-1-sulfonyl)-phenyl]-[(tetrahydro-pyran-4-yloxy)]-acetic acid

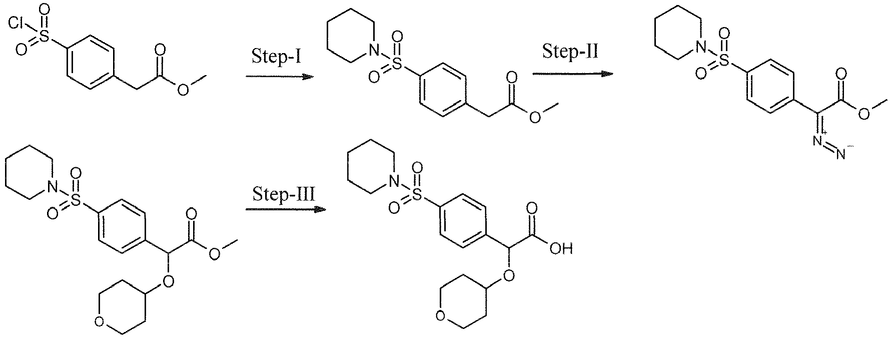

### Step I: (4-(Piperidine-1-sulfonyl)-phenyl)-acetic acid methyl ester:

(4-Chlorosulfonyl-phenyl)-acetic acid methyl ester, obtained similarly as described in Preparation 2, (1.0 gm, 4.02 mmol) was taken in dry DCM (40 ml) under argon atmosphere and cooled to 0-5°C, piperidine (0.50 ml, 4.42 mmol) followed by triethylamine (0.61 ml, 4.42 mmol) was added dropwise to the mixture at 0-5°C. Reaction mixture was then brought to room temperature and stirred for additional 2 hours. Reaction mixture was washed with water followed by brine solution, dried over anhydrous sodium sulfate and concentrated under reduced pressure to give the crude product, which was purified by column chromatography using 20-30% ethyl acetate in hexane as eluent to give [4-(Piperidine-1-sulfonyl)-phenyl)-acetic acid methyl ester (0.95 gm).
¹H NMR (400 MHz, CDCl₃): δ 1.38-1.44 (m, 2H), 1.60-1.66 (m, 4H), 2.96-2.99 (m, 4H), 3.70 (s, 2H), 3.72 (s, 3H), 7.43 (d, J = 8.3 Hz, 2H), 7.70 (d, J = 8.3 Hz, 2H)
MS (EI) m/z: 297.9 (M + 1).

### Step II: Diazo-[4-(piperidine-1-sulfonyl)-phenyl]-acetic acid methyl ester:

[4-(Piperidine-1-sulfonyl)-phenyl]-acetic acid methyl ester (0.5g, 1.68 mmol) was dissolved in dry acetonitrile (8ml) under argon atmosphere. To the above solution *para* toluene sulfonyl azide (0.36g, 1.85 mmol) was added, followed by DBU (0.38 ml, 2.52 mmol) in dropwise manner, and stirred for 30 min. Reaction mixture was poured in ice cold water (20ml), extracted with ethyl acetate (3X20 ml), organic layer was washed with brine solution, dried over anhydrous sodium sulfate, and concentrated under reduced pressure to provide crude product, which was purified by column chromatography using 20-25% ethyl acetate in hexane as eluent to give Diazo-[4-(piperidine-1-sulfonyl)-phenyl]-acetic acid methyl ester (0.42 gm).
¹H NMR (400 MHz, CDCl₃): δ 1.38-1.44 (m, 2H), 1.60-1.66m(m, 4H), 2.96-2.99 (m, 4H), 3.90 (s, 3H), 7.64 (d, J = 8.8 Hz, 2H), 7.74(d, J = 8.3 Hz, 2H).
MS (EI) m/z: 324.1 (M+1).

### Step III: [4-(Piperidine-1-sulfonyl)-phenyl]-[(tetrahydro-pyran-4-yloxy)]-acetic acid methyl ester:

Diazo-[4-(piperidine-1-sulfonyl)-phenyl)-acetic acid methyl ester (0.72 gm, 2.13 mmol) was dissolved in DCM (10 ml) under argon atmosphere. To this solution, 4-hydroxy tetrahydropyran (0.24 ml, 2.56 mmol) was added followed by rhodium (II) acetate dimer (19 mg, 0.044 mmol). Mixture was stirred at 25⁰C for 30 min. Reaction mixture was diluted with DCM (10 ml), organic layer was washed with water followed by brine solution, dried over anhydrous sodium sulfate, filtered and concentrated under reduced pressure to give a crude product which was purified by column chromatography using 35-40% ethyl acetate in hexane as eluent to provide [4-(Piperidine-1-sulfonyl)-phenyl]-[(tetrahydro-pyran-4-yloxy)]-acetic acid methyl ester (520 mg).
¹H NMR (400 MHz, CDCl₃): δ 1.25-1.28 (m, 2H), 1.60-1.69 (m, 6H), 1.88-2.00(m, 2H), 2.98-3.01(m, 4H), 3.38-3.49(m, 2H), 3.60-3.66 (m, 1H), 3.75 (s, 3H) 3.91-4.02 (m, 2H), 5.13 (s, 1H), 7.64 (d, J = 8.3 Hz, 2H), 7.75(d, J = 8.3 Hz, 2H).
MS (EI) m/z: 398.2 (M + 1).

### Step IV: [4-(Piperidine-1-sulfonyl)-phenyl]-[(tetrahydro-pyran-4-yloxy)]-acetic acid:

To [4-(Piperidine-1-sulfonyl)-phenyl]-[(tetrahydro-pyran-4-yloxy)]-acetic acid methyl ester (0.52 gm, 1.30 mmol) was added a solution of lithium hydroxide (0.274 gm, 6.54 mmol) in water (3 ml) followed by THF (4 ml) and methanol (0.5 ml) and stirred for 2 hours at 25⁰C. Organic solvents were evaporated from the reaction mixture and aqueous layer was acidified by using 1N HCl, extracted with ethyl acetate (3X20 ml), organic layer was washed with brine solution, dried over anhydrous sodium sulfate, and concentrated under reduced pressure to provide [4-(Piperidine-1-sulfonyl)-phenyl]-[(tetrahydro-pyran-4-yloxy)]-acetic acid (0.35gm).
¹H NMR (400 MHz, DMSO-d₆): δ 1.36-1.37 (m, 2H), 1.48-1.53 (m, 6H), 1.85-1.93 (m, 2H), 2.86-2.89 (m, 4H), 3.30-3.35 (m, 4H), 3.60-3.65(m, 1H), 3.75-3.85(m, 2H), 5.25 (s, 1H), 7.67 (d, J = 8.3 Hz, 2H), 7.74 (d, J = 8.4 Hz, 2H).
MS (EI) m/z: 384.2 (M + 1).

### Preparations 14 was prepared in analogous manner of preparations 13.

| **Preparation No.** | **IUPAC Name** |
|---|---|
| 14 | [4-(Morpholine-4-sulfonyl)-phenyl]-[(tetrahydro-pyran-4-yloxy)]-acetic Acid |

### Preparation 15: (4-Cyclopropanesulfonyl-phenyl)-[(tetrahydro-furan-3-yloxy)]-acetic acid

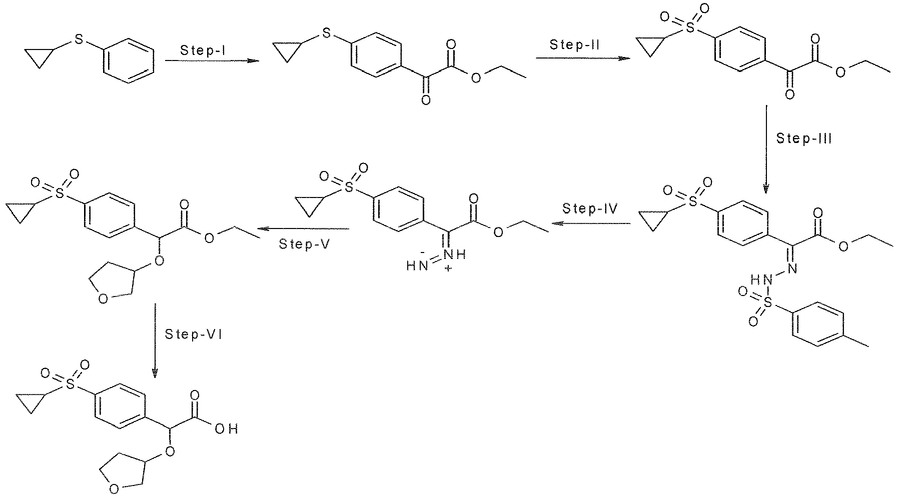

### Step I: (4-Cyclopropylsulfanyl-phenyl)-oxo-acetic acid ethyl ester:

AlCl₃ (7.98 gm, 48.42 mmole) was suspended in DCM (50 ml) and cooled to 0⁰C under argon atmosphere. To this suspension was added chlorooxo ethylacetate (4.5 ml, 39.98 mmol) at 0⁰C and stirred for 45 min. followed by addition of a solution of cyclopropylsulfanyl-benzene (5 gm, 33.28 mmol) in DCM (10 ml) and stirred at 25⁰C for 2 hr. Reaction mixture was slowly poured over crushed ice, organic layer was separated and aqueous layer was extracted with DCM (3X50 ml), combined organic layer was washed with brine solution, dried over anhydrous sodium sulfate, filtered and concentrated under reduced pressure to obtain (4-cyclopropylsulfanyl-phenyl)-oxo-acetic acid ethyl ester (3.1 gm) as an oil.
¹H NMR (400 MHz, CDCl₃): δ 0.72-0.73 (m, 2H), 1.15-1.17 (m, 2H), 1.40 (t, J=6.6 Hz, 3H), 2.18-2.21 (m, 1H), 4.41 (q, J=6.8 Hz, 2H), 7.43 (d, J=8.0 Hz, 2H), 7.90 (d, J=8.0 Hz, 2H). MS (EI) *m*/*z*: 250.9 (M+1).

### Step II: (4-Cyclopropanesulfonyl-phenyl) oxo acetic acid ethyl ester:

(4-Cyclopropylsulfanyl-phenyl)-oxo-acetic acid ethyl ester (3.1 gm, 12.53 mmole) in DCM (50 ml) was cooled to 0-5⁰C followed by addition of mCPBA (9.8 gm, 31.33 mmol) portion wise at 0⁰C. After stirring at 25⁰C for 4 hr, the reaction mixture was filtered; filtrate was washed with saturated aq. Na₂S₂O₃ and satd. aq. sodium bicarbonate solution followed by brine solution, dried over anhydrous sodium sulfate, filtered and concentrated under reduced pressure to give (4-cyclopropanesulfonyl-phenyl) oxo acetic acid ethyl ester (3 gm).
¹H NMR (400 MHz, CDCl₃): δ 1.05-1.10 (m, 2H), 1.36-1.39 (m, 2H), 1.40 (t, J=6.8 Hz, 3H), 2.45-2.50 (m, 1H), 4.42 (q, J=7.2 Hz, 2H), 8.01 (d, J=8.4 Hz, 2H), 8.20 (d, J=8.4 Hz, 2H). MS (EI) *m*/*z*: 297.1 (M+NH₄).

### Step III: p-Toluene sulfonyl hydrazone (4-cyclopropyl sulfonyl) phenyl acetic acid ethyl ester:

A mixture of (4-cyclopropanesulfonyl-phenyl) oxo acetic acid ethyl ester (0.5 gm, 1.77 mmole) and p-toluene sulfonyl hydrazide (0.48 gm, 2.3 mmol) in toluene (15 ml) was refluxed for 16 hr using a Dean-Stark apparatus. Reaction mixture was concentrated to give the crude product which was purified by column chromatography over silica gel using 20-25% ethyl acetate in hexane as eleunt to provide p-toluene sulfonyl hydrazone (4-cyclopropyl sulfonyl) phenyl acetic acid ethyl ester (0.5 gm).
MS (EI) *m*/*z*: 451.0 (M+1).

### Step IV: (4-Cyclopropanesulfonyl-phenyl) diazo acetic acid ethyl ester:

To a solution of p-toluene sulfonyl hydrazone (4-cyclopropyl sulfonyl) phenyl acetic acid ethyl ester (0.5 gm, 1.23 mmole) in dry DCM (6 ml), was added triethylamine (0.17 mL, 1.35 mmol) and stirred at 25⁰C for 1 hr. Reaction mixture was concentrated to provide (4-cyclopropanesulfonyl-phenyl) diazo acetic acid ethyl ester (0.5 gm) which was used as it is for next reaction.
MS (EI) *m*/*z*: 295.1 (M+1).

### Step V: (4-Cyclopropanesulfonyl-phenyl)-[(tetrahydro-furan-3-yloxy)]-acetic acid ethyl ester:

(4-Cyclopropanesulfonyl-phenyl) diazo acetic acid ethyl ester (2 gm, 6.74 mmol) was dissolved in DCM (33 mL) under argon atmosphere. To this solution, 3-hydroxy tetrahydrofuran (1.36 mL, 16.87 mmol) was added followed by rhodium(II)acetate dimer (62 mg, 0.14 mmol). Mixture was stirred at 25⁰C for 3 hr. Reaction mixture was diluted with DCM (25 mL), organic layer was washed with water followed by brine solution, dried over anhydrous sodium sulfate, filtered and concentrated under reduced pressure to give a crude product which was purified by column chromatography using 35-40% ethyl acetate in hexane as eluent to provide (4-cyclopropanesulfonyl-phenyl)-[(tetrahydrofuran-3-yloxy)]-acetic acid ethyl ester (1 gm).
¹H NMR (400 MHz, CDCl₃): δ 0.99-1.05 (m, 2H), 1.18-1.24 (m, 3H), 1.31-1.34 (m, 2H), 1.96-2.10 (m, 2H), 2.38-2.48 (m, 1H), 3.73-3.97 (m, 4H), 4.14-4.19 (m, 2H), 4.20-4.22 (m, 1H), 4.97-4.99 (d, J=8.4,1H), 7.61-7.64 (m, 2H), 7.85-7.88 (m, 2H).
MS (EI) *m*/*z*: 355 (M+1), 372.1 (M+18).

### Step VI: (4-Cyclopropanesulfonyl-phenyl)-[(tetrahydro-furan-3-yloxy)]-acetic acid:

To (4-cyclopropanesulfonyl-phenyl)-[(tetrahydro-furan-3-yloxy)]-acetic acid ethyl ester (1 gm, 2.82 mmol) was added a solution of lithium hydroxide (0.59 gm, 14.12 mmol) in water (5 ml) followed by THF (10 ml) and methanol (2 ml) and stirred for 2 hours at 25 °C.

Organic solvents were evaporated from the reaction mixture and aqueous layer was acidified 1N HCl, extracted with ethyl acetate (3X10 ml), organic layer was washed with brine solution, dried over anhydrous sodium sulfate, and washed concentrated under reduced pressure to provide (4-cyclopropanesulfonyl-phenyl)-[(tetrahydro-furan-3-yloxy)]-acetic acid (0.8 gm)
¹H NMR (400 MHz, CDCl₃): δ 1.05-1.07 (m, 2H), 1.37-1.42 (m, 2H), 2.06-2.10 (m, 2H), 2.47-2.51 (m, 1H), 3.74-4.28 (m, 4H), 4.12-4.28 (m, 1H), 5.04 (s, 1H), 7.66 (t, J=6.6 Hz, 2H), 7.91 (d, J = 8.0 Hz, 2H). MS (EI) *m*/*z*: 327 (M+1), 344.1 (M+18)

### Preparations 16 to 17 were prepared in analogous manner of preparation 15.

| Prreparation No. | IUPAC Name |
|---|---|
| 16 | (4-Cyclopropanesulfonyl-phenyl)-[(S)-(tetrahydro-furan-3-yl)oxy]-acetic acid |
| 17 | (4-Cyclopropanesulfonyl-phenyl)-[(R)-(tetrahydro-furan-3-yl)oxy]-acetic acid |

### Preparation 18: (4-Cyclopropanesulfonyl-phenyl)-[(tetrahydro-pyran-4-yloxy)]-acetic acid

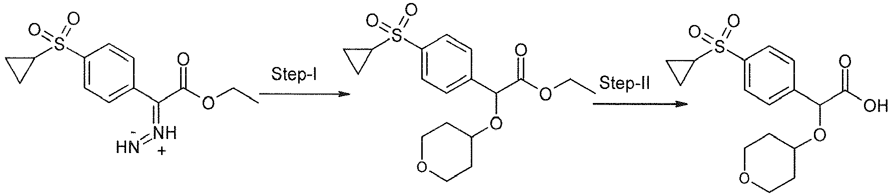

### Step I: (4-Cyclopropanesulfonyl-phenyl)-[(tetrahydro-pyran-4-yloxy)]-acetic acid ethyl ester:

(4-Cyclopropanesulfonyl-phenyl) diazo acetic acid ethyl ester, obtained similarly as described in preparation 13, (5gm, 16.87 mmol) was dissolved in DCM (84 mL) under argon atmosphere. To this solution, 4-hydroxy tetrahydropyran (2.0 mL, 20.24 mmol) was added followed by rhodium(II)acetate dimer (156 mg, 0.35 mmol). Mixture was stirred at 25⁰C for 3 hr. Reaction mixture was diluted with DCM (50 mL), organic layer was washed with water followed by brine solution, dried over anhydrous sodium sulfate, filtered and concentrated under reduced pressure to give a crude product which was purified by column chromatography using 35-40% ethyl acetate in hexane as eluent to provide (4-cyclopropanesulfonyl-phenyl)-[(tetrahydro-pyran-4-yloxy)]-acetic acid ethyl ester (3.5 gm).
¹H NMR (400 MHz, CDCl₃): δ 1.02-1.07 (m, 2H), 1.24 (t, 3H), 1.34-1.37 (m, 2H), 1.65-1.81 (m, 2H), 1.87-1.92 (m, 1H), 1.95-2.01 (m, 1H), 2,43-2.49 (m, 1H), 3.39-3.48 (m, 2H), 3.62-3.68 (m, 1H), 3.91-4.02 (m, 2H) 4.15-4.23 (m, 2H), 5.11 (s,1H), 7.68- (d, J = 8.0 Hz, 2H), 7.90 (d, J = 8.0 Hz, 2H).
MS (EI) *m*/*z*: 369 (M+1), 386.1 (M+18).

### Step II: (4-Cyclopropanesulfonyl-phenyl)-[(tetrahydro-pyran-4-yloxy)]-acetic acid:

To (4-cyclopropanesulfonyl-phenyl)-[(tetrahydro-pyran-4-yloxy)]-acetic acid ethyl ester (23.2 gm, 62.87 mmol) was added a solution of lithium hydroxide (13.0 gm, 314.36 mmol) in water (150 ml) followed by THF (200 ml) and methanol (10 ml) and stirred for 2 hours at 25⁰C. Organic solvents were evaporated from the reaction mixture and aqueous layer was acidified 1N HCl, extracted with ethyl acetate (3X200 ml), organic layer was washed with brine solution, dried over anhydrous sodium sulfate, and washed concentrated under reduced pressure to provide (4-cyclopropanesulfonyl-phenyl)-[(tetrahydro-pyran-4-yloxy)]-acetic acid (18.49 gm).
¹H NMR (400 MHz, CDCl₃): δ 1.02-1.06 (m, 2H), 1.07-1.15 (m, 2H) 1.23-1.56 (m, 2H), 1.83-1.94 (m, 2H), 2.83-2.89 (m, 1H), 3.28-3.35 (m, 2H), 3.69-3.65 (m, 1H), 3.74-3.85 (m, 2H) 5.26 (s, 1H), 7.69 (d, J = 8.0 Hz, 2H), 7.90 (d, J = 8.0 Hz, 2H).
MS (EI) *m*/*z*: 341.0 (M+1), 358.0 (M+18).

### Preparation 19: (4-Cyclopropanesulfonyl-phenyl)-(2,4-difluoro-phenoxy)-acetic acid:

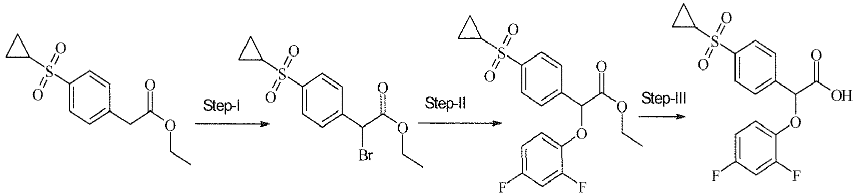

### Step I: Synthesis of ethyl 2-bromo-2-(4-cyclopropanesulfonylphenyl)acetate, (A1-I):

To a solution of ethyl 2-(4-cyclopropanesulfonylphenyl) acetate, obtained according to WO2004072031, in carbon tetrachloride, was added N-Bromosuccinimide followed by catalytic amount of benzoyl peroxide. The reaction mixture was then refluxed for 4 hours. After completion, the reaction mixture was cool to room temperature, filtered; the filtrate was washed with water followed by brine solution, dried over anhydrous sodium sulfate, solvent was removed under reduced pressure to provide title compound as a gummy mass which was used for the next step with out any further purification.
¹H NMR (400 MHz, CDCl₃): δ 1.04-1.10 (m, 2H), 1.31 (t, J = 7.2 Hz, 3H), 1.36-1.40 (m, 2H), 2.45-2.49 (m, 1H), 4.23-4.32 (m, 2H), 5.37 (s, 1H), 7.75 (d, J = 8.4 Hz, 2H), 7.91 (d, J = 8.4 Hz, 2H).
MS (EI) *m*/*z*: 346.9 (M+1)

### Step II: Synthesis of ethyl 2-(4-cyclopropanesulfonylphenyl)-2-(2,4-difluoro-phenoxy)acetate:

To a solution of 2,4-difluorophenol in acetonitrile, was added potassium carbonate followed by bromo-(4-cyclopropanesulfonyl-phenyl)-acetic acid ethyl ester (obtained in step I) and stirred for 10-14 hours. After completion, the reaction mixture was filtered; the filtrate was concentrated to obtain a residue which was dissolved in ethyl acetate; this solution was washed with water followed by brine solution, dried over anhydrous sodium sulfate, solvent was removed under reduced pressure to provide a crude product which was purified by column chromatography to provide the title compound.
¹H NMR (400 MHz, CDCl₃): δ 1.03-1.10 (m, 2H), 1.24 (t, J = 7.2 Hz, 3H), 1.36-1.41 (m, 2H), 2.46-2.50 (m, 1H), 4.19-4.26 (m, 2H), 5.66 (s, 1H), 6.77-6.81 (m, 1H), 6.88-6.93 (m, 1 H), 6.97-7.03 (m, 1H), 7.80 (d, J = 8.4 Hz, 2H), 7.96 (d, J = 8.4 Hz, 2H).
MS (EI) *m*/*z*: 397 (M+1).

### Step III: Synthesis of 2-(4-cyclopropanesulfonylphenyl)-2-(2,4-difluorophenoxy)acetic acid:

To a stirred solution of **A1-II** (obtained in step II) in tetrahydrofuran, was added aqueous solution of NaOH at room temperature and stirred for 4 h. After completion of reaction, THF was removed under reduced pressure. The residue was washed with diethyl ether. The aqueous layer was acidified using HCl and was extracted with ethyl acetate. The organic layer was washed with brine and dried over anhydrous sodium sulfate; solvent was evaporated under reduced pressure to obtain the title compound
¹H NMR (400 MHz, CDCl₃): δ 1.06-1.09 (m, 2H), 1.36-1.39 (m, 2H), 2.46-2.49 (m, 1H), 5.69 (s, 1H), 6.78-6.79 (m, 1H), 6.91-6.99 (m, 2H), 7.80 (d, J = 8.0 Hz, 2H), 7.96 (d, J = 8.4 Hz, 2H).
MS (EI) *m*/*z*: 369 (M+1)

### Preparations 20 to 32 were prepared in analogous manner of preparation 19.

| Prreparation No. | IUPAC Name |
|---|---|
| 20 | (4-Chloro-phenoxy)-(4-cyclopropanesulfonyl-phenyl)-acetic acid |
| 21 | (4-Cyclopropanesulfonyl-phenyl)-(4-fluoro-phenoxy)-acetic acid |
| 22 | (4-Cyclopropanesulfonyl-phenyl)-(3-fluoro-phenoxyl-acetic acid |
| 23 | (4-Cyclopropanesulfonyl-phenyl)-(4-methoxy-phenoxy)-acetic acid |
| 24 | (4-Cyclopropanesulfonyl-phenyl)-(3-methoxy-phenoxy)-acetic acid |
| 25 | (4-Cyclopropanesulfonyl-phenyl)-(4-trifluoromethoxy-phenoxy)-acetic acid |
| 26 | (4-Cyclopropanesulfonyl-phenyl)-(4-trifluoromethyl-phenoxy)-acetic acid |
| 27 | (4-Cyclopropanesulfonyl-phenyl)-(3-trifluoromethyl-phenoxy)-acetic acid |
| 28 | (4-Cyclopropanesulfonyl-phenyl)-p-tolyloxy-acetic acid |
| 29 | (4-Cyclopropanesulfonyl-phenyl)-phenoxy-acetic acid |
| 20 | (4-Cyclopropanesulfonyl-phenyl)-(3,4-dichloro-phenoxy)-acetic acid |
| 31 | (4-Cyclopropancsulfonyl-phenyl)-(3,4-difluoro-phenoxy)-acetic acid |
| 32 | (3-Chloro-4-fluoro-phenoxy)-(4-cyclopropanesulfonyl-phenyl)-acetic acid |

**Intermediates-33-40** were either obtained from commercial source or prepared as per literature method.

| | | | |
|---|---|---|---|
| 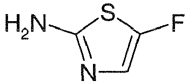 | 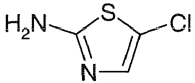 | 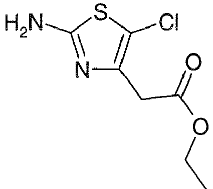 | 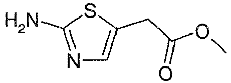 |
| Commercial Source 33-1 | Commercial Source 33-2 | WO2009047798 33-3 | WO2004058752 33-4 |
| 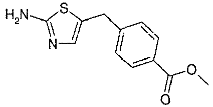 | 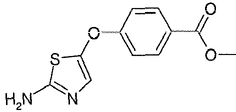 | 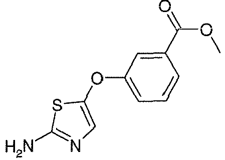 | 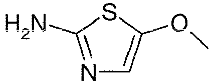 |
| Chem.Pharm.Bull 36, 2969 33-5 | WO2008005914 33-6 | WO2008005914 33-7 | WO2007099317 33-8 |
| 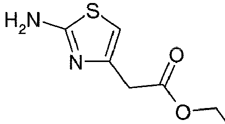 | 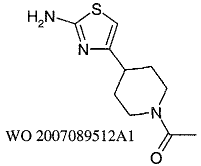 | 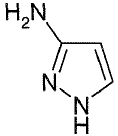 | 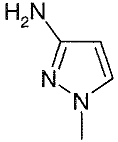 |
| Commercial Source 33-9 | 33-10 | Commercial Source 34-1 | Commercial Sorce 34-2 |
| 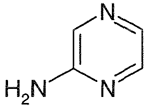 | 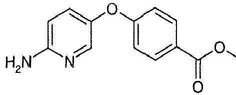 | 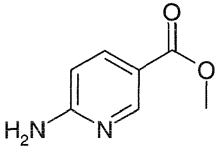 | 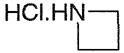 |
| Commercial Source 35 | J.Med.Chem. 2007, 50(8), 4464 36-1 | Commercial Source 36-2 | Commercial Source37 |
| Aq. NH₄OH 38 | 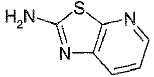 | 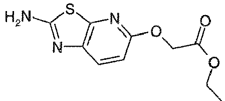 | 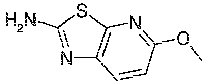 |
| | J.Het.Chem. 1977, 14, 129 39-1 | WO2007007886 39-2 | WO2007041366 39-3 |
| 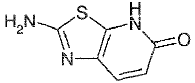 | 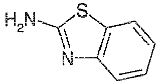 | 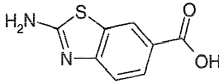 | 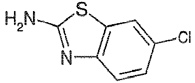 |
| WO2008084873 39-4 | Commercial Source 40-1 | WO2007113644 40-2 | Commercial Source 40-3 |

### Preparation 41: 4-(2-Amino-thiazol-5-yloxy)-3-fluoro-benzoic acid ethyl ester:

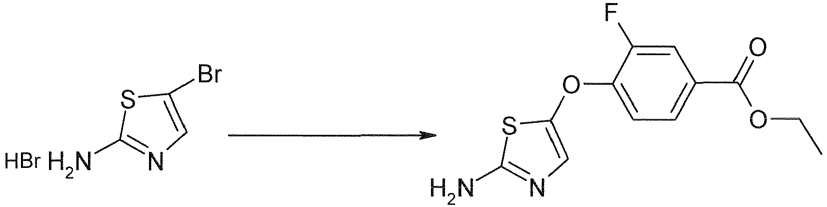

To a solution of 5-Bromothiazole-2-amine hydrobromide (1.55 g, 5.97 mmol) in acetone (100 mL) was added 3-fluoro-4-hydroxy benzoic acid ethyl ester (1.1 g, 5.97 mmol) and cesium carbonate (3.89 g, 11.94 mmol) under argon atmosphere and refluxed for 5 hrs. The reaction mixture was cooled to room temperature and filtered; the filtrate was concentrated under reduced pressure. The residue was partitioned in between ethyl acetate and water. The layers were separated, the organic layer was washed with cold 2 N NaOH (10 mL), brine and dried over anhydrous sodium sulfate, filtered and concentrated under reduced pressure to provide 4-(2-amino-thiazol-5-yloxy)-3-fluoro-benzoic acid ethyl ester (0.68 gm).
¹H NMR (400 MHz, CDCl₃): δ 1.39 (t, 3H), 4.37 (q, 2H), 6.83 (s, 1H), 7.13-7.17 (t, 1H), 7.76-7.84 (m, 2H). MS (EI) *m*/*z*: 282.9 (M+1).

### Preparations 42 to 47 were prepared in analogous manner of preparation 41.

| Preparation No. | IUPAC Name |
|---|---|
| 42 | 4-(2-Amino-thiazol-5-yloxy)-2-fluoro-benzoic acid methyl ester |
| 43 | 5-(5-Trifluoromethyl-pyridin-2-yloxy)-thiazol-2-ylamine |
| 44 | [4-(2-Amino-thiazol-5-yloxy)-phenyl]-acetic acid methyl ester |
| 45 | 4-(2-Amino-thiazol-5-yloxy)-benzonitrile |
| 46 | 6-(2-Amino-thiazol-5-yloxy)-nicotinic acid methyl ester |
| 47 | 4-(2-Amino-thiazol-5-yloxy)-benzaldehyde |

### Preparation 48: 5-Morpholin-4-yl-thiazol-2-ylamine:

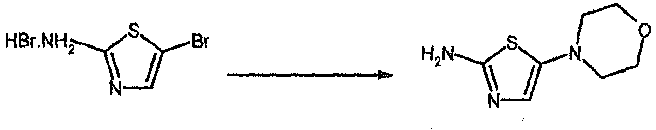

To a mixture of 2-amino-5-bromothiazole monohydrobromide (2 gm, 7.69 mmol) and powdered potassium carbonate (2.1 gm, 15.38 mmol) in DMF (20 mL) was added morpholine (1.34 ml, 15.38 mmol) under argon atmosphere and heated at 60 °C for 3 hr. Reaction mixture was cooled to rt and poured over ice cold water (100 ml), extracted with ethylacetate (3X100ml), washed with brine solution, dried over anhydrous sodium sulfate, filtered and concentrated under reduced pressure. To the residue, diisopropyl ether (50 mL) was added, after stirring for 30 minutes product precipitated out, which was filtered and dried under vacuum to provide 5-morpholin-4-yl-thiazol-2-ylamine (0.95 gm).
¹H NMR (400 MHz, DMSO-d6): δ 2.80-2.82 (m, 4H), 3.66-3.68 (m, 4H), 6.30 (s, 1H)), 6.49 (bs, 2H). MS (EI) m/z: 186 (M + 1).

### Preparation 49 and 50 were prepared in analogous manner of preparation 48.

| Preparation No. | IUPAC Name |
|---|---|
| 49 | 1-(2-Amino-thiaxol-5-yl)-piperidine-4-carboxylic acid ethyl ester |
| 50 | 5-Pyrazol-1-yl-thiazol-2-ylamine |

### Preparation 51 :4-(3-Amino-pyrazol-1-ylmethyl)-benzoic acid methyl ester:

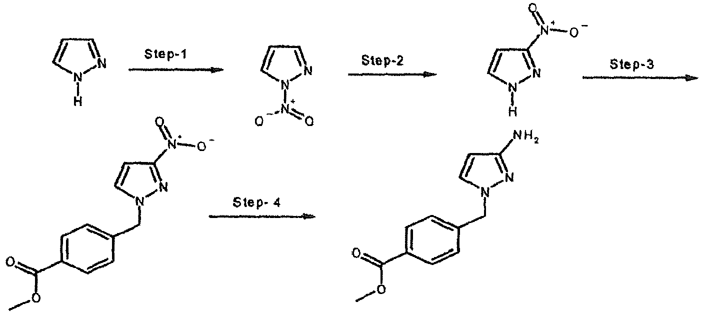

### Step-1: 1-Nitro-1H-pyrazole:

Obtained as described in WO2007/99317 A1

### Step-2: 3-Nitro-1H-pyrazole:

Obtained as described in WO2008/21032 A1

### Step-3: 4-(3-Nitro-pyrazol-1-ylmethyl)-benzoic acid methyl ester:

Obtained as described in US2008/146625 A1

### Step-4: 4-(3-Amino-pyrazol-1-ylmethyl)-benzoic acid methyl ester:

4-(3-Nitro-pyrazol-1-ylmethyl)-benzoic acid methyl ester (530 mg, 2.03 mmol) was dissolved in ethyl acetate (10 ml). Tin(II) chloride dihydrate (2.29 gm, 10.15 mmol) was added, reaction mixture was heated at 70°C. for 1 h. The reaction mixture was cooled to ambient temperature, the pH adjusted to pH 8-9 by addition of aq. saturated sodium carbonate and extracted with ethyl acetate (3X25 ml), dried over anhydrous Na₂SO₄ and concentrated under reduced pressure to provide 4-(3-amino-pyrazol-1-ylmethyl)-benzoic acid methyl ester (480 mg)
¹H NMR (400 MHz, DMSO-d6): δ 3.83(s, 3H), 4.62 (bs, 2H), 5.12 (s, 2H)), 5.43 (d, J = 2.4 Hz, 1H), 7.27 (d, J = 8.4 Hz, 2H), 7.47 (d, J = 2.0 Hz, 1H), 7.90 (d, J = 8.0 Hz, 2H). MS (EI) m/z: 232 (M + 1).

### Preparation 52: (3-Amino-pyrazol-1-yl)-acetic acid methyl ester:

Prepared in an analogous manner of preparation 51.

### Preparation 53: 5-Ethoxy-thiazolo[5,4-b]pyridin-2-ylamine

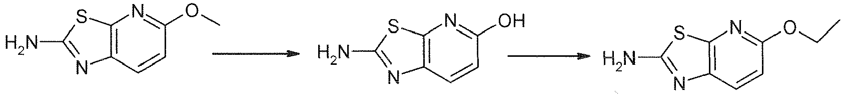

### Step-I: 2-Amino-thiazolo[5,4-b]pyridin-5-ol

Synthesized as described in WO 2007/007886

### Step-II: 5-Ethoxy-thiazolo[5,4-b]pyridin-2-ylamine

To a solution of 2-Amino-thiazolo[5,4-b]pyridin-5-ol (4.7 gm, 28.1 mmol) in Dimethylformamide (50 ml) was added cesium fluoride (12.8 gm, 84.4 mmol) under argon atmosphere. Ethyl iodide (2.7 ml, 33.7 mmol) was added in drop wise manner at room temperature and stirred overnight. Completion of reaction was confirmed by TLC, reaction mixture was diluted with water (100 ml) and extracted with ethyl acetate (100 ml x3),combined organic extracts were washed with water and brine (100 ml each) dried over Na₂SO₄, filtered and concentrated under vaccum to afford ethoxy-thiazolo[5,4-b]pyridin-2-ylamine (2.1 gm).
¹H NMR (400 MHz,DMSO- d₆):δ 1.30 (t, J = 7.2 Hz, 3H), 4.23 (q, J = 7.2, Hz, 2H), 6.65 (d, J = 8.8 Hz, 1H), 7.42 (bs, 2H), 7.59 (d, J= 8.8 Hz, 1H). MS (EI) *m*/*z*: 195.90 (M + 1).

### Preparation 54: 5-iso-propoxy-thiazolo[5,4-b]pyridin-2-ylamine:

Prepared in analogous manner of preparation 53.

### Preparation 55: 5-Vinyl-thiazol-2-ylamine:

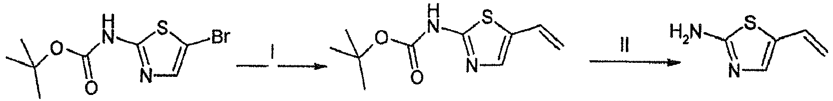

### Step I: (5-Vinyl-thiazol-2-yl)-carbamic acid tert-t-butyl ester:

(5-Bromo-thiazol-2-yl)-carbamic acid tert-butyl ester (0.200 gm, 0.71 mmol), tetrakis(triphenylphospine)palladium (0.042 gm, 0.036 mmol), lithium chloride (0.91 gm, 2.14 mmol), and tributyl (vinyl)stannate (0. 68 gm, 2.14 mmol) were taken in 8 ml of THF and 8 ml of DMF and reflux for 2 hrs. After completion of reaction THF was removed under reduced pressure on rotary evaporator and the residue was partitioned between ethyl acetate (20 ml) and water (20 ml). The layers were separated. Aq. Layer was extracted with ethyl acetate (2x20 ml). The combined organic layer was washed with brine and dried over anhydrous sodium sulfate filtered and concentrated under reduced pressure to get the crude product which was purified by preparative TLC to get the pure product (5-Vinyl-thiazol-2-yl)-carbamic acid tert-butyl ester (0.137 gm,).
¹H NMR- (CDCl₃, 400 MHz): δ 1.57 (s, 9H), 5.12 (d, J = 10.8 Hz, 1H), 5.41 (d, J =17.2 Hz, 1H), 6.71 (dd, J = 17.6, 11 Hz, 1H), 7.19 (s, 1H), 11.55 (bs, 1H).
MS (EI) *m*/*z:* 227.1 (M + 1).

### Step II: 5-Vinyl-thiazol-2-ylamine:

(5-Vinyl-thiazol-2-yl)-carbamic acid tert-butyl ester (0.130 gm, 0.574 mmol) was taken in 15 ml of DCM, to it trifluoroacetic acid (1 ml, 13.46 mmol) was added and the reaction was stirred for room temperature for over night. Solvent was removed under reduced pressure and the residue was partitioned between aq. saturated NaHCO₃ (20 ml) and ethyl acetate (20 ml). The layers were separated. Aq. layer was extracted with ethyl acetate (2X20 ml). The combined organic layer was washed with brine and dried over anhydrous sodium sulfate filtered and concentrated under reduced pressure to get the 5-Vinyl-thiazol-2-ylamine (0.054 gm).
¹H NMR- (CDCl₃, 400 MHz): δ 5.02(d, J = 11 Hz, 1H), 5.16(d, J = 17.4 Hz, 1H), 6.62-6.68 (dd, J = 16.8, 10.8 Hz, 1H), 6.95(s, 1H).
MS (EI) *m*/*z*: 145.2 (M + 18).

### Preparation 56: 4-Vinyl-thiazol-2-ylamine:

Prepared in analogous manner of preparation 55

### Example A1: 4-{2-[2-(4-Cyclopropanesulfonyl-phenyl)-2-(tetrahydro-pyran-4-yloxy)-acetylamino]-thiazol-5-yloxy}-benzoic acid methyl ester

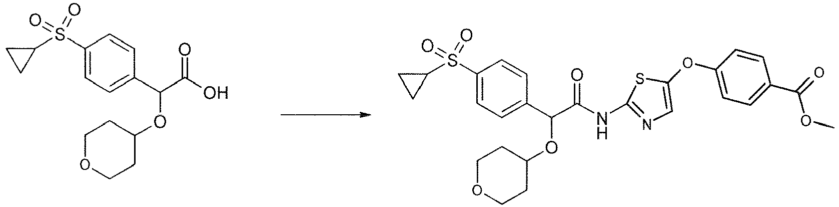

### Procedure-A:

To a mixture of (4-Cyclopropanesulfonyl-phenyl)-[(tetrahydro-pyran-4-yloxy)]-acetic acid (Preparation 18) (0.5g, 1.47 mmol), 4-(2-Amino-thiazol-5-yloxy)-benzoic acid methyl ester (0.441g, 1.76 mmol), HOBt (0.237g, 1.76 mmol), and EDCI (0.337g, 1.76 mmol) in DMF (20 mL), was added *N*-methyl morpholine (0.178g, 1.76 mmol). The resulting mixture was stirred at room temperature overnight followed by dilution with methylene chloride. The reaction mixture was poured into water; organic layer was washed with water, brine, dried over sodium sulfate, and the organic solvent evaporated to get a residue which was purified by preparative TLC or column chromatoghaphy to provide the title compound.

Compound (A1) can also be prepared using procedure-B or procedure-C

**Procedure-B:** (4-Cyclopropanesulfonyl-phenyl)-[(tetrahydro-pyran-4-yloxy)]-acetic acid (Preparation 18) was dissolved in DCM. To this solution was added DMF and cooled to 0 °C, followed by the addition of oxalyl chloride under stirring. To this mixture, a solution of 4-(2-Amino-thiazol-5-yloxy)-benzoic acid methyl ester and pyridine in DCM was added drop wise at 0°C and was stirred further for 4 h. at room temperature. The reaction mixture was poured into 1N aqueous HCl under stirring, organic layer was again washed with 1N HCl, followed by 5% brine, dried over anhydrous sodium sulfate, solvent was removed under reduced pressure to get the crude compound which was purified by flash chromatography to get the title product.

**Procedure-C:** (4-Cyclopropanesulfonyl-phenyl)-[(tetrahydro-pyran-4-yloxy)]-acetic acid (Preparation 18) (1 mmol) and HATU (1.5 mmol) was dissolved in DCM. To this solution was added DIPEA (2 mmol.) and stirred for 15 minutes followed by addition of 4-(2-Amino-thiazol-5-yloxy)-benzoic acid methyl ester (1 mmol.) and continued to stir over night at room temperature. Reaction mixture was diluted with dichloromethane, washed with water, brine, dried over anhydrous sodium sulfate, solvent was removed under reduced pressure to get the crude compound which was purified by flash chromatography to get the title product.
¹H NMR (CDCl₃, 400 MHz): δ - 1.04-1.07 (m, 2H), 1.34-1.37 (m, 2H), 1.68-1.79 (m, 2H), 1.85-1.90 (m, 1H), 2.02-2.10 (m, 1H), 2.42-2.47 (m, 1H), 3.34-3.43 (m, 2H), 3.65-3.70 (m, 1H), 3.90 (s, 3H), 3.93-4.02 (m, 2H), 5.21 (s, 1H), 7.06 (d, J = 7.2Hz, 2H), 7.15 (s, 1H), 7.68 (d, J = 7.6Hz, 2H), 7.93 (d, J = 8.4 Hz, 2H), 7.99 (d, J = 7.2Hz, 2H). MS (EI) m/z: 572.8 (M + 1).

### Example A2: 4-(2-{2-(4-Cyclopropanesulfonyl-phenyl)-2-[(R)-(tetrahydro-furan-3-yl)oxy]-acetylamino}-thiazol-5-yloxy)-benzoic acid methyl ester

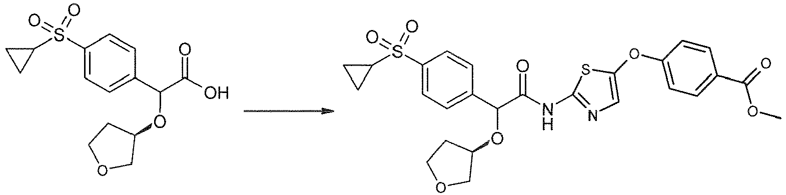

The compound of example A2 was obtained by similar method described in example A1 using (4-Cyclopropanesulfonyl-phenyl)-[(R)-(tetrahydro-furan-3-yl)oxy]-acetic acid (preparation 17) (0.150 gm, 0.46 mmol), 4-(2-Amino-thiazol-5-yloxy)-benzoicacid methyl ester (0.140 gm, 0.55 mmol), HOBt (0.075 gm, 0.55 mmol), and EDCI (0.106 gm 0.55 mmol), *N*-methyl morpholine (0.055 gm, 0.55 mmol) in DMF (10 mL) to provide the title compound (185 mg).
¹H NMR (CDCl₃, 400 MHz): δ - 1.04-1.07 (m, 2H), 1.34-1.37 (m, 2H), 1.68-1.79 (m, 2H), 1.85-1.90 (m, 1H), 2.02-2.10 (m, 1H), 2.42-2.47 (m, 1H), 3.34-3.43 (m, 2H), 3.65-3.70 (m, 1H), 3.90 (s, 3H), 3.93-4.02 (m, 2H), 5.21 (s, 1H), 7.06 (d, J = 7.2 Hz, 2H), 7.15 (s, 1H), 7.68 (d, J = 7.6 Hz, 2H), 7.93 (d, J = 8.4 Hz, 2H), 7.99 (d, J = 7.2 Hz, 2H). MS (EI) m/z: 572.8 (M + 1).

### Example A3: 2-(2,4-Difluoro-phenoxy)-2-[4-(piperidine-1-sulfonyl)-phenyl]-N-thiazolo[5,4-b]pyridin-2-yl-acetamide (Reference)

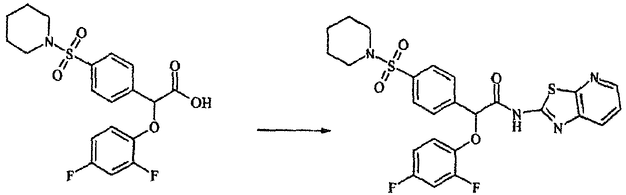

The compound of example A3 was obtained by similar method described in example A1 using (2,4-Difluoro-phenoxy)-[4-(piperidine-1-sulfonyl)-phenyl]-acetic acid (Preparation 2) (0.1 gm, 0.27 mmol), thiazolo[5,4-b]pyridin-2-ylamine (0.042 gm, 0.33 mmol), HOBt (0.044 gm, 0.33 mmol), and EDCI (0.062 gm, 0.33 mmol), *N*-methyl morpholine (0.034 gm, 0.33 mmol) in DMF (10 mL) to provide the title compound (0.175 gm).
¹H NMR (400 MHz, ,DMSO- d₆):- δ 1.34 (bs, 2H), 1.53 (bs, 4H), 2.90 (bs, 4H), 6.27 (s, 1H), 7.02-7.06 (m, 1H), 7.14-7.20 (m, 1H), 7.36-7.41 (m, 1H), 7.50-7.53 (m, 1H), 7.84 (d, J = 8.0 Hz, 2H), 7.91 (d, J = 8.0 Hz, 2H), 8.14 (d, J = 8.0 Hz, 1H), 8.49 (d, J = 4.4 Hz, 1H).
MS (EI) *m*/*z*: 545.0 (M + 1).

### Example A4: N-(5-Fluoro-thiazol-2-yl)-2-[4-(morpholine-4-sulfonyl)-phenyl]-2-(tetrahydro-pyran-4-yloxy)-acetamide (Reference)

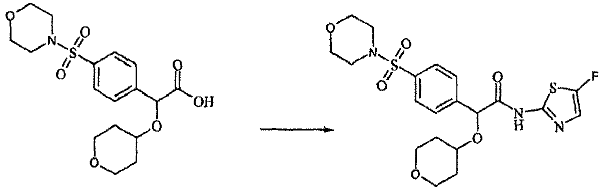

The compound of example A4 was obtained by similar method described in example A1 using [4-(Morpholine-4-sulfonyl)-phenyl]-[(tetrahydro-pyran-4-yloxy)]-acetic acid (preparation 14)(0.1gm, 0.25 mmol), 5-Fluoro-thiazol-2-ylamine hydrochloride (0.048 gm, 0.31 mmol), HOBt (0.042 gm, 0.31 mmol), and EDCI (0.060 gm, 0.31 mmol), *N-*methyl morpholine (0.4 ml, 0.64 mmol) in DMF (5 ml) to provide the title compound (10 mg).
¹H NMR (400 MHz, CDCl3):- δ 1.70-1.77 (m, 2H), 1.86-1.89 (m, 1H), 2.01-2.05 (m, 1H), 2.64-3.03 (m, 4H), 3.36-3.43 (m, 2H), 3.65-3.71 (m, 1H), 3.74-3.76 (m, 4H), 3.94-4.03 (m, 2H), 5.20 (s, 1H), 7.06 (d, J = 2.4 Hz, 1H), 7.68 (d, J = 8.4 Hz, 2H), 7.79 (d, J = 8.4 Hz, 2H).
MS (EI) *m*/*z*: 486.2 (M + 1).

### Example A5: 2-(4-Cyclopropanesulfonyl-phenyl)-2-(tetrahydro-pyran-4-yloxy)-N-thiazolo[5,4-b]pyridin-2-yl-acetamide

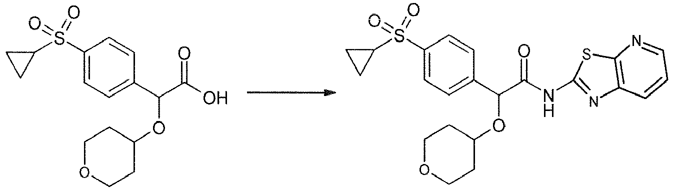

The compound of example A5 was obtained by similar method described in example A1 using (4-cyclopropanesulfonyl-phenyl)-[(tetrahydro-pyran-4-yloxy)]-acetic acid (Preparation 14) (0.2 gm, 0.58 mmol), thiazolo[5,4-b]pyridin-2-ylamine (0.133 gm, 0.88 mmol), HOBt (0.119 gm,0.88 mmol), and EDCI (0.168 gm,0.88 mmol), *N*-methyl morpholine (0.178 gm, 0.88 mmol) in DMF (10 mL) to provide the title compound (0.175 gm).
¹H NMR (400 MHz, DMSO- d₆):- δ 1.00-1.04 (m, 2H), 1.05-1.15 (m, 2H), 1.46-1.61 (m, 2H), 1.62-1.96 (m, 2H), 2.83-2.86 (m, 1H), 3.28-3.34 (m, 2H), 3.65-3.76 (m, 1H), 3.78-3.85 (m, 2H), 5.55 (s, 1H), 7.48 (dd, J = 4.4 Hz, J = 8.0 Hz, 1H), 7.79 (d, J = 8.4 Hz, 2H), 7.92 (d, J = 8.4 Hz, 2H), 8.12 (d, J = 8.0 Hz, 1H), 8.46(dd, J = 1.6 Hz, J = 4.8 Hz, 1H), 12.80 (s, 1H).
MS (EI) *m*/*z*: 474.20 (M + 1).

### Example A6: 2-(4-Cyclopropanesulfonyl-phenyl)-N-(5-methoxy-thiazolo[5,4-b]pyridin-2-yl)-2-'tetrahydro-pyran-4-yloxy)-acetamide

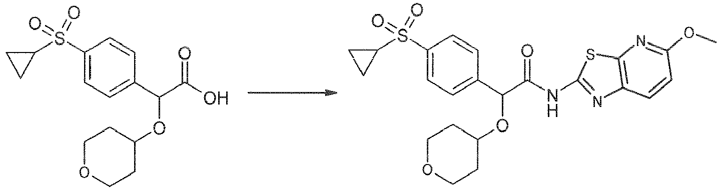

The compound of example A6 was obtained by similar method described in example A1 using (4-cyclopropanesulfonyl-phenyl)-[(tetrahydro-pyran-4-yloxy)]-acetic acid (Preparation 18) (0.5 gm,1.32 mmol), 5-methoxy-thiazolo[5,4-b]pyridin-2-ylamine (0.286 gm, 1.59 mmol), HOBt (0.267 gm,1.98 mmol), and EDCI (0.378 gm,1.98 mmol), *N*-methyl morpholine (0.4 gm, 3.97 mmol) in DMF (10 mL) to provide the title compound (0.280 gm).
¹H NMR (400 MHz, ,DMSO- d₆):- δ 1.02-1.06 (m, 2H), 1.11-1.14 (m, 2H), 1.49-1.63 (m, 2H), 1.85-1.99 (m, 2H), 2.82-2.89 (m, 1H), 3.29-3.36 (m, 2H), 3.65-3.72 (m, 1H), 3.78-3.86 (m, 2H), 3.90 (s, 3H), 5.54 (s, 1H), 6.93 (d, J = 8.8 Hz, 1H), 7.80 (d, J = 8.8 Hz, 2H), 7.94 (d, J = 8.4 Hz, 2H), 8.06 (d, J = 8.8 Hz, 1H), 12.70 (s, 1H).
MS (EI) *m*/*z*: 504.10 (M + 1).

### Example A7: 2-(4-Cyclopropanesulfonyl-phenyl)-N-(5-ethoxy-thiazolo[5,4-b]pyridin-2-yl)-2-(tetrahydro-pyran-4-yloxy)-acetamide

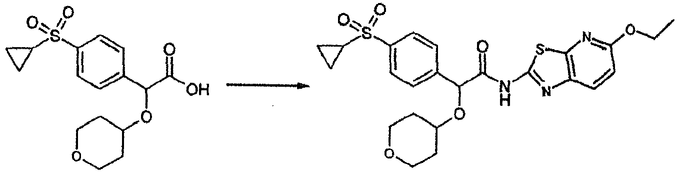

The compound of example A7 was obtained by similar method described in example A1 using (4-cyclopropanesulfonyl-phenyl)-[(tetrahydro-pyran-4-yloxy)]-acetic acid (Preparation 18) (0.25 gm, 0.73 mmol), 5- ethoxy-thiazolo[5,4-b]pyridin-2-ylamine (0.157 gm, 0.80 mmol), HOBt (0.148 gm, 1.10 mmol), and EDCI (0.210 gm, 1.10 mmol), *N*-methyl morpholine (0.22 gm, 2.20 mmol) in DMF (5 mL) to provide the title compound (0.140 gm).
¹H NMR (400 MHz, ,DMSO- d₆):- δ 1.00-1.04 (m, 2H), 1.05-1.13 (m, 2H), 1.35 (t, J = 7.2 Hz, 3H), 1.50-1.60 (m, 2H), 1.85-1.97 (m, 2H), 2.84-2.89 (m, 1H), 3.29-3.34 (m, 2H), 3.65-3.71 (m, 1H), 3.78-3.86 (m, 2H), 4.33 (q, J = 7.2 Hz, 2H), 5.54 (s, 1H), 6.90 (d, J = 8.8 Hz, 1H), 7.80 (d, J = 8.4 Hz, 2H), 7.94 (d, J = 8.0 Hz, 2H), 8.05 (d, J = 8.8 Hz, 1H), 12.70 (s, 1H). MS (EI) *m*/*z*: 518.00 (M + 1).

### Example A8: 2-(4-Cyclopropanesulfonyl-phenyl)-N-(5-isopropoxy-thiazolo[5,4-b]pyridin-2-yl)-2-(tetrahydro-pyran-4-yloxy)-acetamide

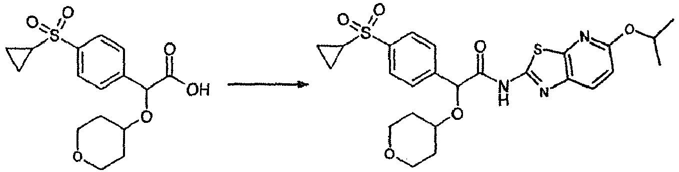

The compound of example A8 was obtained by similar method described in example A1 using (4-cyclopropanesulfonyl-phenyl)-[(tetrahydro-pyran-4-yloxy)]-acetic acid (Preparation 18) (0.100 gm, 0.29 mmol), 5-*iso*-propoxy-thiazolo[5,4-b]pyridin-2-ylamine (0.067 gm, 0.32 mmol), HOBt (0.047 gm, 0.35 mmol), and EDCI (0.067 gm, 0.35 mmol), *N*-methyl morpholine (0.065 gm, 0.58 mmol) in DMF (5 mL) to provide the title compound (0.060 gm).
¹H NMR (DMSO-d₆, 400 MHz): δ 1.01-1.10 (m, 2H), 1.11-1.18 (m, 2H), 1.30 (d, 6H), 1.50-1.60 (m 2H), 1.85-1.96 (m, 2H), 2.70-2.84 (m, 1H), 3.35-3.40 (m, 2H), 3.61-3.72 (m, 1H), 3.79-3.85 (m, 2H), 5.24-5.27 (m, 1H), 5.54 (s, 1H), 6.84 (d, J = 8.8 Hz, 1H), 7.80 (d, J = 8.4 Hz, 2H), 7.94 (d, J = 8.4 Hz, 2H), 8.03 (d, J = 8.8 Hz, 1H).
MS (EI) m/z: 532.2 (M + 1).

### Example (A9) 4-{2-[2-(4-Cyclopropanesulfonyl-phenyl)-2-(2,4-difluoro-phenoxy)-acetylamino]-thiazol-5-yloxy}-benzoic acid methyl ester (Reference):

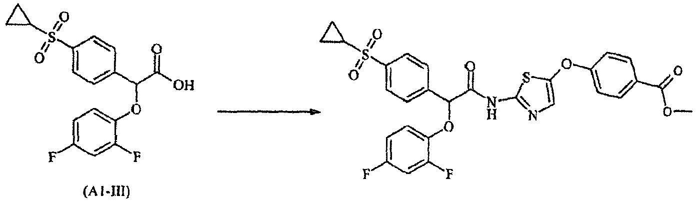

To a mixture of 2-(4-cyclopropanesulfonylphenyl)-2-(2,4-difluorophenoxy)acetic acid (Preparation 19) (2 gm, 5.43 mmol) & HATU (3.1 gm , 8.14 mmol) in anhydrous DCM (50 mL) was added TEA (1.65 gm, 16.3 mmol)& reaction mixture was kept on stirring at room temperature for 0.5 hours. To this solution was added 4-(2-Amino-thiazol-5-yloxy)-benzoic acid methyl ester (1.36 gm, 5.43mmol) and stirring was continued for another 2 hrs. The reaction mixture was quenched with 1N HCl & diluted with DCM. The organic layer was separated, washed with water, brine and dried over sodium sulfate filtered and concentrated under reduced pressure to get a residue which was purified by column chromatography using 50-60% ethylacetate in hexane as eluent to provide the title compound (1.7 gm).
1H NMR- (CDCl3), δ 1.05-1.19 (m, 4H), 2.88 (m, 1H), 3.83 (s, 3H),6.2 (s, 1H), 7.05-7.40 (m, 6H), 7.88-8.01 (m, 5H), 12.97 (s, 1H). MS (EI) *m*/*z*: 601 (M+1)

### Examples A10 to A120 were prepared in analogues manner of examples A1-A9 from the appropriate intermediate that are available commercially or synthesized as above.

| Example No. | **MS (EI)m/z: (M + 1).** | IUPAC Name |
|---|---|---|
| A10 * | 512.0 | 2-(2,4-Difluoro-phenoxy)-N-(5-fluoro-thiazol-2-yl)-2-[4-(piperidine-1-sulfonyl)-phenyl]-acetamide |
| A11 * | 528.0 | N-(5-Chloro-thiazol-2-yl)-2-(2,4-difluoro-phenoxy)-2-[4-(piperidine-1-sulfanyl)-phenyl]-acetamide |
| A12 * | 512.0 | 2-(2,4-Difluoro-phenoxy)-N-(5-fluoro-thiazol-2-yl)-2-(3-(piperidine-1-sulfonyl)-phenyl]-acetamide |
| A13 * | 527.9 | N-(5-Chloro-thiazol-2-yl)-2-(2,4-difluoro-phenoxy)-2-[3-(piperidine-1-sulfonyl)-phenyl]-acetamide |
| A14 * | 498 | 2-(2,4-Difluoro-phenoxy)-N-(5-fluoro-thiazol-2-yl)-2-[3-(pyrrolidine-1-sulfonyl)-phenyl]-acetamide |
| A15 * | 498 | 2-(2,4-Difluoro-phenoxy)-N-(5-fluoro-thiazol-2-yl)-2-[4-(pyrrolidine-1-sulfonyl)-phenyl]-acetamide |
| A16 * | 514 | N-(5-Chloro-thiazol-2-yl)-2-(2,4-difluoro-phenoxy)-2-[3-(pyrrolidine-1-sulfonyl)-phenyl]-acetamide |
| A17 * | 514 | N-(5-Chloro-thiazol-2-yl)-2-(2,4-difluoro-phenoxy)-2-[4-(pyrrolidine-1-sulfonyl)-phenyl]-acetamide |
| A18 * | 499.90 | 2-[3-(Azetidine-1-sulfonyl)-phenyl]-N-(5-chloro-thiazol-2-yl)-2-(2,4-difluoro-phenoxy)-acetamide |
| A19 * | 500.00 | 2-[4-(Azetidine-1-sulfonyl)-phenyl]-N-(5-chloro-thiazol-2-yl)-2-(2,4-difluoro-phenoxy)-acetamide |
| A20 * | 484.00 | 2-[4-(Azetidine-1-sulfonyl)-phenyl]-2-(2,4-difluoro-phenoxy)-N-(5-fluoro-thiazol-2-yl)-acetamide |
| A21 * | 514.2 | 2-(2,4-Difluoro-phenoxy)-N-(5-fluoro-thiazol-2-yl)-2-[4-(morpholine-4-sulfonyl)-phenyl]-acetamide |
| A22 * | 530.1 | N-(5-Chloro-thiazol-2-yl)-2-(2,4-difluoro-phenoxy)-2-[4-(morpholine-4-sulfonyl)-phenyl]-acetamide |
| A23 * | 477.0 | 2-(2,4-Difluoro-phenoxy)-2-[4-(piperidine-1-sulfonyl)-phenyl]-N-(1H-pyrazol-3-yl)-acetamide |
| A24 * | 477.0 | 2-(4-Chloro-phenoxy)-2-[4-(morpholine-4-sulfonyl)-phenyl]-N-(1H-pyrazol-3-yl)-acetamide |
| A25 * | 475.0 | 2-(4-Chloro-phenoxy)-2-[4-(piperidine-1-sulfonyl)-phenyl]-N-(1H-pyrazo 1-3-yl)-acetamide |
| A26 * | 579.3 | 2-(2,4-Difluoro-phenoxy)-N-(5-morpholin-4-yl-thiaxol-2-yl)-2-[4-(piper idine-1-sulfonyl)-phenyl]-acetamide |
| A27 * | 491.3 | 2-(2,4-Difluoro-phenoxy)-N-(1-methyl-1H-pyrazol-3-yl)-2-[4-(piperidine -1-sulfonyl)-phenyl]-acetamide |
| A28 * | 493.3 | 2-(2,4-Difluoro-phenoxy)-N-(1-methyl-1H-pyrazol-3-yl)-2-[4-(morpholine -4-sulfonyl)-phenyl]-acetamide |
| A29 * | 491.3 | 2-(2,4-Difluoro-phenoxy)-2-[4-(morp holine-4-sulfonyl)-phenyl]-N-pyrazin-2-yl-acetamide |
| A30 * | 565.3 | 2-(2,4-Difluoro-phenoxy)-N-(5-morpholin-4-yl-thiazol-2-yl)-2-[4-(pyrrolidine-1-sulfonyl)-phenyl]-acetamide |
| A31 * | 547.2 | 2-(2,4-Difluoro-phenoxy)-2-[4-(morpholine-4-sulfonyl)-phenyl]-N-thiazolo[5,4-b]pyridin-2-yl-acetamide |
| A32 * | 531.0 | 2-(2,4-Difluoro-phenoxy)-2-[4-(pyrr olidine-1-sulfonyl)-phenyl]-N-thiazolo[5,4-b]pyridin-2-yl-acetamide |
| A33 * | 502.30 | 2-(4-Cyclopropanesulfonyl-phenyl)-2-(2,4-difluoro-phenoxy)-N-thiazolo[5,4-b]pyridin-2-yl-acetamide |
| A34 * | 531.8 | 2-(4-Cyclopropanesulfonyl-phenyl)-2-(2,4-difluoro-phenoxy)-N-(5-methoxy-thiazolo[5,4-b]pyridin-2-yl)-acetamide |
| A35 | 489.00 | 2-(4-Cyclopropanesulfonyl-phenyl)-N-(5-pyrazol-1-yl-thiazol-2-yl)-2-(tetrahydro-pyran-4-yloxy)-acetamide |
| A36 | 508.00 | 2-(4-Cyclopropanesulfonyl-phenyl)-N-(5-morpholin-4-yl-thiazol-2-yl)-2-(tetrahydro-pyran-4-yloxy)-acetamide |
| A37 | 440.90 | 2-(4-Cyclopropanesulfonyl-phenyl)-N-(5-fluoro-thiazol-2-yl)-2-(tetrahydro-pyran-4-yloxy)-acetamide |
| A38 | 420.1 | 2-(4-Cyclopropanesulfonyl-phenyl)-N-(1-methyl-1H-pyrazol-3-yl)-2-(tetrahydro-pyran-4-yloxy)-acetamide |
| A39 | 417.5 | 2-(4-Cyclopropanesulfonyl-phenyl)-N-pyraxin-2-yl-2-(tetrahydropyran-4-yloxy)-acetamide |
| A40 | 540.00 | N-[5-(4-Cyano-phenoxy)-thiazol-2-yl]-2-(4-cyclopropanesulfonyl-phenyl)-2-(tetrahydro-pyran-4-yloxy)-acetamide |
| A41 | 452.9 | 2-(4-Cyclopropanesulfonyl-phenyl)-N-(5-methoxy-thiazol-2-yl)-2-(tetrahydro-pyran-4-yloxy)-acetamide |
| A42 * | NA | (5-Chloro-2-{2-(2,4-difluoro-phenoxy)-2-[4-(piperidine-1-sulfonyl)-phenyl]-acetylamino}-thiazol-4-yl)-acetic acid ethyl ester |
| A43 * | NA | 4-(2-{2-(2,4-Difluoro-phenoxy)-2-[4 -(morpholine-4-sulfonyl)-phenyl]-acetylamino}-thiazol-5-yloxy)-benzoic acid methyl ester |
| A44 * | 616.2 | (5-Chloro-2-{2-(2,4-difluoro-phenoxy)-2-[4-(morpholine-4-sulfonyl)-phenyl]-acetylamino}-thiazol-4-yl)-acetic acid ethyl ester |
| A45 * | NA | 3-(2-(2-(2,4-Difluoro-phenoxy)-2-[4 -(morpholine-4-sulfonyl)-phenyl]-acetylammo}-thiazol-5-yloxy)-benzoic acid methyl ester |
| A46 * | 635.4 | 1-(2-{2-(2,4-Difluoro-phenoxy)-2-[4-(pyrrolidine-1-sulfonyl)-phenyl]-acetylamino}-thiazol-5-yl)-piperidine-4-carboxylic acid ethyl ester |
| A47 * | 630.3 | 4-(2-{2-(2,4-Difluoro-phenoxy)-2-[4-(pyrrolidine-1-sulfonyl)-phenyl]-acetylamino}-thiazol-5-yloxy)-benzoic acid methyl ester |
| A48 * | 630.3 | 3-(2-{2-(2,4-Difluoro-phenoxy)-2-[4-(pyrrolidine-1-sulfonyl)-phenyl]-acetylamino}-thiazol-5-yloxy)-benzoic acid methyl ester |
| A49 * | 600 | (5-Chloro-2-{2-(2,4-difluoro-phenoxy)-2-[3-(pyrrolidine-1-sulfonyl)-phenyl]-acetylamino}-thiazol-4-yl)-acetic acid ethyl ester |
| A50 * | 586.00 | {2-[2-[3-(Azetidine-1-sulfonyl)-phenyl]-2-(2,4-difluoro-phenoxy)-acetylamino]-5-chloro-thiazol-4-yl}-acetic acid ethyl ester |
| A51 * | 586.00 | {2-[2-[4-(Azetidine-1-sulfonyl)-phenyl]-2-(2,4-difluoro-phenoxy)-acetylamino]-5-chloro-thiazol-4-yl}-acetic acid |
| A52 * | NA | (5-Chloro-2-{2-(4-chloro-phenoxy)-2-[4-(piperidine-1-sulfonyl)-phenyl]-acetylamino}-thiazol-4-yl)-acetic acid ethyl ester |
| A53 * | NA | (5-Chloro-2-{2-(4-chloro-2-fluoro-phenoxy)-2-[4-(piperidine-1-sulfonyl )-phenyl]-acetylamino}-thiazol-4-yl)-acetic acid ethyl ester |
| A54* | NA | (5-Chloro-2-{2-(4-chloro-phenoxy)-2-[4-(morpholine-4-sulfonyl)-phenyl]-acetylamino}-thiazol-4-yl)-acetic acid ethyl ester |
| A55 * | NA | 3-(2-{2-(4-Chloro-phenoxy)-2-[4-(piperidine-1-sulfonyl)-phenyl]-acetylamino}-thiazol-5-yloxy)-benzoic acid methyl ester |
| A56 * | NA | 4-(2-{2-(4-Chloro-phenoxy)-2-[4-(pi peridine-1-sulfonyl)-phenyl]-acetylamino}-thiazol-5-yloxy)-benzoic acid methyl ester |
| A57 * | NA | 1-(2-{2-(2,4-Difluoro-phenoxy)-2-[4-(piperidine-1-sulfonyl)-phenyl]-acetylamino}-thiazol-5-yl)-iperidine-4-carboxylic acid ethyl ester |
| A58 * | 634.1 | (5-Chloro-2-{2-(4-chloro-2-fluoro-phenoxy)-2-[4-(morpholine-4-sulfonyl )-phenyl]-acetylamino}-thiazol-4-yl)-acetic acid ethyl eter |
| A59 * | 644.2 | 4-(2-{2-(2,4-Difluoro-phenoxy)-2-[4-(piperidine-1-sulfonyl)-phenyl]-acetylamino}-thiazol-5-yloxy)-benzoic acid methyl ester |
| A60 * | 644.2 | 3-(2-{2-(2,4-Difluoro-phenoxy)-2-[4-(piperidine-1-sulfonyl)-phenyl]-acetylamino}-thiazol-5-yloxy)-benzoic acid methyl ester |
| A61 * | 598.1 | (2-{2-(4-Chloro-2-fluoro-phenoxy)-2-[4-(morpholine-4-sulfonyl)-phenyl]-acetylamino}-thiazol-4-yl)-acetic acid ethyl ester |
| A62 * | 645.30 | 4-(2-{2-(2,4-Difluoro-phenoxy)-2-[4 -(tetrahydro-pyran-4-sulfonyl)-phenyl]-acetylamino}-thiazol-5-yloxy)-benzoicacid methyl ester |
| A63 | 529.1 | {5-Chloro-2-[2-(4-cyclopropanesulfonyl-phenyl)-2-tetrahydrofuran-3-yloxy)-acetylamino]-thiazol-4-yl}-aceticacid ethyl ester |
| A64 | 559.2 | 4-{2-[2-(4-Cyclopropanesulfonyl-phenyl)-2-(tetrahydro-furan-3-yloxy)-acetylamino]-thiazol-5-yloxy}-benzoic acid methyl ester |
| A65 | 528.9 | (5-Chloro-2-{2-(4-cyclopropanesulfonyl-phenyl)-2-[(R)-(tetrahydro-furan-3-yl)oxy]-acetylamino}-thiazol-4-yl)-acetic acid ethyl ester |
| A66 | 558.8 | 4-(2-{2-(4-Cyclopropanesulfonyl-phenyl)-2-[(S)-(tetrahydrofuran-3-yl)oxy]-acetylamino}-thiazol-5-yloxy)-benzoic acid methyl ester |
| A67 | 529.9 | (5-Chloro-2-{2-(4-cyclopropanesulfonyl-phenyl)-2-[(S)-(tetrahydro-furan-3-yl)oxy]-acetylamino}-thiazol-4-yl)-acetic acid ethyl ester |
| A68 * | 604.2 | {2-[2-(4-Cyclopropanesulfonyl-phenyl)-2-(2,4-difluoro-phenoxy)-acetylamino]-thiazolo[5,4-b]pyridin-5-yloxy}-acetic acid ethyl ester |
| A69 | 554.3 | 4-{3-[2-(4-Cyclopropanesulfonyl-phenyl)-2-(tetrahydro-pyran-4-yloxy)-acetylamino]-pyrazol-1-ylmethyl}-benzoic acid methyl ester |
| A70 | 542.90 | {5-Chloro-2-[2-(4-cyclopropanesulfonyl-phenyl)-2-(tetrahydropyran-4-yloxy)-acetylamino]-thiazol-4-yl}-acetic acid ethyl ester |
| A71 | 571 | 3-{2-[2-(4-Cyclopropanesulfonyl-phenyl)-2-(tetrahydro-pyran-4-yloxv)-acetylamino]-thiazol-5-yloxy}-benzoic acid methyl ester |
| A72 | 645.3 | 4-{2-[2-(4-Cyclopropanesulfonyl-phenyl)-2-(tetrahydro-pyran-4-yloxy)-acetylamino]-thiazol-5-yloxy}-2-fluoro-benxoic acid methyl ester |
| A73 | 605.3 | 4-{2-[2-(4-Cyclopropanesulfonyl-phenyl)-2-(tetrahydro-pyran-4-yloxy)-acetylamino]-thiazol-5-yloxy}-3-fluoro-benzoic acid methyl ester |
| A74 | 576.2 | {2-[2-(4-Cyclopropanesulfonyl-phenyl)-2-(tetrahydro-pyran-4-yloxy)-acetylamino]-thiazolo[5,4-b]pyridin-5-yloxy}-acetic acid ethyl ester |
| A75 * | 652 | 4-{2-[2-(4-Cyclopropanesulfonyl-phenyl)-2-(2,4-difluorophenoxy)-acetylamino]-thiazolo[5,4-b]pyridin-5-yloxy}-benzoic acid methyl ester |
| A76 | 624 | 4-{2-[2-(4-Cyclopropanesulfonyl-phenyl)-2-(tetrahydro-pyran-4-yloxy)-acetylamino]-thiazolo[5,4-h]pyridin-5-yloxy}-benzoic acid methyl ester |
| A77 | 502.9 | 2-(4-Cyclopropanesulfonyl-phenyl)-N-(6-methoxy-benzothiazol-2-yl)-2-(tetrahydro-pyran-4-yloxy)-acetamide |
| A78 | 489.9 | 2-(4-Cyclopropanesulfonyl-phenyl)-N-(5-methoxy-thiazolo[5,4-b]pyridin-2-yl)-2-[(R)-(tetrahydro-furan-3-yl)oxy]-acetamide |
| A79 | 540.3 | 4-[(4-Cyclopropanesulfonyl-phenyl)-(5-fluoro-thiazol-2-ylcarbamoyl)-methoxy]-piperidine-1-carboxylic acid tert-butyl ester |
| A80 | 440.2 | 2-(4-Cyclopropanesulfonyl-phenyl)-N-(5-fluoro-thiazol-2-yl)-2-(piperidin-4-yloxy)-acetamide |
| A81 | 601.2 | (4-{2-(2-(4-Cyclopropanesulfonyl-phenyl)-2-(tetrahydro-pyran-4-yloxy)-acetylamino)-thiazol-5-yloxy}-phenyl)-acetic acid methyl ester |
| A82 | 545.1 | 2-[2-(4-Cyclopropanesnlfonyl-phenyl)-2-(tetrahydro-pyran-4-yloxy)-acetyiamino]-benzothiazole-6-carboxylic acid methyl ester |
| A83 | 531.2 | 2-{2-(4-Cyclopropanesulfonyl-phenyl)-2-[(R)-(tetrahydro-furan-3-yl)oxy]-acetylamino}-benzothiazole-6-carboxylic acid methyl ester |
| A84 | 490.1 | 2-(4-Cyclopropanesulfonyl-phenyl)-N-(5-oxo-4,5-dihydrothiazolo[5,4-b]pyridin-2-yl)-2-(tetrahydro-pyran-4-yloxy)-acetamide |
| A85 | 518.2 | 2-(4-Cyclopropanesulfonyl-phenyl)-N-(5-isopropoxy-thiazolo[5,4-b]pyridin-2-yl)-2-[(R)-(tetrahydro-furan-3-yl)oxy]-acetamide |
| A86 * | 484.1 | N-(5-Fluoro-thiazol-2-yl)-2-[4-(piperidine-1-sulfonyl)-phenyl]-2-(tetrahydro-pyran-4-yloxy)-acetamide |
| A87 * | 517.2 | 2-[4-(Piperidine-1-sulfonyl)-phenyl]-2-(tetrahydro-pyran-4-yloxy)-N-thiazolo[5,4-b]pyridin-2-yl-acetamide |
| A88 * | 547.2 | N-(5-Methoxy-thiazolo[5,4-b]pyridin-2-yl)-2-[4-(piperidine-1-sulfonyl)-phenyl]-2-(tetrahydro-pyran-4-yloxy)-acetamide |
| A89 * | 615.0 | (5-Chloro-2-{2-(2,4-difluoro-phenoxy)-2-[4-(tetrahydro-pyran-4-sulfonyl)-phenyl]-acetylamino}-thiazol-4-yl)-acetic acid ethyl ester |
| A90 * | 486.2 | N-(5-Fluoro-thiazol-2-yl)-2-[4-(morpholine-4-sulfonyl)-phenyl]-2-(tetrahydro-pyran-4-yloxy)-acetamide |
| A91 * | 519.2 | 2-[4-(Morpholine-4-sulfonyl)-phenyl]-2-(tetrahydro-pyran-4-yloxy)-N-thiazolo[5,4-b]pyridin-2-yl-acetamide |
| A92 * | 549.2 | N-(5-Methoxy-thiazolo[5,4-b]pyridin-2-yl)-2-[4-(morpholine-4-sulfonyl)-phenyl]-2-(tetrahydro-pyran-4-yloxy)-acetamide |
| A93 | 587.2 | 4-{2-[2-(4-Cyclopropanesulfonyl-phenyl)-2-(tetrahydro-pyran-4-yloxy)-acetylamino]-thiazol-5-yloxy} -2-methyl-benzoic acid methyl ester |
| A94 | 495.2 | 2-[2-(4-Cyclopropanesulfonyl-phenyl)-2-(tetrahydro-pyran-4-yloxy)-acetylamino]-5-methyl-thiazole-4-carboxylic acid methyl ester |
| A95 | 552.1, 554.2 | N-(5-Bromo-thiazolo[5,4-b]pyridin-2-yl)-2-(4-cyclopropanesulfonyl-phenyl)-2-(tetrahydro-pyran-4-yloxy)-ac etamide |
| A96 * | 601.3 | 3-{2-[2-(4-Cyclopropanesulfonyl-phenyl)-2-(2,4-difluorophenoxy)-acetylamino]-thiazol-5-yloxy}-benzoicacid methyl ester |
| A97 * | 598.5 | 4-{2-[2-(4-Chloro-phenoxy)-2-(4-cyclopropanesulfonyl-phenyl)-acetylamino]-thiazol-5-yloxy}-benzoicacid methyl ester |
| A98 * | 583 | 4-{2-[2-(4-Cyclopropanesulfonyl-phenyl)-2-(4-fluoro-phenoxy)-acetylamino]-thiazol-5-yloxy)-benzoicacid methyl ester |
| A99 * | 635.1 | 4-{2-[2-(4-Cyclopropanesulfonyl-phenyl)-2-(3,4-dichlorophenoxy)-acetylamino]-thiazol-5-yloxy}-benzoicacid methyl ester |
| A100 * | 601.2 | 4-{2-[2-(4-Cyclopropanesulfonyl-phenyl)-2-(3,4-difluorophenoxy)-acetylamino]-thiazol-5-yloxy}-benzoic acid methyl ester |
| A101 * | 583.2 | 4-{2-[2-(4-Cyclopropanesulfonyl-phenyl)-2-(3-fluoro-phenoxy)-acetylamino]-thiazol-5-yloxy}-benzoic acid methyl ester |
| A102 * | 601.2 | 4-{2-[2-(3-Chloro-4-fluoro-phenoxy)-2-(4-cyclopropanesulfonylphenyl)-acetylamino]-thiazol-5-yloxy}-benzoic acid methyl ester |
| A103 * | 595.20 | 4-{2-[2-(4-Cyclopropanesulfonyl-phenyl)-2-(4-methoxyphenoxy)-acetylamino]-thiazol-5-yloxy}-benzoic acid methyl ester |
| A104 * | 595.20 | 4-{2-[2-(4-Cyclopropanesulfonyl-phenyl)-2-(3-methoxyphenoxy)-acetylamino]-thiazol-5-yloxy}-benzoic acid ethyl ester |
| A105 * | 633.10 | 4-{2-[2-(4-Cyclopropanesulfonyl-phenyl)-2-(4-trifluoromethylphenoxy)-acetylamino]-thiazol-5-yloxy}-benzoic acid methyl ester |
| A106 * | 633.20 | 4-{2-[2-(4-Cyclopropanesulfonyl-phenyl)-2-(3-trifluoromethylphenoxy)-acetylamino]-thiazol-5-yloxy}-benzoic acid methyl ester |
| A107 * | 649.10 | 4-{2-[2-(4-Cyclopropanesulfonyl-phenyl)-2-(4-trifluoromethoxyphenoxy)-acetylamino]-thiazol-5-yloxy}-benzoic acid methyl ester |
| A108 * | 602.20 | 6-{2-[2-(4-Cyclopropanesulfonyl-phenyl)-2-(2,4-difluorophenoxy)-acetylamino]-thiazol-5-yloxy}-nicotinicacid methyl ester |
| A109 * | 579.2 | 4-{2-[2-(4-Cyclopropanesulfonyl-phenyl)-2-(4-methyl-phenoxy)-acetylamino]-thiazol-5-yloxy}-benzoic acid methyl ester |
| A110 * | 565.2 | 4-{2-[2-(4-Cyclopropanesulfonyl-phenyl)-2-phenoxyacetylamino]-thiazol-5-yloxy}-benzoic acid methyl ester |
| A111 * | 615.1 | (4-{2-[2-(4-Cyclopropanesulfonyl-phenyl)-2-(2,4-difluorophenoxy)-acetylamino]-thiazol-5-yloxy}-phenyl)-acetic acid methyl ester |
| A112 * | 614.8 | 4-{2-[2-(4-Cyclopropanesulfonyl-phenyl)-2-(2,4-difluorophenoxy)-acetylamino]-4-methyl-thiazol-5-yloxy}-benzoic acid methyl ester |
| A113 * | 632.8 | 4-{2-[2-(4-Cyclopropanesulfonyl-phenyl)-2-(2,4-difluorophenoxy)-acetylamino]-thiazol-5-yloxy}-3-fluoro-benzoic acid methyl ester |
| A114 * | 632.8 | 4-{2-[2-(4-Cyclopropanesulfonyl-phenyl)-2-(2,4-difluorophenoxy)-acetylamino]-thiazol-5-yloxy}-2-fluoro-benzoic acid methyl ester |
| A115 * | 612.30 | 2-(4-Cyclopropanesulfonyl-phenyl)-2-(2,4-difluoro-phenoxy)-N-[5-(5-trifluoromethyl-pyridin-2-yloxy)-thiazol-2-yl]-acetamide |
| A116 * | 560.56 | 2-(4-Cyclopropanesulfonyl-phenyl)-2-(2,4-difluoro-phenoxy)-N-[5-(4-fluoro-phenoxy)-thiazol-2-yl]-acetamide |
| A117 | 543.2 | 2-(4-Cyclopropanesulfonyl-phenyl)-N-[5-(4-formyl-phenoxy)-thiazol-2-yl]-2-(tetrahydro-pyran-4-yloxy)-acetamide |
| A118 | 449.2 | 2-(4-Cyclopropanesulfonyl-phenyl)-2-(tetrahydro-pyran-4-yloxy)-N-(5-vinyl-thiazol-2-yl)-acetamide |
| A119 | 449.1 | 2-(4-Cyclopropanesulfonyl-phenyl)-2-(tetrahydro-pyran-4-yloxy)-N-(4-vinyl-thiazol-2-yl)-acetamide |
| A120 * | 494.1 | 2-[4-(Morpholine-4-sulfonyl)-phenyl]-2-(tetrahydro-pyran-4-yloxy)-N-(5-vinyl-thiazol-2-yl)-acetamide |

| | | |
|---|---|---|
| *Reference Example | | |

### Example (B1): 4-{2-[2-(4-Cyclopropanesulfonyl-phenyl)-2-(2,4-difluoro-phenoxy)-acetylamino]-thiazol-5-yloxy}-benzoic acid. (Reference):

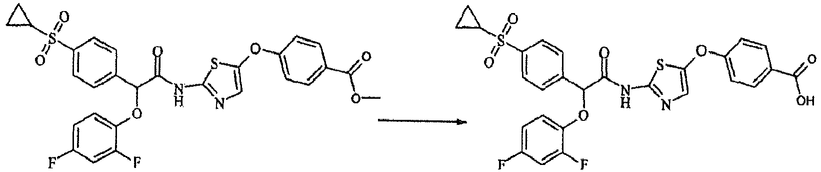

4-{2-[2-(4-Cyclopropanesulfonyl-phenyl)-2-(2,4-difluoro-phenoxy)-acetylamino]-thiazol-5-yloxy}-benzoic acid methyl ester (1.4 gm, 2.33 mmol, obtained in example A74) was taken in THF: MeOH (1:1, 20 mL), to it was added aqueous solution of LiOH (0.34 gm, 7.94 mmol in 10 mL water) and stirred for 1-4 h. After completion of the reaction, organic solvent was removed under reduced pressure. The aqueous layer was washed with diisopropyl ether then acidified with 1 N HCl to pH 2. The solid formed was filtered, washed with water, followed by washing with diisopropyl ether & dried under vacuum to get the title compound (1.17g, 86%).
1H NMR- (CDCl₃), δ 1.04-1.13 (m, 4H), 2.88 (m, 1H), 6.2 (s, 1H), 7.05-7.39 (m, 6H), 7.88-8.01 (m, 5H), 12.94 (bs, 1H). MS (EI) *m*/*z*: 587 (M+1)

### Example B2: 4-{2-[2-(4-Cyclopropanesulfonyl-phenyl)-2-(tetrahydro-pyran-4-yloxy)-acetylamino]-thiazol-5-yloxy}-benzoic acid

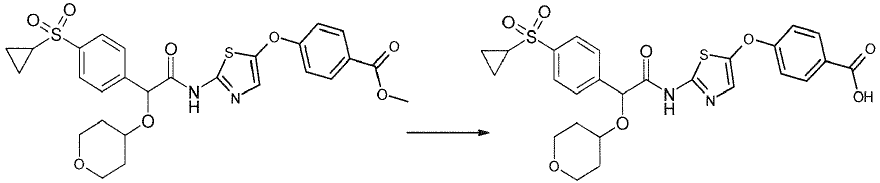

The compound of example B2 was obtained by similar method described in example B1 using 4-{2-[2-(4-Cyclopropanesulfonyl-phenyl)-2-(2,4-difluoro-phenoxy)-acetylamino]-thiazol-5-yloxy}-benzoic acid methyl ester (0.17 gm, 0.29mmol), LiOH.H₂O (0.062 gm, 1.48 mmol) in THF: MeOH: Water (1:1:1, 9 mL) to provide the title compound (0.117 gm, 72 %).
¹H NMR (DMSO-d₆, 400 MHz): δ - 1.03-1.06 (m, 2H), 1.10-1.34 (m, 2H), 1.46-1.60 (m, 2H), 1.85-1.94 (m, 2H), 2.83-2.89 (m, 1H), 3.29-3.39 (m, 2H), 3.63-3.68 (m, 1H), 3.77-3.85 (m, 2H), 5.48 (s, 1H), 7.18 (d, J = 9.2 Hz, 2H), 7.38 (s, 1H), 7.78 (d, J = 8.0 Hz, 2H), 7.93-7.95 (m, 4H). MS (EI) m/z: 558.8 (M + 1).

### Example B3: 4-(2-{2-(4-Cyclopropanesulfonyl-phenyl)-2-[(R)-(tetrahydro-furan-3-yl)oxy]-acetylamino}-thiazol-5-yloxy)-benzoic acid

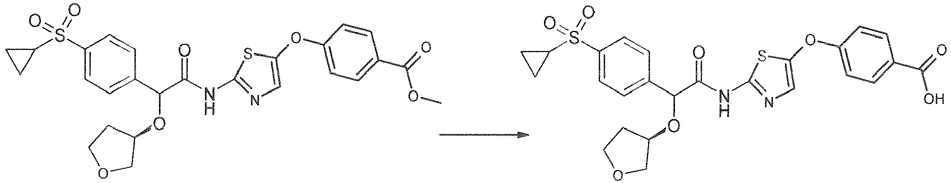

The compound of example B3 was obtained by similar method described in example B2 using 4-(2-{2-(4-Cyclopropanesulfonyl-phenyl)-2-[(R)-(tetrahydro-furan-3-yl)oxy]-acetylamino}-thiazol-5-yloxy)-benzoic acid methyl ester (0.180 gm, 0.32 mmol), LiOH.H₂O (0.067 gm, 1.61 mmol) in THF: MeOH: Water (1:1:1, 9 mL) to provide the title compound (0.160 gm, 92 %).
¹H NMR (DMSO-d₆, 400 MHz): δ- 1.01-1.05 (m, 2H), 1.09-1.31 (m, 2H), 1.94-1.99 (m, 2H), 2.80-2.68 (m, 1H), 3.60-3.85 (m, 4H), 4.20-4.30 (m, 1H), 5.36 (s,1H), 7.16 (d, J = 7.2 Hz , 2H), 7.38 (s, 1H), 7.73-7.76 (m, 2H), 7.92 (d, J = 8.4 Hz, 4H).
MS (EI) m/z: 544.9 (M + 1).

### Examples B4 to B64 were prepared in analogues manner of examples B1-B3 from the appropriate intermediate that are available commercially or synthesized as above.

| | | |
|---|---|---|
| B4 | 500.8 | {5-Chloro-2-[2-(4-cyclopropanesulfonyl-phenyl)-2-(tetrahydro-furan-3-yloxy)-acetylamino]-thiazol-4-yl}-acetic acid |
| B5 * | 584(M-1) | (5-Chloro-2-{2-(2,4-difluoro-phenoxy)-2-[4-(piperidine-1-sulfonyl)-phenyl]-acetylamino}-thiazol-4-yl)-acetic acid |
| B6 * | 632.3 | 4-(2-{2-(2,4-Difluoro-phenoxy)-2-[4 -(morpholine-4-sulfonyl)-phenyl]-acetylamino}-thiazol-5-yloxy)-benzoic acid |
| B7 * | 588.3 | (5-Chloro-2-{2-(2,4-difluoro-phenoxy)-2-[4-(morpholine-4-sulfonyl)-phenyl]-acetylamino}-thiazol-4-yl)-acetic acid |
| B8 * | 632.3 | 3-(2-{2-(2,4-Difluoro-phenoxy)-2-[4 -(morpholine-4-sulfonyl)-phenyl]-acetylamino}-thiazol-5-yloxy)-benzoic acid |
| B9 * | 607.4 | 1-(2-{2-(2,4-Difluoro-phenoxy)-2-[4-(pyrrolidine-1-sulfonyl)-phenyl]-acetylamino}-thiazol-5-yl)-piperidine-4-carboxylic acid |
| B10 * | 614.1(M-1) | 4-(2-{2-(2,4-Difluoro-phenoxy)-2-[4 -(pyrrolidine-1-sulfonyl)-phenyl]-acetylamino}-thiazol-5-yloxy)-benzoic acid |
| B11 * | 614.2(M-1) | 3-(2-{2-(2,4-Difluoro-phenoxy)-2-[4-(pyrrolidine-1-sulfonyl)-phenyl]-acetylamino}-thiazol-5-yloxy)-benzoic acid |
| B12 * | 572 | (5-Chloro-2-{2-(2,4-difluoro-phenoxy)-2-[3-(pyrrolidine-1-sulfonyl)-phenyl]-acetylamino}-thiazol-4-yl)-acetic acid |
| B13 * | 557.90 | {2-[2-[3-(Azetidine-1-sulfonyl)-phenyl]-2-(2,4-difluorophenoxy)-acetylamino]-5-chloro-thiazol-4-yl}-aceticacid |
| B14 * | 557.90 | {2-[2-[4-(Azetidine-1-sulfonyl)-phenyl]-2-(2,4-difluorophenoxy)-acetylamino]-5-chloro-thiazol-4-yl}-acetic acid |
| B15 * | 582.0 | (5-Chloro-2-{2-(4-chloro-phenoxy)-2-[4-(piperidine-1-sulfonyl)-phenyl]-acetylamino}-thiazol-4-yl)-acetic acid |
| B16 * | 604.1 | (5-Chloro-2-{2-(4-chloro-2-fluoro-phenoxy)-2-[4-(piperidine-1-sulfonyl )-phenyl]-acetylamino}-thiazol-4-yl)-acetic acid |
| B17 * | 585.9 | (5-Chloro-2-{2-(4-chloro-phenoxy)-2-[4-(morpholine-4-sulfonyl)-phenyl]-acetylamino}-thiazol-4-yl)-acetic acid |
| B18 * | 628.2 | 3-(2-{2-(4-Chloro-phenoxy)-2-[4-(piperidine-1-sulfonyl)-phenyl]-acetylamino}-thiazol-5-yloxy)-benzoic acid |
| B19 * | 628.0 | 4-(2-{2-(4-Chloro-phenoxy)-2-[4-(pi peridine-1-sulfonyl)-phenyl]-acetylamino}-thiazol-5-yloxy)-benzoic acid |
| B20 * | 621.4 | 1-(2-{2-(2,4-Difluoro-phenoxy)-2-[4-(piperidine-1-sulfonyl)-phenyl]-acetylamino}-thiazol-5-yl)-iperidine-4-carboxylic acid |
| B21 * | 603.5 | (5-Chloro-2-{2-(4-chloro-2-fluoro-phenoxy)-2-[4-(morpholine-4-sulfonyl )-phenyl]-acetylamino}-thiazol-4-yl)-acetic acid |
| B22 * | 630.2 | 4-(2-{2-(2,4-Difluoro-phenoxy)-2-[4-(piperidine-1-sulfonyl)-phenyl]-acetylamino}-thiazol-5-yloxy)-benzoic acid |
| B23 *. | 630.2 | 3-(2-{2-(2,4-Difluoro-phenoxy)-2-[4-(piperidine-1-sulfonyl)-phenyl]-acetylamino}-thiazol-5-yloxy)-benzoic acid |
| B24 * | 570.1 | (2-{2-(4-Chloro-2-fluoro-phenoxy)-2-[4-(morpholine-4-sulfonyl)-phenyl]-acetylamino}-thiazol-4-yl)-acetic acid |
| B25 * | 631.30 | 4-(2-{2-(2,4-Difluoro-phenoxy)-2-[4 -(tetrahydro-pyran-4-sulfonyl)-phenyl]-acetylamino}-thiazol-5-yloxy)-benzoic acid |
| B26 | 545.1 | 4-{2-[2-(4-Cyclopropanesulfonyl-phenyl)-2-(tetrahydro-furan-3-yloxy)-acetylamino]-thiazol-5-yloxy}-benzoic acid |
| B27 | 500.9 | (5-Chloro-2-{2-(4-cyclopropanesulfonyl-phenyl)-2-[(R)-(tetrahydro-furan-3-yl)oxy]-acetylamino}-thiazol-4-yl)-acetic acid |
| B28 | 544.9 | 4-(2-{2-(4-Cyclopropanesulfonyl-phenyl)-2-[(S)-(tetrahydrofuran-3-yl)oxy]-acetylamino}-thiazol-5-yloxy)-benzoic acid |
| B29 | 500.9 | (5-Chloro-2-{2-(4-cyclopropanesulfonyl-phenyl)-2-[(S)-(tetrahydro-furan-3-yl)oxy]-acetylamino}-thiazol-4-yl)-acelic acid |
| B30 | 540.3 | 4-{3-[2-(4-Cyclopropanesulfonyl-phenyl)-2-(tetrahydropyran-4-yloxy)-acetylamino]-pyrazol-1-ylmethyl}-benzoic acid |
| B31 | 514.90 | {5-Chloro-2-[2-(4-cyclopropanesulfonyl-phenyl)-2-(tetrahydro-pyran-4-yloxy)-acetylamino]-thiazol-4-yl}-acetic acid |
| B32 | 558.9 | 3-{2-[2-(4-Cyclopropanesulfonyl-phenyl)-2-(tetrahydropyran-4-yloxy)-acetylamino]-thiazol-5-yloxy}-benzoic acid |
| B33 | 576.9 | 4-{2-[2-(4-Cyclopropanesulfonyl-phenyl)-2-(tetrahydropyran-4-yloxy)-acetylamino]-thiazol-5-yloxy}-2-fluorobenzoic acid |
| B34 | 576.9 | 4-{2-[2-(4-Cyclopropanesulfonyl-phenyl)-2-(tetrahydropyran-4-yloxy)-acetylamino]-thiazol-5-yloxy}-3-fluorobenzoic acid |
| B35 | 548.0 | {2-[2-(4-Cyclopropanesulfonyl-phenyl)-2-(tetrahydro-pyran-4-yloxy)-acetylamino]-thiazolo[5,4-b]pyridin-5-yloxy}-acetic acid |
| B36 * | 638.00 | 4-{2-[2-(4-Cyclopropanesulfonyl-phenyl)-2-(2,4-difluorophenoxy)-acetylamino]-thiazolo[5,4-b]pyridin-5-yloxy}-benzoic acid |
| B37 | 610.20 | 4-{2-[2-(4-Cyclopropanesulfonyl-phenyl)-2-(tetrahydropyran-4-yloxy)-acetylamino]-thiaxolo[5,4-b]pyridin-5-yloxy}-benzoic acid |
| B38 | 615.9 | 4-[(4-Carboxymethyl-5-chloro-thiazol-2-ylcarbamoyl)-(4-cyclopropanesulfonyl-phenyl)-methoxy]-piperidine-1-carboxylic acid tert-butyl ester |
| B39 | 515 | {5-Chloro-2-[2-(4-cyclopropanesulfonyl-phenyl)-2-(piperidin-4-yloxy)-acetylamino]-thiazol-4-yl} -acetic acid |
| B40 | 573.2 | (4-{2-[2-(4-Cyclopropanesulfonyl-phenyl)-2-(tetrahydropyran-4-yloxy)-acetylamino]-thiazol-5-yloxy}-phenyl)-acetic acid |
| B41 | 517.2 | 2-[2-(4-Cyclopropanesulfonyl-phenyl)-2-(tetrahydro-pyran-4-yloxy)-acetylamino]-benzothiazole-6-carboxylic acid |
| B42 | 503.2 | 2-{2-(4-Cyclopropanesulfonyl-phenyl)-2-[(R)-(tetrahydrofuran-3-yl)oxy]-acetylamino}-benzothiazole-6-carboxylic acid |
| B43 * | 587.0 | (5-Chloro-2-{2-(2,4-difluoro-phenoxy)-2-[4-(tetrahydropyran-4-sulfonyl)-phenyl]-acetylamino}-thiazol-4-yl)-acetic acid |
| B44 | 573.2 | 4-{2-[2-(4-Cyclopropanesulfonyl-phenyl)-2-(tetrahydropyran-4-yloxy)-acetylamino]-thiazol-5-yloxy}-2-methylbenzoic acid |
| B45 | 481.1 | 2-[2-(4-Cyclopropanesulfonyl-phenyl)-2-(tetrahydro-pyran-4-yloxy)-acetylamino]-5-methyl-thiazole-4-carboxylic acid |
| B46 * | 587.3 | 3-{2-[2-(4-Cyclopropanesulfonyl-phenyl)-2-(2,4-difluorophenoxy)-acetylamino]-thiazol-5-yloxy}-benzoic acid |
| B47 * | 584.9 | 4-{2-[2-(4-Chloro-phenoxy)-2-(4-cyclopropanesulfonylphenyl)-acetylamino]-thiazol-5-yloxy}-benzoic acid |
| B48 * | 568.6 | 4-{2-[2-(4-Cyclopropanesulfonyl-phenyl)-2-(4-fluoro-phenoxy)-acetylamino]-thiazol-5-yloxy}-benzoic acid |
| B49 * | 619.1 | 4-{2-[2-(4-Cyclopropanesulfonyl-phenyl)-2-(3,4-dichlorophenoxy)-acetylamino]-thiazol-5-yloxy}-benzoic acid |
| B50 * | 587.2 | 4-{2-[2-(4-Cyclopropanesulfonyl-phenyl)-2-(3,4-difluorophenoxy)-acetylamino]-thiazol-5-yloxy}-benzoic acid |
| B51 * | 569.2 | 4-{2-[2-(4-Cyclopropanesulfonyl-phenyl)-2-(3-fluoro-phenoxy)-acetylamino]-thiazol-5-yloxy}-benzoic acid |
| B52 * | 617.2 | 4-{2-[2-(3-Chloro-4-fluoro-phenoxy)-2-(4-cyclopropanesulfonyl-phenyl)-acetylamino]-thiazol-5-yloxy}-benzoic acid |
| B53 * | 581.20 | 4-{2-[2-(4-Cyclopropanesulfonyl-phenyl)-2-(4-methoxyphenoxy)-acetylamino]-thiazol-5-yloxy}-benzoic acid |
| B54 * | 581.20 | 4-{2-[2-(4-Cyclopropanesulfonyl-phenyl)-2-(3-methoxyphenoxy)-acetylamino]-thiazol-5-yloxy}-benzoic acid |
| B55 * | 619.20 | 4-{2-[2-(4-Cyclopropanesulfonyl-phenyl)-2-(4-trifluoromethyl-phenoxy)-acetylamino]-thiazol-5-yloxy}-benzoic acid |
| B56 * | 619.20 | 4-{2-[2-(4-Cyclopropanesulfonyl-phenyl)-2-(3-trifluoromethyl-phenoxy)-acetylamino]-thiazol-5-yloxy}-benzoic acid |
| B57 * | 635.20 | 4-{2-[2-(4-Cyclopropanesulfonyl-phenyl)-2-(4-trifluoromethoxy-phenoxy)-acetylamino]-thiazol-5-yloxy}-benzoic acid |
| B58 * | 588.20 | 6-{2-[2-(4-Cyclopropanesulfonyl-phenyl)-2-(2,4-difluorophenoxy)-acetylamino]-thiazol-5-yloxy}-nicotinicacid |
| B59 * | 565.2 | 4-{2-[2-(4-Cyclopropanesulfonyl-phenyl)-2-(4-methylphenoxy)-acetylamino]-thiazol-5-yloxy} -benzoic acid |
| B60 * | 551.1 | 4-{2-[2-(4-Cyclopropanesulfonyl-phenyl)-2-phenoxyacetylamino]-thiazol-5-yloxy}-benzoic acid |
| B61 * | 601.1 | (4-{2-[2-(4-Cyclopropanesulfonyl-phenyl)-2-(2,4-difluorophenoxy)-acetylamino]-thiazol-5-yloxy}-phenyl)-acetic acid |
| B62 * | 600.8 | 4-{2-[2-(4-Cyclopropanesulfonyl-phenyl)-2-(2,4-difluorophenoxy)-acetylamino]-4-methyl-thiazol-5-yloxy}-benzoic acid |
| B63 * | 604.8 | 4-{2-[2-(4-Cyclopropanesulfonyl-phenyl)-2-(2,4-difluorophenoxy)-acetylamino]-thiazol-5-yloxy}-3-fluoro-benzoic acid |
| B64 * | 604.8 | 4-{2-[2-(4-Cyclopropanesulfonyl-phenyl)-2-(2,4-1-difluorophenoxy)-acetylamino]-thiazol-5-yloxy}-2-fluoro-benzoic acid |

| | | |
|---|---|---|
| *Reference Example | | |

### Example C1: N-{5-[4-(Azetidine-1-carbonyl)-phenoxy]-thiazol-2-yl}-2-(4-cyclopropanesulfonyl-phenyl)-2-(tetrahydro-pyran-4-yloxy)-acetamide:

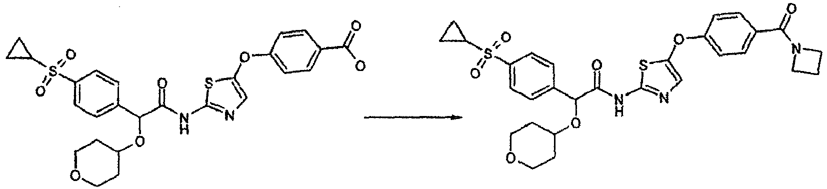

To a solution of 4-{2-[2-(4-Cyclopropanesulfonyl-phenyl)-2-(tetrahydro-pyran-4-yloxy)-acetylamino]-thiazol-5-yloxy}-benzoic acid (0.250 gm, 0.44 mmol) in DMF (7 ml) was added azetidine hydrochloride (0.052 gm, 0.56 mmol), HOAt (0.5M in DMF) (1.16 ml, 0.58 mmol), EDCI (0.106 gm, 0.56 mmol) and diisopropylethylamine (0.2 ml, 1.16 mmol) and mixture was stirred for 18 hours at 25°C under argon atmosphere. Reaction mixture was diluted with water (25 ml) and extracted with ethylacetate (3X30 ml), combined organic layer was washed with brine solution, dried over anhydrous sodium sulfate, filtered and concentrated under reduced pressure. The residue was triturated with diisopropylether to get the title compound (0.174 gm).
¹H NMR (400 MHz, DMSOd₆): δ 1.02-1.04 (m, 2H), 1.08-1.13 (m, 2H), 1.40-1.60 (m, 2H), 1.82-1.95 (m, 2H), 2.21-2.30 (m, 2H), 2.82-2.90 (m, 1H), 3.29-3.40 (m, 2H), 3.60-3.70 (m, 1H), 3.78-3.90 (m, 2H), 3.99-4.03 (m, 2H), 4.26-4.29 (m, 2H), 5.48 (s, 1H), 7.13 (d, J = 8.8 Hz, 2H), 7.37 (s, 1H), 7.63 (d, J = 8.8 Hz, 2H), 7.78 (d, J = 8.4 Hz, 2H), 7.93 (d, J = 8.4 Hz, 2H).
MS (EI) *m*/*z*: 598.2 (M+1).

### Examples C2 and C7 were prepared in analogues manner of example C1

| Example No. | Mass | IUPAC Name |
|---|---|---|
| C2 | 543.9 | 4-{2-[2-(4-Cyclopropanesulfonyl-phenyl)-2-(tetrahydro-furan-3-yloxy)-acetylamino]-thiazol-5-yloxy}-benzamide |
| C3 | 554.9 | N-[4-(2-Azetidin-1-yl-2-oxo-ethyl)-5-chloro-thiazol-2-yl]-2-(4-cyclopropanesulfonyl-phenyl)-2-(tetrahydro-pyran-4-yloxy)-acetamide |
| C4 * | 626.3 | N-{5-[4-(Azetidine-1-carbonyl)-phenoxy]-thiazol-2-yl}-2-(4-cyclopropanesulfonyl-phenyl)-2-(2,4-difluoro-phenoxy)-acetamide |
| C5 * | 626.3 | N-{5-[3-(Azetidine-1-carbonyl)-phenoxy]-thiazol-2-yl}-2-(4-cyclopropanesulfonyl-phenyl)-2-(2,4-difluoro-phenoxy)-acetamide |
| C6 * | 586.2 | 3-{2-[2-(4-Cyclopropanesulfonyl-phenyl)-2-(2,4-difluorophenoxy)-acetylamino]-thiazol-5-yloxy}-benzamide |
| C7 * | 586.2 | 4-{2-[2-(4-Cyclopropanesulfonyl-phenyl)-2-(2,4-difluorophenoxy)-acetylamino]-thiazol-5-yloxy}-benzamide |

| | | |
|---|---|---|
| *Reference Example | | |

### Example D1A/B-D12A/B Chirally Pure Compounds

### Analytical methods used for chiral separation:

**Method-1:**-Column-OJ-RH (150X4.6)mm, 5µm; Flow: 1.0 ml min-1; M.Phase:(98:02) Methanol : 0.05% Formic acid in water; Column Temp.: 40°C, Detection wavelength: 220 nm.
**Method-2:**-Column-OJ-RH (150X4.6)mm, 5µm; Flow:1.0 ml min-1; M.Phase: 100% Methanol, Column Temp.: ambient, Detection wavelength : 220 nm.
**Method-3:**-Column-OJ-RH (150X4.6)mm, 5µm; Flow:1.0 ml min-1; M.Phase:(95:5) Methanol : 0.05% Formic acid in water; Column Temp.: 40°C, Detection wavelength : 220/295 nm.
**Method-4:**-Column-OJ-RH (150X4.6)mm, 5µm; Flow:1.0 ml min-1; M.Phase:(98:2) Methanol (0.05% Formic acid) : 0.05% Formic acid in water; Column Temp.: 40°C, Detection wavelength : 220 nm.
**Method-5:**-Column-OJ-RH (150X4.6)mm, 5µm; Flow:0.7 ml min-1; M.Phase:(90:10) Methanol : 0.05% Formic acid in water; Column Temp.: 40°C, Detection wavelength : 220 nm.
**Method-6:**-Column-OJ-RH (150X4.6)mm, 5µm; Flow:1.4 ml min-1; M.Phase:(95:5) Methanol : 0.05% Formic acid in water; Column Temp.: 40°C, Detection wavelength : 274 nm.
**Method-7:**-Column-OJ-RH (150X4.6)mm, 5µm; Flow:1.0 ml min-1; M.Phase: 100% Methanol, Column Temp.: 40°C, Detection wavelength : 220 nm.
**Method-8:**-Column-OJ-RH (150X4.6)mm, 5µm; Flow:1.0 ml min-1; M.Phase:(90:10) Methanol (0.05% Formic acid) : 0.05% Formic acid in water; Column Temp.: 40°C, Detection wavelength: 220 nm.
**Method-9:**-Column-OJ-RH (150X4.6)mm, 5µm; Flow:1.5 ml min-1; M.Phase: Methanol, Column Temp.: 40°C, Detection wavelength: 220 nm.

| **Exam pie No.** | **Mass** | **IUPAC Name** | **Examples Used/ Method Used** |
|---|---|---|---|
| D1A * | 545.0 | (-)2-(2,4-Difluoro-phenoxy)-2-[4-(piperidine-1-sulfonyl)-phenyl]-N-thiazo lo[5,4-b]pyridin-2-yl-acetamide | A-3 Method-3 |
| D1B * | 545.0 | (+)2-(2,4-Difluoro-phenoxy)-2-[4-(piperidine-1-sulfonyl)-phenyl]-N-thiazo lo[5,4-b]pyridin-2-yl-acetamide | |
| D2A * | 587.8 | (-) (5-Chloro-2-{2-(2,4-difluoro-phenoxy)-2-[4-(morpholine-4-sulfonyl)-phenyl]-acetylamino}-thiazol-4-yl)-acetic acid | B-7 Method-4 |
| D2B * | 587.8 | (+) (5-Chloro-2-{2-(2,4-difluorophenoxy)-2-[4-(morpholine-4-sulfonyl)-phenyl]-acetylamino}-thiazol-4-yl)-acetic acid | |
| D3A * | 585.8 | (-) (5-Chloro-2-{2-(2,4-difluorophenoxy)-2-[4-(piperidine-1-sulfonyl)-phenyl]-acetylamino}-thiazol-4-yl)-acetic acid | B-5 Method-5 |
| D3B * | 585.8 | (+) (5-Chloro-2-{2-(2,4-difluorophenoxy)-2-[4-(piperidine-1-sulfonyl)-phenyl]-acetylamino}-thiazol-4-yl)-acetic acid | |
| D4A | 559.3 | (+) 4-{2-[2-(4-Cyclopropanesulfonylphenyl)-2-(tetrahydro-pyran-4-yloxy)-acetylamino]-thiazol-5-yloxy}-benzoic acid | B-2 Method-7 |
| D4B | 559.3 | (-) 4-{2-[2-(4-Cyclopropanesulfonylphenyl)-2-(tetrahydro-pyran-4-yloxy)-acetylamino]-thiazol-5-yloxy}-benzoic acid | |
| D5A | 544.9 | 4-(2-{2-(4-Cyclopropanesulfonyl-phenyl)-2-[(S)-(tetrahydro-furan-3-yl)oxy]-acetylamino}-thiazol-5-yloxy)-benzoic acid | B-28 Method-1 |
| D5B | 544.9 | 4-(2-{2-(4-Cyclopropanesulfonyl-phenyl)-2-[(S)-(tetrahydro-furan-3-yl)oxy]-acetylamino}-thiazol-5-yloxy)-benzoic acid | |
| D6A | 545.0 | 4-(2-{2-(4-Cyclopropanesulfonyl-phenyl)-2-[(R)-(tetrahydro-furan-3-yl)oxy]-acetylamino}-thiazol-5-yloxy)-benzoic acid | B-2 Method-1 |
| D6B | 545.0 | 4-(2-{2-(4-Cyclopropanesulfonyl-phenyl)-2-[(R)-(tetrahydro-furan-3-yl)oxy]-acetylamino}-thiazol-5-yloxy)-benzoic acid | |
| D7A | 474.00 | (-)2-(4-Cyclopropanesulfonyl-phenyl)-2-(tetrahydro-pyran-4-yloxy)-N-thiazolo[5,4-b]pyridin-2-yl-acetamide | A-79 Method-2 |
| D7B | 474.00 | (+)2-(4-Cyclopropanesulfonyl-phenyl)-2-(tetrahydro-pyran-4-yloxy)-N-thiazolo[5,4-b]pyridin-2-yl-acetamide | |
| D8A * | 603.5 | (-)(5-Chloro-2-{2-(4-chloro-2-fluoro-phenoxy)-2-[4-(morpholine-4-sulfonyl )-phenyl]-acetylamino}-thiazol-4-yl)-acetic acid | B-21 Method-8 |
| D8B * | 603.5 | (+)(5-Chloro-2-{2-(4-chloro-2-fluoro-phenoxy)-2-[4-(morpholine-4-sulfonyl )-phenyl]-acetylamino}-thiazol-4-yl)-acetic acid | |
| D9A | 504.20 | (+)2-(4-Cyclopropanesulfonyl-phenyl)-N -(5-methoxy-thiazolo[5,4-b]pyridin-2-yl)-2-(tetrahydro-pyran-4-yloxy)-Acetamide | A-6 Method-9 |
| D9B | 504.20 | (-)2-(4-Cyclopropanesulfonyl-phenyl)-N -(5-methoxy-thiazolo[5,4-b]pyridin-2-yl)-2-(tetrahydro-pyran-4-yloxy)-Acetamide | |
| D10A | 518.20 | (+)2-(4-Cyclopropanesulfonyl-phenyl)-N -(5-ethoxy-thiazolo[5,4-b]pyridin-2 -yl)-2-(tetrahydro-pyran-4-yloxy)-a Cetamide | A-7 Method-2 |
| D10B | 518.20 | (-)2-(4-Cyclopropanesulfonyl-phenyl)-N -(5-ethoxy-thiazolo[5,4-b]pyridin-2 -yl)-2-(tetrahydro-pyran-4-yloxy)-a Cetamide | |
| D11A * | 587 | (+)4-{2-[2-(4-Cyclopropanesulfonylphenyl)-2-(2,4-difluoro-phenoxy)-acetylamino]-thiazol-5-yloxy}-benzoic acid | B-1 Method-6 |
| D11B * | 587 | (-)4-{2-[2-(4-Cyclopropanesulfonylphenyl)-2-(2,4-difluoro-phenoxy)-acetylamino]-thiazol-5-yloxy}-benzoic acid | |
| D12A * | 626.0 | (+)N-{5-[4-(Azetidine-1-carbonyl)-phenoxy]-thiazol-2-yl}-2-(4-cyclopropanesulfonyl-phenyl)-2-(2,4-difluoro-phenoxy)-acetamide | |
| D12B * | 626.0 | (-)N-{5-[4-(Azetidine-1-carbonyl)-phenoxy]-thiazol-2-yl}-2-(4-cyclopropanesulfonyl-phenyl)-2-(2,4-difluoro-phenoxy)-acetamide | C-4 Method-3 |

| | | | |
|---|---|---|---|
| *Reference Example | | | |

### Example E1: 2-(4-Cyclopropanesulfonyl-phenyl)-2-(tetrahydro-pyran-4-yloxy)-N-{5-[4-(2H-tetrazol-5-yl)-phenoxy]-thiazol-2-yl}-acetamide.

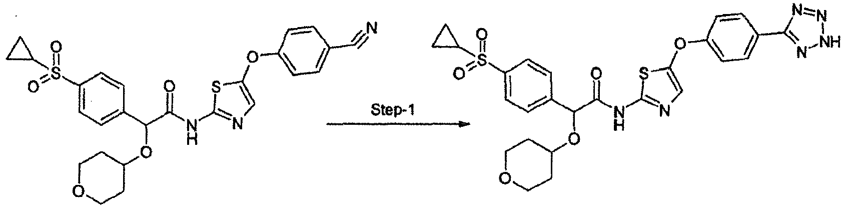

The mixture of N-[5-(4-Cyano-phenoxy)-thiazol-2-yl]-2-(4-cyclopropanesulfonylhenyl)-2-(tetrahydro-pyran-4-yloxy)-acetamide (0.25 gm, 0.46 mmol), sodium azide (0.24 gm, 3.71 mmol) and ammonium chloride (0198 gm, 3.71 mmol) in DMF (10 ml) was refluxed for 4-5 hr. After completion, reaction mixture was diluted with water and extracted with ethyl acetate, washed with water and brine, dried (Na₂SO₄) and concentrated in vacuo. Purified by PREP TLC to afford title compound (0.120 g, 44 %).
¹H NMR (400 MHz, ,CDCl₃): δ 1.03-1.08 (m, 2H), 1.34-1.49 (m, 2H), 1.73-1.81 (m, 2H), 1.87-2.05 (m, 2H), 2.43-2.48 (m ,1H), 3.37-3.44 (m, 2H), 3.67-3.74 (m, 1H), 3.93-4.03 (m, 2H), 5.24 (s, 1H), 7.16 (d, J = 8.8 Hz, 2H), 7.18 (s, 1H), 7.69 (d, J = 8.4 Hz, 2H), 7.91 (d, J = 8.8 Hz, 2H), 8.02 (d, J = 8.8 Hz, 2H).
MS (EI) *m*/*z*: 583.30 (M + 1).

### Example F1: 2-(4-Cyclopropanesulfonyl-phenyl)-N-[5-(4-hydroxymethyl-phenoxy)-thiazol-2-yl]-2-(tetrahydro-pyran-4-yloxy)-acetamide

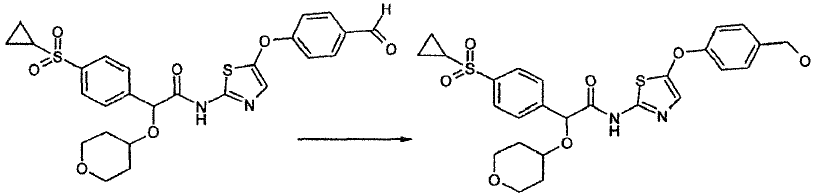

To a stirred solution of 2-(4-Cyclopropanesulfonyl-phenyl)-N-[5-(4-formyl-phenoxy)-thiazol-2-yl]-2-(tetrahydro-pyran-4-yloxy)-acetamide (Example A117) (0.2g, 0.36 mmol) in 10 mL methanol, sodium borohydride (0.027g, 0.73 mmol) was added in portions at 0°C After complete addition reaction mixture was stirred for 1hr at 0°C to 10 °C. Completion of the reaction was confirmed by TLC, then methanol was evaporated on rotavapour. Reaction mixture was diluted with ethyl acetate (20mL X 3), organic layer was washed with water and brine, dried (Na₂SO₄) and concentrated in vacuo. Purified by PREP TLC to afford title compound (0.110 gm).
¹H NMR (400 MHz, CDCl₃): δ 1.04-1.06 (m, 2H), 1.35-1.37 (m, 2H), 1.69-1.76 (m, 2H), 1.84-1.88 (m, 1H), 2.01-2.04 (m, 1H), 2.43-2.46 (m, 1H), 3.35-3.42 (m, 2H), 3.65-3.69 (m, 1H), 3.94-4.02 (m, 2H), 4.65 (bs, 2H), 5.20 (s, 1H), 7.06 (d, J = 8.8 Hz, 2H), 7.09 (s, 1H), 7.32 (d, J = 8.8 Hz, 2H), 7.68 (d, J = 7.6 Hz, 2H), 7.92 (d, J = 8.4 Hz, 2H).
MS (EI) *m*/*z*: 545.2 (M + 1).

### Example F2 : 2-(4-Cyclopropanesulfonyl-phenyl)-N-(4-hydroxymethyl-5-methylthiazol-2-yl)-2-(tetrahydro-pyran-4-yloxy)-acetamide

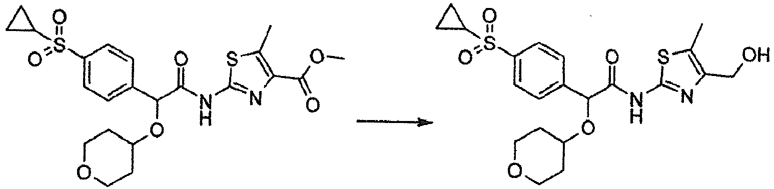

To a solution of 2-[2-(4-Cyclopropanesulfonyl-phenyl)-2-(tetrahydro-pyran-4-yloxy)-acetylamino]-5-methyl-thiazole-4-carboxylic acid methyl ester(Example A94) (0.23 gm, 0.46 mmol) in toluene (5 ml) was added DIBALH (1M in toluene,0.13 g, 0.93 mmol) at 0°C and reaction mixture was stirred overnight at room temperature. Solvent was removed in vacuo and taken into water, extracted with ethyl acetate (10 ml x 3), combined organic layers were washed with 1 N NaOH solution, water and brine, dried (Na₂SO₄) and concentrated in vacuo. Purified by preparative HPLC to afford title compound (0.012 gm)
¹HNMR (CDCl₃, 400 MHz):- δ 1.02-1.05 (m, 2H), 1.33-1.36 (m, 2H), 1.67-1.79 (m, 2H), 1.81-1.85 (m,1H), 1.89-2.30 (m,1H), 2.35 (s, 3H), 2.40-2.47(m, 1H), 3.34-3.42 (m, 2H), 3.61-3.65(m,1H), 3.93-4.02 (m, 2H), 4.60 (s, 2H), 5.20 (s, 1H), 7.67 (d, J = 8.0 Hz, 2H),7.91 (d, J = 8.4 Hz, 2H).
MS (EI) *m*/*z*: 467.20 (M+1).

### Example G1 : 2-(4-Cyclopropanesulfonyl-phenyl)-N-[5-(1,2-dihydroxy-ethyl)-thiazol-2-yl]-2-(tetrahydro-pyran-4-yloxy)-acetamide

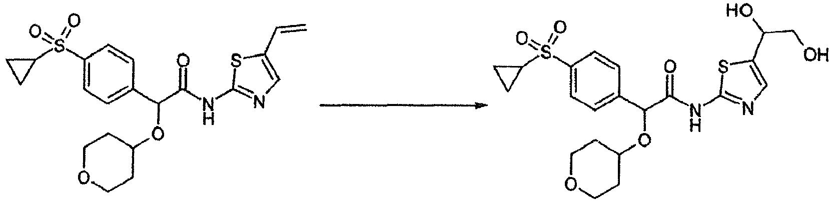

2-(4-Cyclopropanesulfonyl-phenyl)-2-(tetrahydro-pyran-4-yloxy)-N-(5-vinyl-thiazol-2-yl)-acetamide (Example A118) (0.107 gm, 0.238 mmol), osmium tetroxide (0.0061 gm , 0.0238 mmol) and N-methylmorpholine N-oxide (0.041 gm, 0.353 mmol) were taken in 4 ml of THF and 1 ml of water and stirred at room temperature for 3 hrs. The reaction mixture was partitioned between 10% sodium thiosulphate (10 ml) and ethyl acetate (10 ml). The layers were separated. Aq. layer was extracted with ethyl acetate (2x10 ml). The combined organic layer was washed with brine and dried over anhydrous sodium sulfate filtered and concentrated under reduced pressure to get a residue which was purified by preparative TLC to get the required product (0.070 gm).
¹H NMR (400 MHz, DMSOd₆): δ 1.00-1.05 (m,2H), 1.09-1.13 (m, 2H), 1.48-1.58 (m, 2H), 1.85-1.93 (m, 2H), 2.82-2.86 (m, 1H), 3.29-3.36 (m, 2H), 3.40-3.59 (m, 2H), 3.62-3.66 (m, 1H), 3.77-3.85 (m, 2H), 4.69-4.73 (q, J = 5.2 Hz, 1H), 4.89-4.93 (q, J = 5.6 Hz,1H), 5.47 (s, 1H), 5.59-5.61(t, J = 4.4 Hz, 1H), 7.31 (s, 1H), 7.77(d, J = 8.4 Hz, 2H), 7.92(d, J = 8.4 Hz, 2H), 12.25 (s, 1H).
MS (EI) *m*/*z*: 483.1 (M + 1).

### Example G2: 2-(4-Cyclopropanesulfonyl-phenyl)-N-[5-(1,2-dihydroxy-ethyl)-thiazolo[5,4-b]pyridin-2-yl]-2-(tetrahydro-pyran-4-yloxy)-acetamide

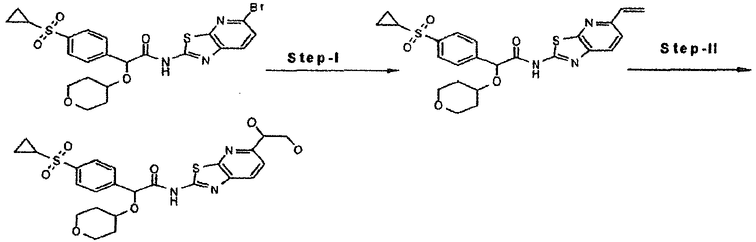

### Step-I: Synthesis of 2-(4-Cyclopropanesulfonyl-phenyl)-2-(tetrahydro-pyran-4-yloxy)-N-(5-vinyl-thiazolo[5,4-b]pyridin-2-yl)-acetamide.

To mixture of N-(5-Bromo-thiazolo[5,4-b]pyridin-2-yl)-2-(4-cyclopropanesulfonylphenyl)-2-(tetrahydro-pyran-4-yloxy)-acetamide (Example-A95) (0.135 gm, 0.24 mmol), Pd (PPh₃)₄ (0.014 gm, 0.012 mmol) and LiCl (0.031 gm, 0.73 mmol) in THF:DMF (1:1, 5 ml) under inert atmosphere was added tributyl(vinyl)stannane (0.23 gm, 0.73 mmol) and heated under reflux for 8 hrs.Solvent was removed in vacuo and taken into brine and extracted with ethyl aceatate (3X5 ml),washed with water and brine, dried (Na₂SO₄) and concentrated in vacuo to afford titled compound (0.080 gm)
MS (EI) *m*/*z*: 500.20 (M+1).

### Step-II: 2-(4-Cyclopropanesulfonyl-phenyl)-N-[5-(1,2-dihydroxy-ethyl)-thiazolo[5,4-b]pyridin-2-yl]-2-(tetrahydro-pyran-4-yloxy)-acetamide.

### Prepared in analogues manner of Example G1

¹HNMR (DMSO-d₆, 400 MHz):- δ 1.00-1.06 (m, 2H), 1.10-1.1.14 (m, 2H), 1.47-1.64 (m, 2H), 1.84-2.02 (m,2H), 2.83-2.89 (m,1H), 3.25-3.35 (m, 2H), 3.51-3.57 (m, 1H), 3.65-3.72 (m, 2H), 3.78-3.87 (m, 2H), 4.66-4.74 (m,2H), 5.54 (d, J = 4.8 Hz, 1H), 5. 56 (s, 1H), 7.59 (d, J = 8.4 Hz, 1H), 7.81 (d, J = 8.0 Hz, 2H), 7.95 (d, J = 8.4 Hz, 2H), 8.11 (d, J = 8.4 Hz, 1H), 12.78 (bs, 1H).
MS (EI) m/z: 534.20 (M+1).

### Examples G3 and G4 were prepared in analogues manner of example G1

| Example No. | Mass | IUPAC Name |
|---|---|---|
| G3 | 483.1 | 2-(4-Cyclopropanesulfonyl-phenyl)-N-[4-(1,2-dihydroxy-ethyl)-thiazol-2-yl]-2-(tetrahydro-pyran-4-yloxy)-acetamide |
| G4* | 528.2 | N-[5-(1,2-Dihydroxy-ethyl)-thiazol-2-yl]-2-[4-(morpholine-4-sulfonyl)-phenyl]-2-(tetrahydro-pyran-4-yloxy)-acetamide |

| | | |
|---|---|---|
| *Reference Example | | |

The list of examples below, but not to be limited to these, can also be synthesized following the general synthesis described above:
2-(4-Cyclopropanesulfonyl-phenyl)-N-[3-(1,2-dihydroxy-ethyl)-[1,2,4]thiadiazol-5-yl]-2-(tetrahydro-pyran-4-yloxy)-acetamide and its (+) and (-) enantiomers;
{5-Chloro-2-[2-(4-cyclopropanesulfonyl-phenyl)-2-(2-methyl-tetrahydro-pyran-3-yloxy)-acetylamino]-thiazol-4-yl}-acetic acid and its (+) and (-) enantiomers;
2-[2-[4-(3-Oxo-cyclopentanesulfonyl)-phenyl]-2-(tetrahydro-furan-3-yloxy)-acetylamino]-thiazole-4-carboxylic acid and its (+) and (-) enantiomers;
N-[4-(1-Acetyl-piperidin-4-yl)-thiazol-2-yl]-2-(4-cyclopropanesulfonyl-phenyl)-2-(tetrahydro-pyran-4-yloxy)-acetamide and its (+) and (-) enantiomers;
2-(4-Cyclopropanesulfonyl-phenyl)-N-{5-[4-(1,2-dihydroxy-ethyl)-phenoxy]-thiazol-2-yl}-2-(tetrahydro-pyran-4-yloxy)-acetamide and its (+) and (-) enantiomers;
2-(4-Cyclopropanesulfonyl-phenyl)-N-(4,5,6,7-tetrahydro-benzothiazol-2-yl)-2-(tetrahydro-pyran-4-yloxy)-acetamide and its (+) and (-) enantiomers;
2-(4-Cyclopropanesulfonyl-phenyl)-2-(tetrahydro-pyran-4-yloxy)-N-thiazolo[5,4-d]pyrimidin-2-yl-acetamide and its (+) and (-) enantiomers;
2-(4-Cyclopropanesulfonyl-phenyl)-N-[5-(4-hydroxymethyl-phenoxy)-thiazol-2-yl]-2-(tetrahydro-pyran-4-yloxy)-acetamide and its (+) and (-) enantiomers;
2-(2,4-Difluoro-phenoxy)-N-(5-methyl-4,5,6,7-tetrahydro-thiazolo[4,5-c]pyridin-2-yl)-2-[4-(piperidine-1-sulfonyl)-phenyl]-acetamide and its (+) and (-) enantiomers (Reference Example);
2-(2,4-Difluoro-phenoxy)-N-[3-(1,2-dihydroxy-ethyl)-[1,2,4]thiadiazol-5-yl]-2-[4-(piperidine-1-sulfonyl)-phenyl]-acetamide and its (+) and (-) enantiomers (Reference Example);
N-[3-(1,2-Dihydroxy-ethyl)-[1,2,4]thiadiazol-5-yl]-2-[4-(piperidine-1-sulfonyl)-phenyl]-2-(tetrahydro-pyran-4-yloxy)-acetamide and its (+) and (-) enantiomers (Reference Example);
2-(4-Cyclopropanesulfonyl-phenyl)-N-[4-(1,2-dihydroxy-ethyl)-thiazol-2-yl]-2-(tetrahydro-pyran-4-yloxy)-acetamide and its (+) and (-) enantiomers;
2-(4-Cyclopropanesulfonyl-phenyl)-N-[4-(1,2-dihydroxy-ethyl)-5-fluoro-thiazol-2-yl]-2-(tetrahydro-pyran-4-yloxy)-acetamide and its (+) and (-) enantiomers;
2-(4-Cyclopropanesulfonyl-phenyl)-N-[5-(1,2-dihydroxy-ethyl)-thiazol-2-yl]-2-(tetrahydro-pyran-4-yloxy)-acetamide and its (+) and (-) enantiomers;
2-(2,4-Difluoro-phenoxy)-N-[4-(1,2-dihydroxy-ethyl)-thiazol-2-yl]-2-[4-(piperidine-1-sulfonyl)-phenyl]-acetamide and its (+) and (-) enantiomers (Reference Example);
2-(4-Cyclopropanesulfonyl-phenyl)-N-[5-(1,2-dihydroxy-ethyl)-thiazolo[5,4-b]pyridin-2-yl]-2-(tetrahydro-pyran-4-yloxy)-acetamide and its (+) and (-) enantiomers;
N-[5-(1,2-Dihydroxy-ethyl)-thiazolo[5,4-b]pyridin-2-yl]-2-[4-(morpholine-4-sulfonyl)-phenyl]-2-(tetrahydro-pyran-4-yloxy)-acetamide and its (+) and (-) enantiomers (Reference Example);
2-(4-Chloro-2-fluoro-phenoxy)-N-[5-(1,2-dihydroxy-ethyl)-thiazolo[5,4-b]pyridin-2-yl]-2-[4-(pyrrolidine-1-sulfonyl)-phenyl]-acetamide and its (+) and (-) enantiomers (Reference Example);
{2-[2-(4-Cyclopropanesulfonyl-phenyl)-2-(4,6-difluoro-indan-2-yloxy)-acetylamino]-thiazol-4-yl}-acetic acid and its (+) and (-) enantiomers (Reference Example);
2-(3-{2-(4-Cyclopropanesulfonyl-phenyl)-2-[(R)-(tetrahydro-furan-3-yl)oxy]-acetylamino}-pyrazol-1-yl)-2-methyl-propionic acid and its (+) and (-) enantiomers;
2-(4-Cyclopentanesulfonyl-phenyl)-2-(2,4-difluoro-phenoxy)-N-(4-hydroxymethyl-5-methyl-thiazol-2-yl)-acetamide and its (+) and (-) enantiomers (Reference Example);
N-[5-Chloro-4-(2-hydroxy-ethyl)-thiazol-2-yl]-2-[4-(tetrahydro-pyran-4-sulfonyl)-phenyl]-2-(tetrahydro-pyran-4-yloxy)-acetamide and its (+) and enantiomers (Reference Example);
N-(5-Hydroxymethyl-pyridin-2-yl)-2-[4-(morpholine-4-sulfonyl)-phenyl]-2-(tetrahydropyran-4-yloxy)-acetamide and its (+) and (-) enantiomers (Reference Example);
6-{2-(Tetrahydro-furan-3-yloxy)-2-[4-(tetrahydro-pyran-4-sulfonyl)-phenyl]-acetylamino}-nicotinic acid and its (+) and (-) enantiomers (Reference Example).

### Glucokinase Activity Assay:

The glucokinase (GK) assay is a coupled enzymatic assay. GK catalyzes the first step of glycolysis, the conversion of glucose to glucose-6-phosphate (G6P) in the presence of ATP. G6P in turn is converted by glucose-6-phosphate dehydrogenase (G6PD) to 6-phosphogluconate, a process that requires NAD, resulting in NADH formation. Since the GK-catalyzed step is the rate-limiting step of this coupled enzymatic process, the rate of accumulation of 6-phosphogluconate and NADH is directly proportional to the rate of glucose phosphorylation by GK. The rate of the GK-catalyzed reaction can therefore be measured by monitoring the increase in NADH absorbance at 340 nm.

The assay is carried out according to the protocol outlined in Hariharan et al (1997), Diabetes 46: 11-16. Briefly, the test compounds are incubated in a reaction mix containing 25 mM HEPES (pH 7.2), 10 mM MgCl₂, 100 mM KCl, 5 mM ATP, 2 mM DTT, 0.5 mM NAD, 1 U/ml *Leuconostoc mesenteroides* G6PD, 0.3 U/ml of purified human recombinant GK, and different concentrations of glucose. Enzymatic activity is calculated from the initial reaction velocity, measured from the change in NADH absorbance as a function of time.

Compounds described in formula (I), in concentration ranges from 1.0 nM to 500 µM, are tested in the purified human recombinant glucokinase assay described above.

A compound is considered to be a glucokinase activator if it, in its testable range of concentrations, yields a higher rate of glucose phosphorylation than in its absence at a particular glucose concentration, for example at 5 mM glucose.

### Characterization of glucokinase activators from the in vitro glucokinase assay:

Compounds of general formula (I) are tested in the *in vitro* GK enzymatic assay to monitor dose-dependent effect on glucokinase activation (in kinetic mode), as described above, at various glucose concentrations. The S_{0.5} of glucokinase for glucose at different concentration of each compound of interest is calculated from the following modified version of the Michaelis-Menten equation, V = Vₘₐₓ [S]*ⁿ*/(S_{0.5}*ⁿ* + [S]*ⁿ*), where [S] is the glucose concentration and *n* is the Hill coefficient (taken as 1.7 to account for the sigmoidal kinetics of glucokinase with respect to glucose). The S_{0.5} is plotted against the log of the compound concentration. The change in the S_{0.5} of glucokinase (ΔS_{0.5}) for glucose is calculated by subtracting the S_{0.5} at each concentration of the compound from the S_{0.5} in the vehicle control. The ΔS_{0.5} is then normalized to a percent scale, where the S_{0.5} in the vehicle control is set to 0% and 0 mM glucose is set to 100%. The % ΔS_{0.5} is then plotted against the log of the compound concentration. The EC₅₀ and Eₘₐₓ of % change in S_{0.5} is obtained from the sigmoidal fit of the data. Detailed protocol has been described in our copending application no. 409/CHE/2007 which is incorporated herein by reference. Characterization data of some representative glucokinase activators of the present disclosure, which are illustrative but not limiting, are given in table I below.

The glucokinase activation data of some representative compounds of the present disclosure, which are illustrative but not to be construed as limiting the scope or spirit of the disclosure, are given in the table I below.

**Table I: EC₅₀ and Eₘₐₓ (with respect to % ΔS_{0.5}) of GK activators**

| **Example No.** | **EC₅₀ (µM)** | **% Eₘₐₓ** |
|---|---|---|
| F1 | 0.1 | 91 |
| D4A | 0.069 | 92 |
| D6A | 0.022 | 88 |
| B34 | 0.045 | 91 |
| E1 | 0.099 | 88 |
| D9A | 0.068 | 95 |
| D10A | 0.054 | 92 |
| B41 | 0.1 | 75 |
| D1A | 0.084 | 85 |
| D11A | 0.034 | 95 |

### Measurement of glycogen synthesis in primary rat hepatocytes:

Primary hepatocytes are collected from male Wistar rats, and tested for viability by trypan blue exclusion. Primary hepatocytes cultures with viability greater than 95% are selected for the glycogen synthesis assay. The cells are seeded in a 48-well plate at a density of 200,000 cells/well in 250 µl Minimal Essential Medium (MEM) containing 10% foetal calf serum (FCS) and 1.7 µM insulin, and incubated for 4 hours at 37°C to allow attachment. The medium is replaced with fresh MEM containing 10% FCS, 1.7 µM insulin and 10 nM dexamethasone, and the cells are incubated for 16 hours at 37°C. The medium is then replaced with fresh MEM (serum-free) containing 2 µCi/ml of D-[U¹⁴C]-Glucose along with 10 µM of the compound in a final DMSO concentration of 0.1%. The final glucose concentration is brought to 10 mM by the addition of D-Glucose (MEM already contains 5 mM glucose). The cells are incubated for 3 hours at 37°C. The cells are washed twice with 150 mM NaCl, lysed with 0.1 N NaOH, and the lysate precipitated with 8% w/v trichloroacetic acid (TCA) and 1 mg/well unlabeled glycogen as carrier. Cell debris is pelleted by centrifugation, the supernatant is removed, and the glycogen is precipitated with 63% ethanol. After another round of centrifugation, the supernatant is removed, and the pellet containing the precipitated glycogen is dried overnight. *De novo* synthesized glycogen is estimated in a scintillation counter (MicroBeta Trilux, Perkin Elmer), and expressed as fold increase over DMSO control.

The protocol for the glycogen assay is based on the method described in "Biochem J. 1990 Feb 15; 266(1): 91-102" with a few minor modifications. The protocol for isolation of primary rat hepatocytes is based on the method described in "Methods in Enzymology, Vol. III. pp 34-50. Ed. by S.P. Colowick and N.O. Kaplan. New York, Academic Press, 1957" with a few minor modifications.

Compounds described in formula (I), in concentration ranges from 1.0 nM to 500 µM, are tested in glycogen synthesis assay described above.

A compound is considered to be a glucokinase activator in a cellular environment if it demonstrates significant increase of glycogen synthesis over DMSO control as described in the above mentioned glycogen synthesis assay.

The glycogen synthesis data of some representative compounds of the present invention, which are illustrative but not limiting, is given in the table 2 below.

**Table 2: Glycogen synthesis data**

| **Examples** | **Fold increase in glycogen synthesis at 10 µM** |
|---|---|
| D6A | 11 |
| D11A | 15.5 |
| C6 | 13.5 |

## Claims

1. A compound of formula (I) 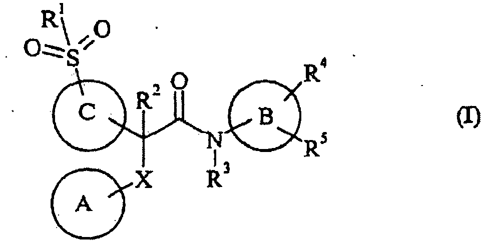 or its stereoisomers, tautomers, pharmaceutically acceptable salts, polymorphs, solvates and formulations wherein,
R¹ is a cycloalkyl; R² and R³ are hydrogen;
R⁴ and R⁵ are independently selected from a group consisting of hydrogen, halogen, alkyl, cycloalkyl, heterocyclyl, aryl, heteroaryl, mono, di or tri substituted haloalkyl, nitrile, -NR⁶C(O)OR⁷, -(CR⁸R⁹)ₙC(O)OR⁶, -(CR⁸R⁹)ₙC(O)NR⁶R⁷, - CR⁸R⁹)ₙS(O)ₚNR⁶R⁷, -(CR⁸R⁹)ₙN(R⁶)C(O)R⁷; -(CR⁸R⁹)ₙOR⁶, -C(R⁸R⁹)ₙNR⁶R⁷, - C(R⁸R⁹)ₙCO(R⁶) and -s(O)ₚC(R⁸R⁹)ₙC(O)OR⁶; wherein each of R⁴ and R⁵ is optionally substituted with one or more substituents selected from halo, alkyl, alkenyl, alkynyl, cycloalkyl, aryl, heteroaryl, heterocyclyl, alkylsulfonyl, oxo, nitro, cyano, -(CR⁸R⁹)ₙR⁶, -(CR⁸R⁹)ₙCOOR⁶, -(CR⁸R⁹)ₙC(O)NR⁶R⁷, - CR⁸R⁹)ₙOR⁶, -SR⁶ or -NR⁶R⁷; wherein n = 0-4;
X represents O;
Ring A represents a monocyclic heterocyclyl;
Ring B is heteroaryl and Ring C is phenyl;
Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, cycloalkyl, cycloalkylalkyl heterocyclyl, heterocycloalkyl, aryl, arylalkyl, heteroaryl, heteroarylalkyl, cycloalkenyl, aryloxy, heteroaryloxy groups;
p = 0-2; n= 0-4;
R⁶ and R⁷ are independently selected from a group consisting of hydrogen, alkyl, alkenyl, alkynyl, aryl, arylalkyl, heteroaryl, heteroarylalkyl, cycloalkyl, cycloalkylalkyl heterocyclyl and heterocyclylalkyl wherein each of R⁶ and R⁷ is optionally substituted with one or more substituents selected from halo, alkyl, cycloalkyl, aryl, heteroaryl, heterocyclyl, alkylsulfonyl, cyano, (CR⁸R⁹)ₙOR⁶, - (CR⁸R⁹)ₙCOOR⁶ or -(CR⁸R⁹)ₙC(O)NR⁶R⁷; wherein n = 0-4;
or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nitro, cyano, -OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl;
wherein R⁶ and R⁷ are as described above;
R⁸ and R⁹ are independently selected from a group consisting of hydrogen, fluorine, OR⁶, alkyl, and perfluoroalkyl,
or
R⁸ and R⁹ taken together form a monocyclic or a bicyclic ring system which is saturated or partially unsaturated and optionally have additional heteroatoms selected from O, N or S, the said ring system may further be optionally substituted with 1 to 4 substituents independently selected from halo, alkyl, alkenyl, alkynyl, nitro, cyano, oxo, -OR⁶, -SR⁶, -NR⁶R⁷, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl.

2. The compound as claimed in claim 1, or its stereoisomers, tautomers, pharmaceutically acceptable salts, polymorphs, solvates and formulations wherein ring-A is selected from 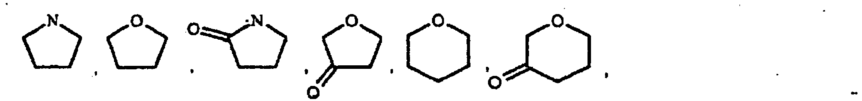 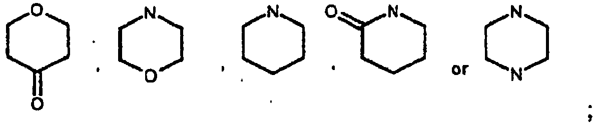
Ring B is selected from 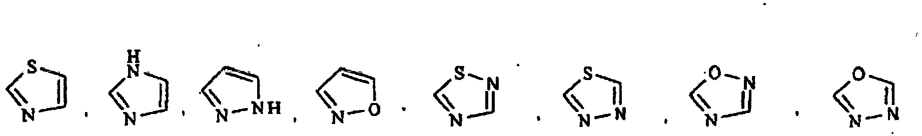 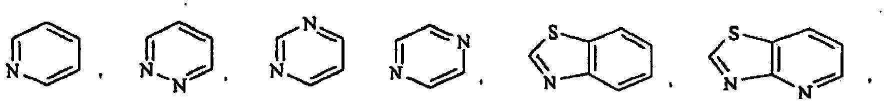 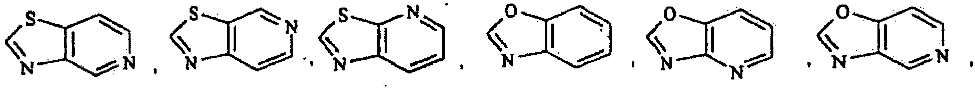 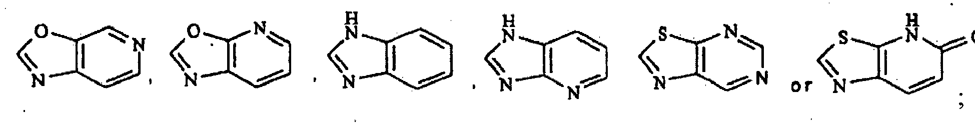 Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino, - NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, =(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
p = 0-2; n= 0-4;
R¹ is selected from C₃-C₆ cycloalkyl;
R⁴ and R⁵ are independently selected from a group consisting of hydrogen, halogen, alkyl, cycloalkyl, heterocyclyl, aryl, heteroaryl, mono, di or tri substituted haloalkyl, nitrile, nitro, -NR⁶R⁷, -OR⁶, -NR⁶C(O)R⁷, -NR⁶C(O)OR⁷, -CR⁸R⁹)ₚC(O)OR⁶, -(CR⁸R⁹)ₙ(CO)NR⁶R⁷, -(CR⁸R⁹)ₙS(O)ₚNR⁶R⁷, -CR⁸R⁹)ₙN(R⁶)C(O)R⁷, -(CR⁸R⁹)ₙOR⁶, -C(R⁸R⁹)ₙNR⁶R⁷, -C(R⁸R⁹)ₙCO(R⁶) and - S(O)ₚC(R⁸R⁹)ₙC(O)OR⁶; wherein each of R⁴ and R⁵ is optionally substituted with one or more substituents selected from halo, straight chain or branched chain alkyl, alkenyl, alkynyl, cycloalkyl, aryl, heteroaryl, heterocyclyl, alkylsulfonyl, oxo, nitro, cyano, -(CR⁸R⁹)ₙCOOR⁶, -C(O)NR⁶R⁷-OR⁶, -SR⁶ or -NR⁶R⁷;
wherein n = 0-4;
R⁶ and R⁷ are independently selected from a group consisting of hydrogen, alkyl, aryl, arylalkyl, heteroaryl, heteroarylalkyl, cycloalkyl, cycloalkylalkyl, heterocyclyl and heterocyclylalkyl; wherein each of R⁶ and R⁷ is optionally substituted with one or more substituents selected from halo, alkyl, cycloalkyl, aryl, heteroaryl, heterocyclyl, alkylsulfonyl, cyano, -(CR⁸R⁹)ₙOR⁶, -(CR⁸R⁹)ₙCOOR⁶ or (CR⁸R⁹)ₙC(O)NR⁶R⁷;
wherein n = 0-4;
or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nitro, cyano, -OR⁶, -SR⁶, - NR⁶R⁷, oxo, alkylsulfonyl, -COOR⁶, - C(O)NR⁶R⁷, cycloalkyl, cycloalkylalkyl, aryl, arylalkyl, heterocyclyl, heterocyclylalkyl, heteroaryl, heteroarylalkyl
wherein R⁶ and R⁷ are as described above;
R⁸ and R⁹ are independently selected from a group consisting of hydrogen, fluorine, alkyl, and perfluoroalkyl;

3. The compound as claimed in claim 1, or its stereoisomers, tautomers, pharmaceutically acceptable salts, polymorphs, solvates and formulations thereof, wherein ring B is selected from 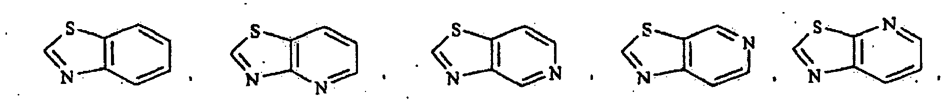 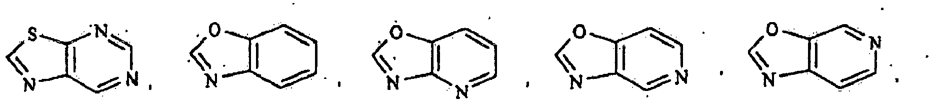 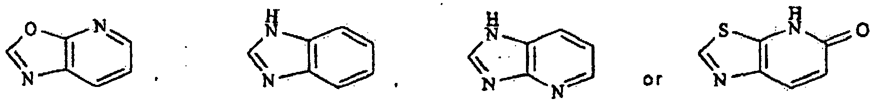
Ring A is selected from 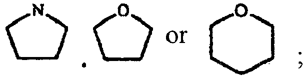 Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino -NR⁶R⁷, -OR⁶, -S(O)ₚR⁶, -S(O)ₚNR⁶R⁷, -NR⁶S(O)ₚR⁷, NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
p = 0-2; n= 0-4;
R¹ is selected from C₃-C₆ cycloalkyl;
R², R³ and R⁵ are hydrogen;
R⁴ is selected from a group consisting of hydrogen, halogen, alkyl, cycloalkyl, heterocyclyl, aryl, heteroaryl, mono, di or tri substituted haloalkyl, nitrile, nitro, -NR⁶R⁷, -OR⁶, -NR⁶C(O)R⁷, -NR⁶C(O)OR⁷, -(CR⁸R⁹)ₙC(O)OR⁶, - (CR⁸R⁹)ₙ(CO)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -C(R⁸R⁹)ₙNR⁶R⁷, -C(R⁸R⁹)ₙCO(R⁶) and -S(O)ₚC(R⁸R⁹)ₙC(O)OR⁶; wherein R⁴ is optionally substituted with one or more substituents selected from halo, straight chain or branched chain alkyl, alkenyl, alkynyl, cycloalkyl, aryl, heteroaryl, heterocyclyl, alkylsulfonyl, oxo, nitro, cyano, -(CR⁸R⁹)ₙCOOR⁶, -C(O)NR⁶R⁷, -OR⁶, -SR⁶ or NR⁶R⁷;
wherein n = 0-4;
R⁶ and R⁷ are independently selected from a groups consisting of hydrogen, alkyl, aryl, heteroaryl, cycloalkyl, heterocyclyl; wherein each of R⁶ and R⁷ is optionally substituted with one or more substituents selected from halo, alkyl, cycloalkyl, aryl, heteroaryl, heterocyclyl, alkylsulfonyl, cyano, -(CR⁸R⁹)ₙOR⁶, - (CR⁸R⁹)ₙCOOR⁶ or -(CR⁸R⁹)ₙC(O)NR⁶R⁷;
wherein n = 0-4;
or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl; alkynyl, nitro, cyano, -OR⁶, -SR⁶, - NR⁶R⁷, oxo, alkylsulfonyl, -COOR⁶, - C(O)NR⁶R⁷, cycloalkyle, cycloalkylalkyl, aryl, arylalkyl, heterocyclyl, heterocyclylalkyl, heteroaryl, heteroarylalkyl
wherein R⁶ and R⁷ are as described above;
R⁸ and R⁹ are independently selected from a group consisting of hydrogen, fluorine, alkyl, and perfluoroalkyl;

4. The compound as claimed in claim 1, or its stereoisomers, tautomers, pharmaceutically acceptable salts, polymorphs, solvates and formulations thereof, wherein Ring B is selected from 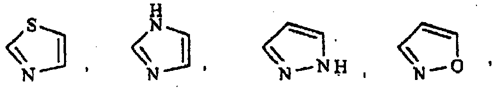 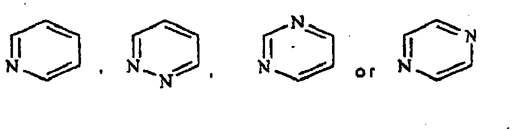
Rings A, B and C are optionally substituted with 1 to 4 substituents independently selected from halogen, monohaloalkyl, dihaloalkyl or perhaloalkyl, monohaloalkoxy, dihaloalkoxy or perhaloalkoxy, cyano, nitro, alkyl, alkenyl, alkynyl, methylenedioxy, amidino -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, - (CR⁸R⁹)ₙC(O)OR⁶, -(CR⁸R⁹)ₙC(O)NR⁶R⁷, -(CR⁸R⁹)ₙC(O)R⁶, cycloalkyl, cycloalkylalkyl, heterocyclyl, heterocycloalkyl, aryl, arylalkyl, heteroaryl, heteroarylalkyl, cycloalkenyl, aryloxy, heteroaryloxy groups;
p = 0-2; n= 0-4;
R¹ is selected from C₃-C₆ cycloalkyl;
R⁴ is -OR⁶, wherein R^{6'} is phenyl or pyridyl which is optionally substituted with one or more substituents selected from halo, alkyl, cycloalkyl, alkylsulfonyl, cyano, - (CR⁸R⁹)ₙOR⁶, - (CR⁸R⁹)ₙCOOR⁶, -(CR⁸R⁹)ₙC(O)NR⁶R⁷;
wherein n = 0-4;
R⁶ and R⁷ are independently selected from a group consisting of hydrogen, alkyl, aryl, arylalkyl, heteroaryl, heteroarylalkyl, cycloalkyl, cycloalkylalkyl, heterocyclyl and heterocyclylalkyl; wherein each of R⁶ and R⁷ is optionally substituted with one or more substituents selected from halo, alkyl, cycloalkyl, aryl, heteroaryl, heterocyclyl, alkylsulfonyl, cyano, -(CR⁸R⁹)ₙOR⁶, -(CR⁸R⁹)ₙCOOR⁶ or -(CR⁸R⁹)ₙC(O)NR⁶R⁷;
wherein n = 0-4;
or
R⁶ and R⁷ taken together form a monocyclic or a bicyclic ring system which may be saturated or partially unsaturated and optionally have additional heteroatoms selected from O, N or S, the said ring system may further be optionally substituted with 1 to 4 substituents independently selected from halo, alkyl, alkenyl, alkynyl, nitro, cyano, -OR⁶, -SR⁶, - NR⁶R⁷, oxo, alkylsulfonyl, -COOR⁶, -C(O)NR⁶R⁷, cycloalkyl, cycloalkylalkyl, aryl, arylalkyl, heterocyclyl, heterocyclylalkyl, heteroaryl, heteroarylalkyl
wherein R⁶ and R⁷ are as described above;
R⁸ and R⁹ are independently selected from a group consisting of hydrogen, fluorine, alkyl, and perfluoroalkyl.

5. A compound as claimed in claim 1 which is
4-{2-[2-(4-Cyclopropanesulfonyl-phenyl)-2-(tetrahydro-pyran-4-yloxy)-acetylamino]-thiazol-5-yloxy}-benzoic acid methyl ester and its (+) and (-) enantiomers;
4-(2-{2-(4-Cyclopropanesulfonyl-phenyl)-2-[(R)-(tetrahydro-furan-3-yl)oxy]-acetylamino}-thiazol-5-yloxy)-benzoic acid methyl ester and its (+) and (-) enantiomers;
2-(4-Cyclopropanesulfonyl-phenyl)-2-(tetrahydro-pyran-4-yloxy)-N-thiazolo[5,4-b]pyridin-2-yl-acetamide and its (+) and (-) enantiomers;
2-(4-Cyclopropanesulfonyl-phenyl)-N-(5-methoxy-thiazolo[5,4-b]pyridin-2-yl)-2-(tetrahydro-pyran-4-yloxy)-acetamide and its (+) and (-) enantiomers;
2-(4-Cyclopropanesulfonyl-phenyl)-N-(5-ethoxy-thiazolo[5,4-b]pyridin-2-yl)-2-(tetrahydro-pyran-4-yloxy)-acetamide and its (+) and (-) enantiomers;
2-(4-Cyclopropanesulfonyl-phenyl)-N-(5-isopropoxy-thiazolo[5,4-b]pyridin-2-yl)-2-(tetrahydro-pyran-4-yloxy)-acetamide and its (+) and (-) enantiomers;
2-(4-Cyclopropanesulfonyl-phenyl)-N-(5-pyrazol-1-yl-thiazol-2-yl)-2-(tetrahydropyran-4-yloxy)-acetamide and its (+) and (-) enantiomers;
2-(4-Cyclopropanesulfonyl-phenyl)-N-(5-morpholin-4-yl-thiazol-2-yl)-2-(tetrahydro-pyran-4-yloxy)-acetamide and its (+) and (-) enantiomers;
2-(4-Cyclopropanesulfonyl-phenyl)-N-(5-fluoro-thiazol-2-yl)-2-(tetrahydro-pyran-4-yloxy)-acetamide and its (+) and (-) enantiomers;
2-(4-Cyclopropanesulfonyl-phenyl)-N-(1-methyl-1H-pyrazol-3-yl)-2-(tetrahydropyran-4-yloxy)-acetamide and its (+) and (-) enantiomers;
2-(4-Cyclopropanesulfonyl-phenyl)-N-pyrazin-2-yl-2-(tetrahydro-pyran-4-yloxy)-acetamide and its (+) and (-) enantiomers;
N-[5-(4-Cyano-phenoxy)-thiazol-2-yl]-2-(4-cyclopropanesulfonyl-phenyl)-2-(tetrahydro-pyran-4-yloxy)-acetamide and its (+) and (-) enantiomers;
2-(4-Cyclopropanesulfonyl-phenyl)-N-(5-methoxy-thiazol-2-yl)-2-(tetrahydropyran-4-yloxy)-acetamide and its (+) and (-) enantiomers;
{5-Chloro-2-[2-(4-cyclopropanesulfonyl-phenyl)-2-tetrahydro-furan-3-yloxy)-acetylamino]-thiazol-4-yl}-aceticacid ethyl ester and its (+) and (-) enantiomers;
4-{2-[2-(4-Cyclopropanesulfonyl-phenyl)-2-(tetrahydro-furan-3-yloxy)-acetylamino]-thiazol-5-yloxy}-benzoic acid methyl ester and its (+) and (-) enantiomers;
(5-Chloro-2-{2-(4-cyclopropanesulfonyl-phenyl)-2-[(R)-(tetrahydro-furan-3-yl)oxy]-acetylamino}-thiazol-4-yl)-acetic acid ethyl ester and its (+) and (-) enantiomers;
4-(2-{2-(4-Cyclopropanesulfonyl-phenyl)-2-[(S)-(tetrahydro-furan-3-yl)oxy]-acetylamino}-thiazol-5-yloxy)-benzoic acid methyl ester and its (+) and (-) enantiomers;
(5-Chloro-2-{2-(4-cyclopropanesulfonyl-phenyl)-2-[(S)-(tetrahydro-furan-3-yl)oxy]-acetylamino}-thiazol-4-yl)-acetic acid ethyl ester and its (+) and (-) enantiomers;
4-{3-[2-(4-Cyclopropanesulfonyl-phenyl)-2-(tetrahydro-pyran-4-yloxy)-acetylamino]-pyrazol-1-ylmethyl}-benzoic acid methyl ester and its (+) and (-) enantiomers;
{5-Chloro-2-[2-(4-cyclopropanesulfonyl-phenyl)-2-(tetrahydro-pyran-4-yloxy)-acetylamino]-thiazol-4-yl}-acetic acid ethyl ester and its (+) and (-) enantiomers;
3-{2-[2-(4-Cyclopropanesulfonyl-phenyl)-2-(tetrahydro-pyran-4-yloxy)-acetylamino]-thiazol-5-yloxy}-benzoic acid methyl ester and its (+) and (-) enantiomers;
4-{2-[2-(4-Cyclopropanesulfonyl-phenyl)-2-(tetrahydro-pyran-4-yloxy)-acetylamino]-thiazol-5-yloxy}-2-fluoro-benzoic acid methyl ester and its (+) and (-) enantiomers;
4-{2-[2-(4-Cyclopropanesulfonyl-phenyl)-2-(tetrahydro-pyran-4-yloxy)-acetylamino]-thiazol-5-yloxy}-3-fluoro-benzoic acid methyl ester and its (+) and (-) enantiomers;
{2-[2-(4-Cyclopropanesulfonyl-phenyl)-2-(tetrahydro-pyran-4-yloxy)-acetylamino]-thiazolo[5,4-b]pyridin-5-yloxy}-acetic acid ethyl ester and its (+) and (-) enantiomers;
4-{2-[2-(4-Cyclopropanesulfonyl-phenyl)-2-(tetrahydro-pyran-4-yloxy)-acetylamino]-thiazolo[5,4-b]pyridin-5-yloxy}-benzoic acid methyl ester and its (+) and (-) enantiomers;
2-(4-Cyclopropanesulfonyl-phenyl)-N-(6-methoxy-benzothiazol-2-yl)-2-(tetrahydro-pyran-4-yloxy)-acetamide and its (+) and (-) enantiomers;
2-(4-Cyclopropanesulfonyl-phenyl)-N-(5-methoxy-thiazolo[5,4-b]pyridin-2-yl)-2-[(R)-(tetrahydro-furan-3-yl)oxy]-acetamide and its (+) and (-) enantiomers;
4-[(4-Cyclopropanesulfonyl-phenyl)-(5-fluoro-thiazol-2-ylcarbamoyl)-methoxy]-piperidine-1-carboxylic acid tert-butyl ester and its (+) and (-) enantiomers;
2-(4-Cyclopropanesulfonyl-phenyl)-N-(5-fluoro-thiazol-2-yl)-2-(piperidin-4-yloxy)-acetamide and its (+) and (-) enantiomers;
(4-{2-[2-(4-Cyclopropanesulfonyl-phenyl)-2-(tetrahydro-pyran-4-yloxy)-acetylamino]-thiazol-5-yloxy}-phenyl)-acetic acid methyl ester and its (+) and (-) enantiomers;
2-[2-(4-Cyclopropanesulfonyl-phenyl)-2-(tetrahydro-pyran-4-yloxy)-acetylamino]-benzothiazole-6-carboxylic acid methyl ester and its (+) and (-) enantiomers;
2-{2-(4-Cyclopropanesulfonyl-phenyl)-2-[(R)-(tetrahydro-furan-3-yl)oxy]-acetylamino}-benzothiazole-6-carboxylic acid methyl ester and its (+) and (-) enantiomers;
2-(4-C-yclopropanesulfonyl-phenyl)-N-(5-oxo-4,5-dihydro-thiazolo[5,4-b]pyridin-2-yl)-2-(tetrahydro-pyran-4-yloxy)-acetamide and its (+) and (-) enantiomers;
2-(4-Cyclopropanesulfonyl-phenyl)-N-(5-isopropoxy-thiazolo[5,4-b]pyridin-2-yl)-2-[(R)-(tetrahydro-furan-3-yl)oxy]-acetamide and its (+) and (-) enantiomers;
4-{2-[2-(4-Cyclopropanesulfonyl-phenyl)-2-(tetrahydro-pyran-4-yloxy)-acetylamino]-thiazol-5-yloxy}-2-methyl-benzoic acid methyl ester and its (+) and (-) enantiomers;
2-[2-(4-Cyclopropanesulfonyl-phenyl)-2-(tetrahydro-pyran-4-yloxy)-acetylamino]-5-methyl-thiazole-4-carboxylic acid methyl ester and its (+) and (-) enantiomers;
N-(5-Bromo-thiazolo[5,4-b]pyridin-2-yl)-2-(4-cyclopropanesulfonyl-phenyl)-2-(tetrahydro-pyran-4-yloxy)-acetamide and its (+) and (-) enantiomers;
2-(4-Cyclopropanesulfonyl-phenyl)-N-[5-(4-formyl-phenoxy)-thiazol-2-yl]-2-(tetrahydro-pyran-4-yloxy)-acetamide and its (+) and (-) enantiomers;
2-(4-Cyclopropanesulfonyl-phenyl)-2-(tetrahydro-pyran-4-yloxy)-N-(5-vinyl-thiazol-2-yl)-acetamide and its (+) and (-) enantiomers;
2-(4-Cyclopropanesulfonyl-phenyl)-2-(tetrahydro-pyran-4-yloxy)-N-(4-vinyl-thiazol-2-yl)-acetamide and its (+) and (-) enantiomers;
4-{2-[2-(4-Cyclopropanesulfonyl-phenyl)-2-(tetrahydro-pyran-4-yloxy)-acetylamino]-thiazol-5-yloxy}-benzoic acid and its (+) and (-) enantiomers;
4-(2-{2-(4-Cyclopropanesulfonyl-phenyl)-2-[(R)-(tetrahydro-furan-3-yl)oxy]-acetylamino}-thiazol-5-yloxy)-benzoic acid and its (+) and (-) enantiomers;
{5-Chloro-2-[2-(4-cyclopropanesulfonyl-phenyl)-2-(tetrahydro-furan-3-yloxy)-acetylamino]-thiazol-4-yl}-acetic acid and its (+) and (-) enantiomers;
4-{2-[2-(4-Cyclopropanesulfonyl-phenyl)-2-(tetrahydro-furan-3-yloxy)-acetylamino]-thiazol-5-yloxy}-benzoic acid and its (+) and (-) enantiomers;
(5-Chloro-2-{2-(4-cyclopropanesulfonyl-phenyl)-2-[(R)-(tetrahydro-furan-3-yl)oxy]-acetylamino}-thiazol-4-yl)-acetic acid and its (+) and (-) enantiomers;
4-(2-{2-(4-Cyclopropanesulfonyl-phenyl)-2-[(S)-(tetrahydro-furan-3-yl)oxy]-acetylamino}-thiazol-5-yloxy)-benzoic acid and its (+) and (-) enantiomers;
(5-Chloro-2-{2-(4-cyclopropanesulfonyl-phenyl)-2-[(S)-(tetrahydro-furan-3-yl)oxy]-acetylamino}-thiazol-4-yl)-acetic acid and its (+) and (-) enantiomers;
4-{3-[2-(4-Cyclopropanesulfonyl-phenyl)-2-(tetrahydro-pyran-4-yloxy)-acetylamino]-pyrazol-1-ylmethyl}-benzoic acid and its (+) and (-) enantiomers;
{5-Chloro-2-[2-(4-cyclopropanesulfonyl-phenyl)-2-(tetrahydro-pyran-4-yloxy)-acetylamino]-thiazol-4-yl}-acetic acid and its (+) and (-) enantiomers;
3-{2-[2-(4-Cyclopropanesulfonyl-phenyl)-2-(tetrahydro-pyran-4-yloxy)-acetylamino]-thiazol-5-yloxy}-benzoic acid and its (+) and (-) enantiomers;
4-{2-[2-(4-Cyclopropanesulfonyl-phenyl)-2-(tetrahydro-pyran-4-yloxy)-acetylamino]-thiazol-5-yloxy}-2-fluoro-benzoic acid and its (+) and (-) enantiomers;
4-{2-[2-(4-Cyclopropanesulfonyl-phenyl)-2-(tetrahydro-pyran-4-yloxy)-acetylamino]-thiazol-5-yloxy}-3-fluoro-benzoic acid and its (+) and (-) enantiomers;
{2-[2-(4-Cyclopropanesulfonyl-phenyl)-2-(tetrahydro-pyran-4-yloxy)-acetylamino]-thiazolo[5,4-b]pyridin-5-yloxy}-acetic acid and its (+) and (-) enantiomers;
4-{2-[2-(4-Cyclopropanesulfonyl-phenyl)-2-(tetrahydro-pyran-4-yloxy)-acetylamino]-thiazolo[5,4-b]pyridin-5-yloxy}-benzoic acid and its (+) and (-) enantiomers;
4-[(4-Carboxymethyl-5-chloro-thiazol-2-ylcarbamoyl)-(4-cyclopropanesulfonyl-phenyl)-methoxy]-piperidine-1-carboxylic acid tert-butyl ester and its (+) and (-) enantiomers;
{5-Chloro-2-[2-(4-cyclopropanesuifonyl-phenyl)-2-(piperidin-4-yloxy)-acetylamino]-thiazol-4-yl}-acetic acid and its (+) and (-) enantiomers;
(4-{2-[2-(4-Cyclopropanesulfonyl-phenyl)-2-(tetrahydro-pyran-4-yloxy)-acetylamino]-thiazol-5-yloxy}-phenyl)-acetic acid and its (+) and (-) enantiomers;
2-[2-(4-Cyclopropanesulfonyl-phenyl)-2-(tetrahydro-pyran-4-yloxy)-acetylamino]-benzothiazole-6-carboxylic acid and its (+) and (-) enantiomers;
2-{2-(4-Cyclopropanesulfonyl-phenyl)-2-[(R)-(tetrahydro-furan-3-yl)oxy]-acetylamino}-benzothiazole-6-carboxylic acid and its (+) and (-) enantiomers;
4-{2-[2-(4-Cyclopropanesulfonyl-phenyl)-2-(tetrahydro-pyran-4-yloxy)-acetylamino]-thiazol-5-yloxy}-2-methyl-benzoic acid and its (+) and (-) enantiomers;
2-[2-(4-Cyclopropanesulfonyl-phenyl)-2-(tetrahydro-pyran-4-yloxy)-acetylamino]-5-methyl-thiazole-4-carboxylic acid and its (+) and (-) enantiomers;
N-{5-[4-(Azetidine-1-carbonyl)-phenoxy]-thiazol-2-yl}-2-(4-cyclopropanesulfonyl-phenyl)-2-(tetrahydro-pyran-4-yloxy)-acetamide and its (+) and (-) enantiomers;
4-{2-[2-(4-Cyclopropanesulfonyl-phenyl)-2-(tetrahydro-furan-3-yloxy)-acetylamino]-thiazol-5-yloxy}-benzamide and its (+) and (-) enantiomers;
N-[4-(2-Azetidin-1-yl-2-oxo-ethyl)-5-chloro-thiazol-2-yl]-2-(4-cyclopropanesulfonyl-phenyl)-2-(tetrahydro-pyran-4-yloxy)-acetamide and its (+) and (-) enantiomers;
2-(4-Cyclopropanesulfonyl-phenyl)-2-(tetrahydro-pyran-4-yloxy)-N-{5-[4-(2H-tetrazol-5-yl)-phenoxy]-thiazol-2-yl}-acetamide and its (+) and (-) enantiomers;
2-(4-Cyclopropanesulfonyl-phenyl)-N-[5-(4-hydroxymethyl-phenoxy)-thiazol-2-yl]-2-(tetrahydro-pyran-4-yloxy)-acetamide and its (+) and (-) enantiomers;
2-(4-Cyclopropanesulfonyl-phenyl)-N-(4-hydroxymethyl-5-methyl-thiazol-2-yl)-2-(tetrahydro-pyran-4-yloxy)-acetamide and its (+) and (-) enantiomers;
2-(4-Cyclopropanesulfonyl-phenyl)-N-[5-(1,2-dihydroxy-ethyl)-thiazol-2-yl]-2-(tetrahydro-pyran-4-yloxy)-acetamide and its (+) and (-) enantiomers;
2-(4-Cyclopropanesulfonyl-phenyl)-N-[5-(1,2-dihydroxy-ethyl)-thiazolo[5,4-b]pyridin-2-yl]-2-(tetrahydro-pyran-4-yloxy)-acetamide and its (+) and (-) enantiomers;
2-(4-Cyclopropanesulfonyl-phenyl)-N-[4-(1,2-dihydroxy-ethyl)-thiazol-2-yl]-2-(tetrahydro-pyran-4-yloxy)-acetamide and its (+) and (-) enantiomers;
2-(4-Cyclopropanesulfonyl-phenyl)-N-[3-(1,2-dihydroxy-ethyl)-[1,2,4]thiadiazol-5-yl]-2-(tetrahydro-pyran-4-yloxy)-acetamide and its (+) and (-) enantiomers;
{5-Chloro-2-[2-(4-cyclopropanesulfonyl-phenyl)-2-(2-methyl-tetrahydro-pyran-3-yloxy)-acetylamino]-thiazol-4-yl}-acetic acid and its (+) and (-) enantiomers;
2-[2-[4-(3-Oxo-cyclopentanesulfonyl)-phenyl]-2-(tetrahydro-furan-3-yloxy)-acetylamino]-thiazole-4-carboxylic acid and its (+) and (-) enantiomers;
N-[4-(1-Acetyl-piperidin-4-yl)-thiazol-2-yl]-2-(4-cyclopropanesulfonyl-phenyl)-2-(tetrahydro-pyran-4-yloxy)-acetamide and its (+) and (-) enantiomers;
2-(4-Cyclopropanesulfonyl-phenyl)-N-{5-[4-(1,2-dihydroxy-ethyl)-phenoxy]-thiazol-2-yl}-2-(tetrahydro-pyran-4-yloxy)-acetamide and its (+) and (-) enantiomers;
2-(4-Cyclopropanesulfonyl-phenyl)-N-(4,5,6,7-tetrahydro-benzothiazol-2-yl)-2-(tetrahydro-pyran-4-yloxy)-acetamide and its (+) and (-) enantiomers;
2-(4-Cyclopropanesulfonyl-phenyl)-2-(tetrahydro-pyran-4-yloxy)-N-thiazolo[5,4-d]pyrimidin-2-yl-acetamide and its (+) and (-) enantiomers;
2-(4-Cyclopropanesulfonyl-phenyl)-N-[5-(4-hydroxymethyl-phenoxy)-thiazol-2-yl]-2-(tetrahydro-pyran-4-yloxy)-acetamide and its (+) and (-) enantiomers;
2-(4-Cyclopropanesulfonyl-phenyl)-N-[4-(1,2-dihydroxy-ethyl)-thiazol-2-yl]-2-(tetrahydro-pyran-4-yloxy)-acetamide and its (+) and (-) enantiomers;
2-(4-Cyclopropanesulfonyl-phenyl)-N-[4-(1,2-dihydroxy-ethyl)-5-fluoro-thiazol-2-yl]-2-(tetrahydro-pyran-4-yloxy)-acetamide and its (+) and (-) enantiomers;
2-(4-Cyclopropanesulfonyl-phenyl)-N-[5-(1,2-dihydroxy-ethyl)-thiazol-2-yl]-2-(tetrahydro-pyran-4-yloxy)-acetamide and its (+) and (-) enantiomers;
2-(4-Cyclopropanesulfonyl-phenyl)-N-[5-(1,2-dihydroxy-ethyl)-thiazolo[5,4-b]pyridin-2-yl]-9-(tetrahydro-pyran-4-yloxy)-acetamide and its (+) and (-) enantiomers;
2-(3-{2-(4-Cyclopropanesulfonyl-phenyl)-2-[(R)-(tetrahydro-furan-3-yl)oxy]-acetylamino}-pyrazol-1-yl)-2-methyl-propionic acid and its (+) and (-) enantiomers.

6. A compound of formula (I), as claimed in any one of the claims 1 to 5, or its stereoisomers, tautomers, pharmaceutically acceptable salts, polymorphs, solvates and formulations thereof, for use in treating a disease through Glucokinase activation.

7. A compound of formula (I), as claimed in any one of the claims 1 to 5, or its stereoisomers, tautomers, pharmaceutically acceptable salts, polymorphs, solvates and formulations thereof, for use in treating a disease through Glucokinase deinhibition.

8. A compound of formula (I), as claimed in any one of the claims 1 to 5, or its stereoisomers, tautomers, pharmaceutically acceptable salts, polymorphs, solvates and formulations thereof, for use in prophylactic or therapeutic treatment of hyperglycemia or diabetes, particularly type II diabetes.

9. A compound of formula (I), as claimed in any one of the claims 1 to 5, or its stereoisomers, tautomers, pharmaceutically acceptable salts, polymorphs, solvates and formulations thereof, for use in preventing diabetes, particularly type II diabetes, in a human demonstrating pre-diabetic hyperglycemia or impaired glucose tolerance.

10. A compound of formula (I), as claimed in any one of the claims 1 to 5, or its stereoisomers, tautomers, pharmaceutically acceptable salts, polymorphs, solvates and formulations thereof, for use in combined treatment or prevention of diabetes and obesity.

11. A compound of formula (I), as claimed in any one of the claims 1 to 5, or its stereoisomers, tautomers, pharmaceutically acceptable salts, polymorphs, solvates and formulations thereof, for use in treating or preventing obesity.

12. A compound of formula (I), as claimed in any one of the claims 1 to 5, or its stereoisomers, tautomers, pharmaceutically acceptable salts, polymorphs, solvates and formulations thereof, for use in treatment or prevention of dyslipidemia.

13. A compound of formula (I), as claimed in any one of the claims 1 to 5, or its stereoisomers, tautomers, pharmaceutically acceptable salts, polymorphs, solvates and formulations thereof, for use in treating hyperglycemia, IGT, Syndrome X, type 2 diabetes, type 1 diabetes, dyslipidemia or hyperlipidemia, hypertension, for use in the treatment or prophylaxis of obesity, for use in lowering of food intake, for use in appetite regulation; and for use in regulating feeding behaviour.

14. A compound of formula (I), as claimed in any one of the claims 1 to 5, or its stereoisomers, tautomers, pharmaceutically acceptable salts, polymorphs, solvates and formulations thereof, for use in enhancing the secretion of enteroincretins, like GLP-1 and GIP, thereby managing diseases or disorders associated with modulation of secretions of enteroincretins, like hyperglycemia, insulin resistance, impaired glucose tolerance, obesity, gastric emptying, gastroparesis, satiety, leptin resistance, dyslipidemia, wound healing, diabetic complications, such as nephropathy, retinopathy, neuropathy and cataracts.

15. A pharmaceutical composition comprising, as an active ingredient, at least one compound of formula (I), as claimed in any one of the claims 1 to 5, or its stereoisomers, tautomers; pharmaceutically acceptable salts, polymorphs, and solvates thereof, together with one or more pharmaceutically acceptable carriers or excipients.

16. A pharmaceutical composition comprising, as an active ingredient, at least one compound of formula (I), as claimed in any one of the claims 1 to 5, or its stereoisomers, tautomers, pharmaceutically acceptable salts, polymorphs, and solvates thereof, in combination with one or more pharmaceutically acceptable therapeutically active agents.

17. The pharmaceutical composition as claimed in claim 16 wherein, the pharmaceutically acceptable therapeutically active agent is selected from antidiabetic agents, anti-hyperglycemic agents, anti-obesity agents, anti-hypertensive agents or anti-dyslipidemic agents.

18. The pharmaceutical composition as claimed in claim 16 or claim 17 wherein the pharmaceutically acceptable therapeutically active agent is selected from insulin secretagogues like sulfonylureas selected from amaryl, glyburide, glimepiride, glipyride, glipizide; insulinotropic sulfonyl urea receptor ligands like meglitinides selected from nateglinide, rapaglinide; biguanides like metformin, phenformin, buformin; glucagon antagonists like a peptide or non-peptide glucagon antagonist; glucosidase inhibitors like acarbose, miglitol; glucose sensitive insulinotropic agents like GLP-1, GLP-1 mimetics like exendin-4; insulin sensitizers like troglitazone, rosiglitazone, pioglitazone; dipeptidyl peptidase-IV inhibitors like sitagliptin, vildagliptin; sibutramine, orlistat, rimonabant; fibrates like gemfibrozil, fenofibrate; niacin; statins like rosuvastatin, atorvastatin, simvastatin; cholesterol absorption inhibitors like ezetimibe; bile acid sequestrants like cholestyramine; diuretics like hydrochlorothiazides, mannitol, indapamide, furosemide; angiotensin converting enzyme (ACE) inhibitors like captopril, enalapril; angiotensin-II receptor type-I blockers (ARB) like losartan, irbesartan; rennin inhibitors like aliskerin; β-adrenergic receptor blockers like atenolol, metoprolol; calcium channel blockers like amlodipine, nifedipine; aldosterone receptor antagonists like spironolactone, aldosterone synthase inhibitors like FAD286.

19. Use of a compound of formula (I) as claimed in any of the claims 1-5, its polymorphs, stereoisomers, pharmaceutically acceptable salt, or solvate thereof, for the manufacture of a medicament for the activation of Glucokinase.

20. Use of a compound of formula (I) as claimed in any of the claims 1-5, its polymorphs, stereoisomers, pharmaceutically acceptable salt, or solvate thereof, for the manufacture of a medicament for the prophylactic or therapeutic treatment of obesity.

21. Use of a compound of formula (I) as claimed in any of the claims 1-5, its polymorphs, stereoisomers, pharmaceutically acceptable salt, or solvate thereof, for the manufacture of a medicament for the prophylactic or therapeutic treatment of hyperglycemia or diabetes, particularly type II diabetes.

22. Use of a compound of formula (I) as claimed in any of the claims 1-5, its polymorphs, stereoisomers, pharmaceutically acceptable salt, or solvate thereof, for the manufacture of a medicament for the prevention of diabetes, particularly type II diabetes, in a human demonstrating pre-diabetic hyperglycemia or impaired glucose tolerance.

23. A process for the preparation of a compound of formula (I) as claimed in any one of the claims 1 to 5 or its stereoisomers, tautomers, pharmaceutically acceptable salts, polymorphs, and solvates thereof, said process comprising: reacting an acid of formula (II) 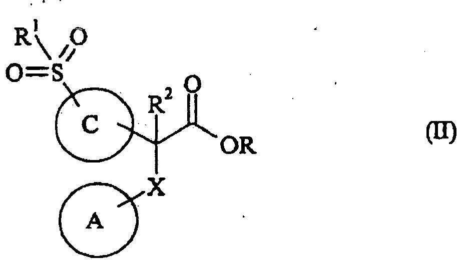 wherein R is hydrogen, alkyl or arylalkyl, with a compound of formula (III) 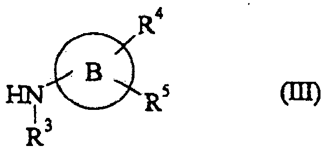 in presence of a suitable amide coupling reagent, optionally hydrolysing and optionally further coupling with an amine of formula NHR⁶R⁷ to obtain the compound of formula (I).

## Patentansprüche

1. Eine Verbindung der Formel (I) 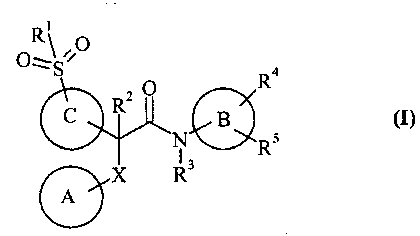 oder deren Stereoisomere, Tautomere, pharmazeutisch verträgliche Salze, Polymorphe, Solvate und Formulierungen, wobei
R¹ ein Cycloalkyl darstellt; R² und R³ Wasserstoff darstellen;
R⁴ und R⁵ unabhängig ausgewählt sind aus einer Gruppe bestehend aus Wasserstoff, Halogen, Alkyl, Cycloalkyl, Heterocyclyl, Aryl, Heteroaryl, mono-, di- oder trisubstituiertem Halogenalkyl, Nitril, -NR⁶C(O)OR⁷, -(CR⁸R⁹)ₙC(O)OR⁶, -(CR⁸R⁹)ₙC(O)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -(CR⁸R⁹)ₙNR⁶R⁷, -C(R⁸R⁹)ₙCO(R⁶) und -S(O)ₚC(R⁸R⁹)ₙC(O)OR⁶; wobei jeder der Reste R⁴ und R⁵ gegebenenfalls mit einem oder mehreren Substituenten, ausgewählt aus Halogen, Alkyl, Alkenyl, Alkinyl, Cycloalkyl, Aryl, Heteroaryl, Heterocyclyl, Alkylsulfonyl, Oxo, Nitro, Cyano, -(CR⁸R⁹)ₙR⁶, -(CR⁸R⁹)ₙCOOR⁶, -(CR⁸R⁹)ₙC(O)NR⁶R⁷, -(CR⁸R⁹)ₙOR⁶, -SR⁶ oder -NR⁶R⁷ substituiert ist; wobei n = 0-4;
X O darstellt;
Ring A ein monocyclisches Heterocyclyl darstellt;
Ring B Heteroaryl darstellt und Ring C Phenyl darstellt;
die Ringe A, B und C gegebenenfalls mit 1 bis 4 Substituenten unabhängig ausgewählt aus Halogen, Monohalogenalkyl, Dihalogenalkyl oder Perhalogenalkyl, Monohalogenalkoxy, Dihalogenalkoxy oder Perhalogenalkoxy, Cyano, Nitro, Alkyl, Alkenyl, Alkinyl, Methylendioxy, Amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, Cycloalkyl, Cycloalkylalkyl, Heterocyclyl, Heterocycloalkyl, Aryl, Arylalkyl, Heteroaryl, Heteroarylalkyl, Cycloalkenyl, Aryloxy, Heteroaryloxygruppen substituiert sind;
p = 0-2; n = 0-4;
R⁶ und R⁷ unabhängig ausgewählt sind aus einer Gruppe bestehend aus Wasserstoff, Alkyl, Alkenyl, Alkinyl, Aryl, Arylalkyl, Heteroaryl, Heteroarylalkyl, Cycloalkyl, Cycloalkylalkyl, Heterocyclyl und Heterocylylalkyl, wobei jeder der Reste R⁶ und R⁷ gegebenenfalls mit einem oder mehreren Substituenten ausgewählt aus Halogen, Alkyl, Cycloalkyl, Aryl, Heteroaryl, Heterocyclyl, Alkylsulfonyl, Cyano, -(CR⁸R⁹)ₙOR⁶, -(CR⁸R⁹)ₙCOOR⁶ oder -(CR⁸R⁹)ₙC(O)NR⁶R⁷ substituiert ist; wobei n = 0-4;
oder
R⁶ und R⁷ zusammen ein monocyclisches oder bicyclisches Ringsystem bilden, welches gesättigt oder teilweise ungesättigt sein kann und gegebenenfalls zusätzliche Heteroatome ausgewählt aus O, N oder S aufweist, wobei das Ringsystem ferner gegebenenfalls mit 1 bis 4 Substituenten unabhängig ausgewählt aus Halogen, Alkyl, Alkenyl, Alkinyl, Nitro, Cyano, -OR⁶, -SR⁶, -NR⁶R⁷, Oxo, Alkylsulfonyl, -COOR⁶, -C(O)NR⁶R⁷, Cycloalkyl, Cycloalkylalkyl, Aryl, Arylalkyl, Heterocyclyl, Heterocyclylalkyl, Heteroaryl, Heteroarylalkyl substituiert sein kann;
wobei R⁶ und R⁷ wie oben beschrieben sind;
R⁸ und R⁹ unabhängig ausgewählt sind aus einer Gruppe bestehend aus Wasserstoff, Fluor, OR⁶, Alkyl und Perfluoralkyl,
oder
R⁸ und R⁹ zusammen ein monocyclisches oder bicyclisches Ringsystem bilden, welches gesättigt oder teilweise ungesättigt ist und gegebenenfalls zusätzliche Heteroatome ausgewählt aus O, N oder S aufweist, wobei das Ringsystem ferner gegebenenfalls mit 1 bis 4 Substituenten unabhängig ausgewählt aus Halogen, Alkyl, Alkenyl, Alkinyl, Nitro, Cyano, Oxo, -OR⁶, -SR⁶, -NR⁶R⁷, Alkylsulfonyl, -COOR⁶, -C(O)NR⁶R⁷, Cycloalkyl, Cycloalkylalkyl, Aryl, Arylalkyl, Heterocyclyl, Heterocyclylalkyl, Heteroaryl, Heteroarylalkyl substituiert sein kann.

2. Die Verbindung wie in Anspruch 1 beansprucht, oder deren Stereoisomere, Tautomere, pharmazeutisch verträglichen Salze, Polymorphe, Solvate und Formulierungen, wobei Ring-A ausgewählt ist aus 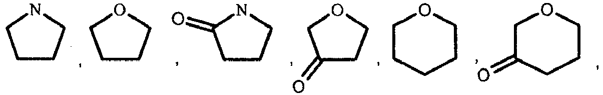 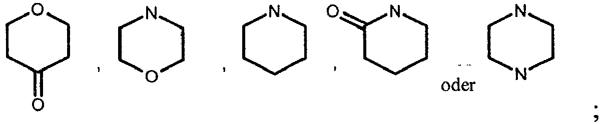 Ring B ausgewählt ist aus 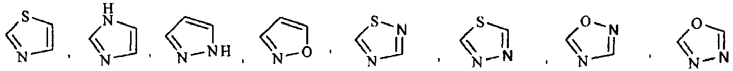 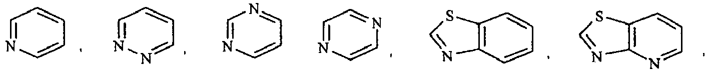 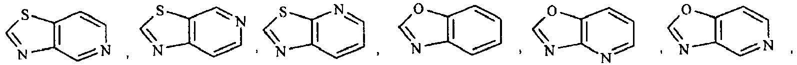 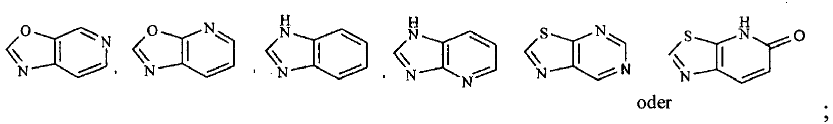
die Ringe A, B und C gegebenenfalls mit 1 bis 4 Substituenten unabhängig ausgewählt aus Halogen, Monohalogenalkyl, Dihalogenalkyl oder Perhalogenalkyl, Monohalogenalkoxy, Dihalogenalkoxy oder Perhalogenalkoxy, Cyano, Nitro, Alkyl, Alkenyl, Alkinyl, Methylendioxy, Amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, Cycloalkyl, Cycloalkylalkyl, Heterocyclyl, Heterocycloalkyl, Aryl, Arylalkyl, Heteroaryl, Heteroarylalkyl, Cycloalkenyl, Aryloxy, Heteroaryloxygruppen substituiert sind;
p = 0-2; n = 0-4;
R¹ ausgewählt ist aus C₃-C₆-Cycloalkyl;
R⁴ und R⁵ unabhängig ausgewählt sind aus einer Gruppe bestehend aus Wasserstoff, Halogen, Alkyl, Cycloalkyl, Heterocyclyl, Aryl, Heteroaryl, mono-, di- oder trisubstituiertem Halogenalkyl, Nitril, Nitro, -NR⁶R⁷, -OR⁶, -NR⁶C(O)R⁷, -NR⁶C(O)OR⁷, -(CR⁸R⁹)ₙC(O)OR⁶, -(CR⁸R⁹)ₙ(CO)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -C(CR⁸R⁹)ₙNR⁶R⁷, -C(R⁸R⁹)ₙCO(R⁶) und -S(O)ₚC(R⁸R⁹)ₙC(O)OR⁶; wobei jeder der Reste R⁴ und R⁵ gegebenenfalls mit einem oder mehreren Substituenten ausgewählt aus Halogen, geradkettigem oder verzweigtkettigem Alkyl, Alkenyl, Alkinyl, Cycloalkyl, Aryl, Heteroaryl, Heterocyclyl, Alkylsulfonyl, Oxo, Nitro, Cyano, -(CR⁸R⁹)ₙCOOR⁶, -C(O)NR⁶R⁷, -OR⁶, -SR⁶ oder -NR⁶R⁷ substituiert ist;
wobei n = 0-4;
R⁶ und R⁷ unabhängig ausgewählt sind aus einer Gruppe bestehend aus Wasserstoff, Alkyl, Aryl, Arylalkyl, Heteroaryl, Heteroarylalkyl, Cycloalkyl, Cycloalkylalkyl, Heterocyclyl und Heterocyclylalkyl; wobei jeder der Reste R⁶ und R⁷ gegebenenfalls mit einem oder mehreren Substituenten ausgewählt aus Halogen, Alkyl, Cycloalkyl, Aryl, Heteroaryl, Heterocyclyl, Alkylsulfonyl, Cyano, -(CR⁸R⁹)ₙOR⁶, -(CR⁸R⁹)ₙCOOR⁶ oder -(CR⁸R⁹)ₙC(O)NR⁶R⁷ substituiert ist;
wobei n = 0-4;
oder
R⁶ und R⁷ zusammen ein monocyclisches oder ein bicyclisches Ringsystem bilden, welches gesättigt oder teilweise ungesättigt sein kann und gegebenenfalls zusätzliche Heteroatome ausgewählt aus O, N oder S aufweist, wobei das Ringsystem ferner gegebenenfalls mit 1 bis 4 Substituenten unabhängig ausgewählt aus Halogen, Alkyl, Alkenyl, Alkinyl, Nitro, Cyano, -OR⁶, -SR⁶, -NR⁶R⁷, Oxo, Alkylsulfonyl, -COOR⁶, -C(O)NR⁶R⁷, Cycloalkyl, Cycloalkylalkyl, Aryl, Arylalkyl, Heterocyclyl, Heterocyclylalkyl, Heteroaryl, Heteroarylalkyl substituiert sein kann;
wobei R⁶ und R⁷ wie oben beschrieben sind;
R⁸ und R⁹ unabhängig ausgewählt sind aus einer Gruppe bestehend aus Wasserstoff, Fluor, Alkyl und Perfluoralkyl.

3. Die Verbindung wie in Anspruch 1 beansprucht oder deren Stereoisomere, Tautomere, pharmazeutisch verträglichen Salze, Polymorphe, Solvate und Formulierungen davon, wobei Ring B ausgewählt ist aus 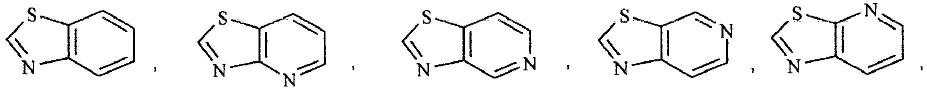 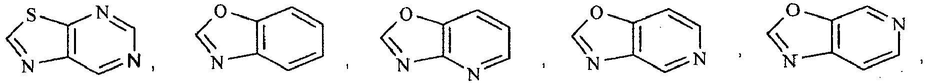 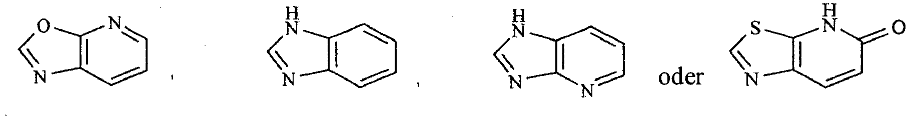 Ring A ausgewählt ist aus 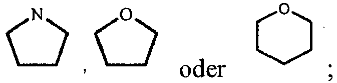
die Ringe A, B und C gegebenenfalls mit 1 bis 4 Substituenten unabhängig ausgewählt aus Halogen, Monohalogenalkyl, Dihalogenalkyl oder Perhalogenalkyl, Monohalogenalkoxy, Dihalogenalkoxy oder Perhalogenalkoxy, Cyano, Nitro, Alkyl, Alkenyl, Alkinyl, Methylendioxy, Amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, Cycloalkyl, Cycloalkylalkyl, Heterocyclyl, Heterocycloalkyl, Aryl, Arylalkyl, Heteroaryl, Heteroarylalkyl, Cycloalkenyl, Aryloxy, Heteroaryloxygruppen substituiert sind;
p = 0-2; n = 0-4;
R¹ ausgewählt ist aus C₃-C₆-Cycloalkyl;
R², R³ und R⁵ für Wasserstoff stehen;
R⁴ ausgewählt ist aus einer Gruppe bestehend aus Wasserstoff, Halogen, Alkyl, Cycloalkyl, Heterocyclyl, Aryl, Heteroaryl, mono-, di- oder trisubstituiertem Halogenalkyl, Nitril, Nitro, NR⁶R⁷, -OR⁶, -NR⁶C(O)R⁷, -NR⁶C(O)OR⁷, -(CR⁸R⁹)ₙC(O)OR⁶, -(CR⁸R⁹)ₙ(CO)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -(CR⁸R⁹)ₙNR⁶R⁷, -C(R⁸R⁹)ₙCO(R⁶) und -S(O)ₚC(R⁸R⁹)ₙC(O)OR⁶; wobei R⁴ gegebenenfalls mit einem oder mehreren Substituenten ausgewählt aus Halogen, geradkettigem oder verzweigtkettigem Alkyl, Alkenyl, Alkinyl, Cycloalkyl, Aryl, Heteroaryl, Heterocyclyl, Alkylsulfonyl, Oxo, Nitro, Cyano, -(CR⁸R⁹)ₙCOOR⁶, -C(O)NR⁶R⁷, -OR⁶, -SR⁶ oder -NR⁶R⁷ substituiert ist;
wobei n = 0-4;
R⁶ und R⁷ unabhängig ausgewählt sind aus einer Gruppe bestehend aus Wasserstoff, Alkyl, Aryl, Heteroaryl, Cycloalkyl, Heterocyclyl und Heterocyclylalkyl; wobei jeder der Reste R⁶ und R⁷ gegebenenfalls mit einem oder mehreren Substituenten ausgewählt aus Halogen, Alkyl, Cycloalkyl, Aryl, Heteroaryl, Heterocyclyl, Alkylsulfonyl, Cyano, -(CR⁸R⁹)ₙOR⁶, -(CR⁸R⁹)ₙCOOR⁶ oder -(CR⁸R⁹)ₙC(O)NR⁶R⁷ substituiert ist;
wobei n = 0-4;
oder
R⁶ und R⁷ zusammen ein monocyclisches oder ein bicyclisches Ringsystem bilden, welches gesättigt oder teilweise ungesättigt sein kann und gegebenenfalls zusätzliche Heteroatome ausgewählt aus O, N oder S aufweist, wobei das Ringsystem ferner gegebenenfalls mit 1 bis 4 Substituenten unabhängig ausgewählt aus Halogen, Alkyl, Alkenyl, Alkinyl, Nitro, Cyano, -OR⁶, -SR⁶, -NR⁶R⁷, Oxo, Alkylsulfonyl, -COOR⁶, -C(O)NR⁶R⁷, Cycloalkyl, Cycloalkylalkyl, Aryl, Arylalkyl, Heterocyclyl, Heterocyclylalkyl, Heteroaryl, Heteroarylalkyl substituiert sein kann;
wobei R⁶ und R⁷ wie oben beschrieben sind;
R⁸ und R⁹ unabhängig ausgewählt sind aus einer Gruppe bestehend aus Wasserstoff, Fluor, Alkyl und Perfluoralkyl.

4. Die Verbindung wie in Anspruch 1 beansprucht, oder deren Stereoisomere, Tautomere, pharmazeutisch verträglichen Salze, Polymorphe, Solvate und Formulierungen davon, wobei Ring B ausgewählt ist aus 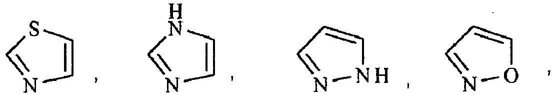 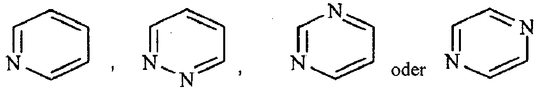
die Ringe A, B und C gegebenenfalls mit 1 bis 4 Substituenten unabhängig ausgewählt aus Halogen, Monohalogenalkyl, Dihalogenalkyl oder Perhalogenalkyl, Monohalogenalkoxy, Dihalogenalkoxy oder Perhalogenalkoxy, Cyano, Nitro, Alkyl, Alkenyl, Alkinyl, Methylendioxy, Amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, Cycloalkyl, Cycloalkylalkyl, Heterocyclyl, Heterocycloalkyl, Aryl, Arylalkyl, Heteroaryl, Heteroarylalkyl, Cycloalkenyl, Aryloxy, Heteroaryloxygruppen substituiert sind;
p = 0-2; n = 0-4;
R¹ ausgewählt ist aus C₃-C₆-Cycloalkyl;
R⁴ für -OR^{6'} steht, wobei R^{6'} für Phenyl oder Pyridyl steht, welches gegebenenfalls mit einem oder mehreren Substituenten ausgewählt aus Halogen, Alkyl, Cycloalkyl, Alkylsulfonyl, Cyano, -(CR⁸R⁹)ₙOR⁶, -(CR⁸R⁹)ₙCOOR⁶, -(CR⁸R⁹)ₙC(O)NR⁶R⁷ substituiert ist;
wobei n = 0-4;
R⁶ und R⁷ unabhängig ausgewählt sind aus einer Gruppe bestehend aus Wasserstoff, Alkyl, Aryl, Arylalkyl, Heteroaryl, Heteroarylalkyl, Cycloalkyl, Cycloalkylalkyl, Heterocyclyl und Heterocyclylalkyl; wobei jeder der Reste R⁶ und R⁷ gegebenenfalls mit einem oder mehreren Substituenten ausgewählt aus Halogen, Alkyl, Cycloalkyl, Aryl, Heteroaryl, Heterocyclyl, Alkylsulfonyl, Cyano, -(CR⁸R⁹)ₙOR⁶, -(CR⁸R⁹)ₙCOOR⁶ oder -(CR⁸R⁹)ₙC(O)NR⁶R⁷ substituiert ist;
wobei n = 0-4;
oder
R⁶ und R⁷ zusammen ein monocyclisches oder ein bicyclisches Ringsystem bilden, welches gesättigt oder teilweise ungesättigt sein kann und gegebenenfalls zusätzliche Heteroatome ausgewählt aus O, N oder S aufweist, wobei das Ringsystem ferner gegebenenfalls mit 1 bis 4 Substituenten unabhängig ausgewählt aus Halogen, Alkyl, Alkenyl, Alkinyl, Nitro, Cyano, -OR⁶, -SR⁶, -NR⁶R⁷, Oxo, Alkylsulfonyl, -COOR⁶, -C(O)NR⁶R⁷, Cycloalkyl, Cycloalkylalkyl, Aryl, Arylalkyl, Heterocyclyl, Heterocyclylalkyl, Heteroaryl, Heteroarylalkyl substituiert sein kann;
wobei R⁶ und R⁷ wie oben beschrieben sind;
R⁸ und R⁹ unabhängig ausgewählt sind aus einer Gruppe bestehend aus Wasserstoff, Fluor, Alkyl und Perfluoralkyl.

5. Eine Verbindung wie in Anspruch 1 beansprucht, welche
4-{2-[2-(4-Cyclopropansulfonylphenyl)-2-(tetrahydro-pyran-4-yloxy)-acetylamino]-thiazol-5-yloxy}-Benzoesäuremethylester und dessen (+)- und (-)-Enantiomere;
4-(2-{2-(4-Cyclopropansulfonylphenyl)-2-[(R)-(tetrahydro-furan-3-yl)oxy]-acetylamino}-thiazol-5-yloxy)-Benzoesäuremethylester und dessen (+)- und (-)-Enantiomere;
2-(4-Cyclopropansulfonylphenyl)-2-(tetrahydro-pyran-4-yloxy)-N-thiazolo[5,4-b]-pyridin-2-yl-acetamid und dessen (+)- und (-)-Enantiomere;
2-(4-Cyclopropansulfonylphenyl)-N-(5-methoxy-thiazolo[5,4-b]pyridin-2-yl)-2-(tetrahydro-pyran-4-yloxy)acetamid und dessen (+)- und (-)-Enantiomere;
2-(4-Cyclopropansulfonylphenyl)-N-(5-ethoxy-thiazolo[5,4-b]pyridin-2-yl)-2-(tetrahydro-pyran-4-yloxy)-acetamid und dessen (+)- und (-)-Enantiomere;
2-(4-Cyclopropansulfonylphenyl)-N-(5-isopropoxy-thiazolo[5,4-b]pyridin-2-yl)-2-(tetrahydro-pyran-4-yloxy)-acetamid und dessen (+)- und (-)-Enantiomere;
2-(4-Cyclopropansulfonylphenyl)-N-(5-pyrazol-1-yl-thiazol-2-yl)-2-(tetrahydro-pyran-4-yloxy)-acetamid und dessen (+)- und (-)-Enantiomere;
2-(4-Cyclopropansulfonylphenyl)-N-(5-morpholin-4-yl-thiazol-2-yl)-2-(tetrahydropyran-4-yloxy)acetamid und dessen (+)- und (-)-Enantiomere;
2-(4-Cyclopropansulfonyl-phenyl)-N-(5-fluor-thiazol-2-yl)-2-(tetrahydro-pyran-4-yloxy)acetamid und dessen (+)- und (-)-Enantiomere;
2-(4-Cyclopropansulfonylphenyl)-N-(1-methyl-1H-pyrazol-3-yl)-2-(tetrahydro-pyran-4-yloxy)acetamid und dessen (+)- und (-)-Enantiomere;
2-(4-Cyclopropansulfonylphenyl)-N-pyrazin-2-yl-2-(tetrahydro-pyran-4-yloxy)acetamid und dessen (+)- und (-)-Enantiomere;
N-[5-(4-Cyanophenoxy)-thiazol-2-yl]-2-(4-cyclopropansulfonylphenyl)-2-(tetrahydropyran-4-yloxy)-acetamid und dessen (+)- und (-)-Enantiomere;
2-(4-Cyclopropansulfonylphenyl)-N-(5-methoxy-thiazol-2-yl)-2-(tetrahydropyran-4-yloxy)acetamid und dessen (+)- und (-)-Enantiomere;
{5-Chlor-2-[2-(4-cyclopropansulfonylphenyl)-2-tetrahydro-furan-3-yloxy)-acetylamino]-thiazol-4-yl}-Essigsäureethylester und dessen (+)- und (-)-Enantiomere;
4-{2-[2-(4-Cyclopropansulfonylphenyl)-2-(tetrahydro-furan-3-yloxy)-acetylamino]-thiazol-5-yloxy}-Benzoesäuremethylester und dessen (+)- und (-)-Enantiomere;
(5-Chlor-2-{2-(4-cyclopropansulfonylphenyl)-2-[(R)-(tetrahydro-furan-3-yl)oxy]-acetylamino}-thiazol-4-yl)-Essigsäureethylester und dessen (+)- und (-)-Enantiomere;
4-(2-{2-(4-Cyclopropansulfonylphenyl)-2-[(S)-(tetrahydro-furan-3-yl)oxy]-acetylamino}-thiazol-5-yloxy)-Benzoesäuremethylester und dessen (+)- und (-)-Enantiomere;
(5-Chlor-2-{2-(4-cyclopropansulfonylphenyl)-2-[(S)-(tetrahydro-furan-3-yl)oxy]-acetylamino}-thiazol-4-yl)-Essigsäureethylester und dessen (+)- und (-)-Enantiomere;
4-{3-[2-(4-Cyclopropansulfonylphenyl)-2-(tetrahydro-pyran-4-yloxy)-acetylamino]-pyrazol-1-ylmethyl}-Benzoesäuremethylester und dessen (+)- und (-)-Enantiomere;
{5-Chlor-2-[2-(4-cyclopropansulfonylphenyl)-2-(tetrahydro-pyran-4-yloxy)-acetylamino]-thiazol-4-yl}-Essigsäureethylester und dessen (+)- und (-)-Enantiomere;
3-{2-[2-(4-Cyclopropansulfonylphenyl)-2-(tetrahydro-pyran-4-yloxy)-acetylamino]-thiazol-5-yloxy}-Benzoesäuremethylester und dessen (+)- und (-)-Enantiomere;
4-{2-[2-(4-Cyclopropansulfonylphenyl)-2-(tetrahydro-pyran-4-yloxy)-acetylamino]-thiazol-5-yloxy}-2-fluor-Benzoesäuremethylester und dessen (+)- und (-)-Enantiomere;
4-{2-[2-(4-Cyclopropansulfonylphenyl)-2-(tetrahydro-pyran-4-yloxy)-acetylamino]-thiazol-5-yloxy}-3-fluor-Benzoesäuremethylester und dessen (+)- und (-)-Enantiomere;
{2-[2-(4-Cyclopropansulfonylphenyl)-2-(tetrahydro-pyran-4-yloxy)-acetylamino]-thiazolo[5,4-b]pyridin-5-yloxy}-Essigsäureethylester und dessen (+)- und (-)-Enantiomere;
4-{2-[2-(4-Cyclopropansulfonylphenyl)-2-(tetrahydro-pyran-4-yloxy)-acetylamino]-thiazolo[5,4-b]pyridin-5-yloxy}-Benzoesäuremethylester und dessen (+)- und (-)-Enantiomere;
2-(4-Cyclopropansulfonylphenyl)-N-(6-methoxy-benzothiazol-2-yl)-2-(tetrahydro-pyran-4-yloxy)-acetamid und dessen (+)- und (-)-Enantiomere;
2-(4-Cyclopropansulfonylphenyl)-N-(5-methoxy-thiazolo[5,4-b]pyridin-2-yl)-2-[(R)-(tetrahydro-furan-3-yl)oxy]-acetamid und dessen (+)- und (-)-Enantiomere;
4-[(4-Cyclopropansulfonylphenyl)-(5-fluor-thiazol-2-ylcarbamoyl)-methoxy]-piperidin-1-Carboxylsäure-tert-butylester und dessen (+)- und (-)-Enantiomere;
2-(4-Cyclopropansulfonylphenyl)-N-(5-fluor-thiazol-2-yl)-2-(piperidin-4-yloxy)-acetamid und dessen (+)- und (-)-Enantiomere;
(4-{2-[2-(4-Cyclopropansulfonylphenyl)-2-(tetrahydro-pyran-4-yloxy)-acetylamino]-thiazol-5-yloxy}-phenyl)-Essigsäuremethylester und dessen (+)- und (-)-Enantiomere;
2-[2-(4-Cyclopropansulfonylphenyl)-2-(tetrahydro-pyran-4-yloxy)-acetylamino]-benzothiazol-6-Carboxylsäuremethylester und dessen (+)- und (-)-Enantiomere;
2-{2-(4-Cyclopropansulfonylphenyl)-2-[(R)-(tetrahydro-furan-3-yl)oxy]-acetylamino}-benzothiazol-6-Carboxylsäuremethylester und dessen (+)- und (-)-Enantiomere;
2-(4-Cyclopropansulfonylphenyl)-N-(5-oxo-4,5-dihydro-thiazolo[5,4-b]pyridin-2-yl)-2-(tetrahydro-pyran-4-yloxy)acetamid und dessen (+)- und (-)-Enantiomere;
2-(4-Cyclopropansulfonylphenyl)-N-(5-isopropoxy-thiazolo[5,4-b]pyridin-2-yl)-2-[(R)-(tetrahydro-furan-3-yl)oxy]acetamid und dessen (+)- und (-)-Enantiomere;
4-{2-[2-(4-Cyclopropansulfonylphenyl)-2-(tetrahydro-pyran-4-yloxy)-acetylamino]-thiazol-5-yloxy}-2-methyl-Benzoesäuremethylester und dessen (+)- und (-)-Enantiomere;
2-[2-(4-Cyclopropansulfonylphenyl)-2-(tetrahydro-pyran-4-yloxy)-acetylamino]-5-methyl-thiazol-4-Carbonsäuremethylester und dessen (+)- und (-)-Enantiomere;
N-(5-Brom-thiazolo[5,4-b]pyridin-2-yl)-2-(4-cyclopropansulfonyl-phenyl)-2-(tetrahydropyran-4-yloxy)acetamid und dessen (+)- und (-)-Enantiomere;
2-(4-Cyclopropansulfonyl-phenyl)-N-[5-(4-formyl-phenoxy)-thiazol-2-yl]-2-(tetrahydropyran-4-yloxy)acetamid und dessen (+)- und (-)-Enantiomere;
2-(4-Cyclopropansulfonylphenyl)-2-(tetrahydro-pyran-4-yloxy)-N-(5-vinyl-thiazol-2-yl)-acetamid und dessen (+)- und (-)-Enantiomere;
2-(4-Cyclopropansulfonylphenyl)-2-(tetrahydro-pyran-4-yloxy)-N-(4-vinyl-thiazol-2-yl)-acetamid und dessen (+)- und (-)-Enantiomere;
4-{2-[2-(4-Cyclopropansulfonylphenyl)-2-(tetrahydro-pyran-4-yloxy)-acetylamino]-thiazol-5-yloxy}-Benzoesäure und dessen (+)- und (-)-Enantiomere;
4-(2-{2-(4-Cyclopropansulfonylphenyl)-2-[(R)-(tetrahydro-furan-3-yl)oxy]-acetylamino}-thiazol-5-yloxy)-Benzoesäure und dessen (+)- und (-)-Enantiomere;
{5-Chlor-2-[2-(4-cyclopropansulfonylphenyl)-2-(tetrahydro-furan-3-yloxy)-acetylamino}-thiazol-4-yl)-Essigsäure und dessen (+)- und (-)-Enantiomere;
4-{2-[2-(4-Cyclopropansulfonylphenyl)-2-(tetrahydro-furan-3-yloxy)-acetylamino]-thiazol-5-yloxy}-Benzoesäure und dessen (+)- und (-)-Enantiomere;
(5-Chlor-2-{2-(4-cyclopropansulfonylphenyl)-2-[(R)-(tetrahydro-furan-3-yl)oxy]-acetylamino}-thiazol-4-yl)-Essigsäure und dessen (+)- und (-)-Enantiomere;
4-(2-{2-(4-Cyclopropansulfonylphenyl)-2-[(S)-(tetrahydro-furan-3-yl)oxy]-acetylamino}-thiazol-5-yloxy)-Benzoesäure und dessen (+)- und (-)-Enantiomere;
(5-Chlor-2-{2-(4-cyclopropansulfonylphenyl)-2-[(S)-(tetrahydro-furan-3-yl)oxy]-acetylamino}-thiazol-4-yl)-Essigsäure und dessen (+)- und (-)-Enantiomere;
4-{3-[2-(4-Cyclopropansulfonylphenyl)-2-(tetrahydro-pyran-4-yloxy)-acetylamino]-pyrazol-1-ylmethyl}-Benzoesäure und dessen (+)- und (-)-Enantiomere;
{5-Chlor-2-[2-(4-cyclopropansulfonylphenyl)-2-(tetrahydro-pyran-4-yloxy)-acetylamino]-thiazol-4-yl}-Essigsäure und dessen (+)- und (-)-Enantiomere;
3-{2-[2-(4-Cyclopropansulfonylphenyl)-2-(tetrahydro-pyran-4-yloxy)-acetylamino]-thiazol-5-yloxy}-Benzoesäure und dessen (+)- und (-)-Enantiomere;
4-{2-[2-(4-Cyclopropansulfonylphenyl)-2-(tetrahydro-pyran-4-yloxy)-acetylamino]-thiazol-5-yloxy}-2-Fluorbenzoesäure und dessen (+)- und (-)-Enantiomere;
4-{2-[2-(4-Cyclopropansulfonylphenyl)-2-(tetrahydro-pyran-4-yloxy)-acetylamino]-thiazol-5-yloxy}-3-Fluorbenzoesäure und dessen (+)- und (-)-Enantiomere;
{2-[2-(4-Cyclopropansulfonylphenyl)-2-(tetrahydro-pyran-4-yloxy)-acetylamino]-thiazolo[5,4-b]pyridin-5-yloxy}-Essigsäure und dessen (+)- und (-)-Enantiomere;
4-{2-[2-(4-Cyclopropansulfonylphenyl)-2-(tetrahydro-pyran-4-yloxy)-acetylamino]-thiazolo[5,4-b]pyridin-5-yloxy}-Benzoesäure und dessen (+)- und (-)-Enantiomere;
4-[(4-Carboxymethyl-5-chlor-thiazol-2-ylcarbamoyl)-(4-cyclopropansulfonylphenyl)-methoxy]-piperidin-1-Carboxylsäure-tert-butylester und dessen (+)- und (-)-Enantiomere;
{5-Chlor-2-[2-(4-cyclopropansulfonylphenyl)-2-(piperidin-4-yloxy)-acetylamino]-thiazol-4-yl}-Essigsäure und dessen (+)- und (-)-Enantiomere;
(4-{2-[2-(4-Cyclopropansulfonylphenyl)-2-(tetrahydro-pyran-4-yloxy)-acetylamino]-thiazol-5-yloxy}-phenyl)-Essigsäure und dessen (+)- und (-)-Enantiomere;
2-[2-(4-Cyclopropansulfonylphenyl)-2-(tetrahydro-pyran-4-yloxy)-acetylamino]-benzothiazol-6-Carboxylsäure und dessen (+)- und (-)-Enantiomere;
2-{2-(4-Cyclopropansulfonylphenyl)-2-[(R)-(tetrahydro-furan-3-yl)oxy]-acetylamino}-benzothiazol-6-Carboxylsäure und dessen (+)- und (-)-Enantiomere;
4-{2-[2-(4-Cyclopropansulfonylphenyl)-2-(tetrahydro-pyran-4-yloxy)-acetylamino]-thiazol-5-yloxy}-2-methyl-Benzoesäure und dessen (+)- und (-)-Enantiomere;
2-[2-(4-Cyclopropansulfonylphenyl)-2-(tetrahydro-pyran-4-yloxy)-acetylamino]-5-methyl-thiazol-4-Carboxylsäure und dessen (+)- und (-)-Enantiomere;
N-{5-[4-(Azetidin-1-carbonyl)phenoxy]-thiazol-2-yl}-2-(4-cyclopropansulfonylphenyl)-2-(tetrahydro-pyran-4-yloxy)acetamid und dessen (+)- und (-)-Enantiomere;
4- {2-[2-(4-Cyclopropansulfonylphenyl)-2-(tetrahydro-furan-3-yloxy)-acetylamino]-thiazol-5-yloxy}benzamid und dessen (+)- und (-)-Enantiomere;
N-[4-(2-Azetidin-1-yl-2-oxo-ethyl)-5-chlor-thiazol-2-yl]-2-(4-cyclopropansulfonyl-phenyl)-2-(tetrahydro-pyran-4-yloxy)acetamid und dessen (+)- und (-)-Enantiomere;
2-(4-Cyclopropansulfonylphenyl)-2-(tetrahydro-pyran-4-yloxy)-N-{5-[4-(2H-tetrazol-5-yl)-phenoxy]-thiazol-2-yl}acetamid und dessen (+)- und (-)-Enantiomere;
2-(4-Cyclopropansulfonylphenyl)-N-[5-(4-hydroxymethyl-phenoxy)-thiazol-2-yl]-2-(tetrahydro-pyran-4-yloxy)acetamid und dessen (+)- und (-)-Enantiomere;
2-(4-Cyclopropansulfonylphenyl)-N-(4-hydroxymethyl-5-methyl-thiazol-2-yl)-2-(tetrahydro-pyran-4-yloxy)acetamid und dessen (+)- und (-)-Enantiomere;
2-(4-Cyclopropansulfonylphenyl)-N-[5-(1,2-dihydroxy-ethyl)-thiazol-2-yl]-2-(tetrahydro-pyran-4-yloxy)acetamid und dessen (+)- und (-)-Enantiomere;
2-(4-Cyclopropansulfonylphenyl)-N-[5-(1,2-dihydroxy-ethyl)-thiazolo[5,4-b]pyridin-2-yl]-2-(tetrahydro-pyran-4-yloxy)-acetamid und dessen (+)- und (-)-Enantiomere;
2-(4-Cyclopropansulfonylphenyl)-N-[4-(1,2-dihydroxy-ethyl)-thiazol-2-yl]-2-(tetrahydro-pyran-4-yloxy)acetamid und dessen (+)- und (-)-Enantiomere;
2-(4-Cyclopropansulfonylphenyl)-N-[3-(1,2-dihydroxy-ethyl)-[1,2,4]thiadiazol-5-yl]-2-(tetrahydro-pyran-4-yloxy)acetamid und dessen (+)- und (-)-Enantiomere;
{5-Chlor-2-[2-(4-cyclopropansulfonylphenyl)-2-(2-methyl-tetrahydro-pyran-3-yloxy)-acetylamino]-thiazol-4-yl}-Essigsäure und dessen (+)- und (-)-Enantiomere;
2-[2-[4-(3-Oxo-cyclopentansulfonyl)-phenyl]-2-(tetrahydro-furan-3-yloxy)-acetyl-amino]-thiazol-4-Carboxylsäure und dessen (+)- und (-)-Enantiomere;
N-[4-(1-Acetyl-piperidin-4-yl)-thiazol-2-yl]-2-(4-cyclopropansulfonylphenyl)-2-(tetrahydro-pyran-4-yloxy)acetamid und dessen (+)- und (-)-Enantiomere;
2-(4-Cyclopropansulfonylphenyl)-N-{5-[4-(1,2-dihydroxy-ethyl)-phenoxy]-thiazol-2-yl}-2-(tetrahydro-pyran-4-yloxy)acetamid und dessen (+)- und (-)-Enantiomere;
2-(4-Cyclopropansulfonylphenyl)-N-(4,5,6,7-tetrahydro-benzothiazol-2-yl)-2-(tetrahydro-pyran-4-yloxy)-acetamid und dessen (+)- und (-)-Enantiomere;
2-(4-Cyclopropansulfonyl-phenyl)-2-(tetrahydro-pyran-4-yloxy)-N-thiazolo[5,4-d]pyrimidin-2-yl-acetamid und dessen (+)- und (-)-Enantiomere;
2-(4-Cyclopropansulfonylphenyl)-N-[5-(4-hydroxymethyl-phenoxy)-thiazol-2-yl]-2-(tetrahydro-pyran-4-yloxy)acetamid und dessen (+)- und (-)-Enantiomere;
2-(4-Cyclopropansulfonylphenyl)-N-[4-(1,2-dihydroxy-ethyl)-thiazol-2-yl]-2-(tetrahydro-pyran-4-yloxy)acetamid und dessen (+)- und (-)-Enantiomere;
2-(4-Cyclopropansulfonylphenyl)-N-[4-(1,2-dihydroxy-ethyl)-5-fluor-thiazol-2-yl]-2-(tetrahydro-pyran-4-yloxy)-acetamid und dessen (+)- and (-)-Enantiomere;
2-(4-Cyclopropansulfonylphenyl)-N-[5-(1,2-dihydroxy-ethyl)-thiazol-2-yl]-2-(tetrahydro-pyran-4-yloxy)-acetamid und dessen (+)- und (-)-Enantiomere;
2-(4-Cyclopropansulfonylphenyl)-N-[5-(1,2-dihydroxy-ethyl)-thiazolo[5,4-b]pyridin-2-yl]-2-(tetrahydro-pyran-4-yloxy)acetamid und dessen (+)- und (-)-Enantiomere;
2-(3-{2-(4-Cyclopropansulfonylphenyl)-2-[(R)-(tetrahydro-furan-3-yl)oxy]-acetyl-amino}-pyrazol-1-yl)-2-methyl-Propansäure und dessen (+)- und (-)-Enantiomere ist.

6. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der Behandlung einer durch Glukokinase-Aktivierung verursachten Erkrankung.

7. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der Behandlung einer durch Glukokinase-Deinhibition verursachten Erkrankung.

8. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der vorbeugenden oder therapeutischen Behandlung von Hyperglykämie oder Diabetes, insbesondere Diabetes Typ II.

9. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der Vorbeugung von Diabetes, insbesondere Diabetes Typ II, bei einem Menschen, welcher prädiabetische Hyperglykämie oder verminderte Glukosetoleranz aufweist.

10. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der kombinierten Behandlung oder Vorbeugung von Diabetes und Adipositas.

11. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der Behandlung oder Vorbeugung von Adipositas.

12. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der Behandlung oder Vorbeugung von Dyslipidämie.

13. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der Behandlung von Hyperglykämie, IGT, Syndrom X, Diabetes Typ 2, Diabetes Typ 1, Dyslipidämie oder Hyperlipidämie, Bluthochdruck, zur Verwendung bei der Behandlung oder Vorbeugung von Adipositas, zur Verwendung bei der Verminderung von Nahrungsaufnahme, zur Verwendung bei der Appetitregulierung; und zur Verwendung bei der Regulierung von Essverhalten.

14. Eine Verbindung der Formel (I) wie in einem der Ansprüche 1 bis 5 beansprucht oder deren Stereoisomere, Tautomere, pharmazeutisch verträglichen Salze, Polymorphe, Solvate und Formulierungen davon, zur Verwendung bei der Steigerung der Entero-Inkretinausschüttung, wie GLP-1 und GIP, wobei dadurch die Erkrankungen oder Störungen in Zusammenhang mit der Steuerung der Ausschüttung von Entero-Inkretinen, wie Hyperglykämie, Insulinresistenz, verminderter Glukosetoleranz, Adipositas, Magenentleerung, Gastroparese, Übersättigung, Leptinresistenz, Dyslipidämie, Wundheilung, diabetischen Komplikationen, wie Nephropathie, Retinopathie, Neuropathie und Katarakten gesteuert werden.

15. Ein Arzneimittel, umfassend, als Wirkstoff, mindestens eine Verbindung der Formel (I) wie in einem der Ansprüche 1 bis 5 beansprucht oder deren Stereoisomere, Tautomere, pharmazeutisch verträglichen Salze, Polymorphe und Solvate davon, zusammen mit einem oder mehreren pharmazeutisch verträglichen Trägern oder Exzipienten.

16. Ein Arzneimittel, umfassend, als Wirkstoff, mindestens eine Verbindung der Formel (I) wie in einem der Ansprüche 1 bis 5 beansprucht oder deren Stereoisomere, Tautomere, pharmazeutisch verträglichen Salze, Polymorphe und Solvate davon, in Kombination mit einem oder mehreren pharmazeutisch verträglichen therapeutischen Wirkstoffen.

17. Das Arzneimittel wie in Anspruch 16 beansprucht, wobei der pharmazeutisch verträgliche therapeutische Wirkstoff ausgewählt ist aus Mitteln gegen Diabetes, Mitteln gegen Hyperglykämie, Mitteln gegen Adipositas, Mitteln gegen Bluthochdruck oder Mitteln gegen Dyslipidämie.

18. Das Arzneimittel wie in Anspruch 16 oder 17 beansprucht, wobei der pharmazeutisch verträgliche therapeutische Wirkstoff ausgewählt ist aus Insulinsekretions-anregenden Mitteln, wie Sulfonylharnstoffen ausgewählt aus Amaryl, Glyburid, Glimepirid, Glipyrid, Glipizid; insulinotropen Sulfonylharnstoff-Rezeptorliganden, wie Meglitiniden ausgewählt aus Nateglinid, Rapaglinid; Biguaniden wie Metformin, Phenformin, Buformin; Glukagon-Antagonisten wie einem Peptid- oder Nichtpeptid-Glukagonantagonisten; Glukosidaseinhibitoren wie Acarbose, Miglitol; Glukosesensitiven insulinotropen Mitteln wie GLP-1, GLP-1-Mimetika wie Exendin-4; Insulinsensibilisatoren wie Troglitazon, Rosiglitazon, Pioglitazon; Dipeptidyl-Peptidase-IV-Inhibitoren wie Sitagliptin, Vildagliptin; Sibutramin, Orlistat, Rimonabant; Fibraten wie Gemfibrozil, Fenofibrat; Niacin; Statinen wie Rosuvastatin, Atorvastatin, Simvastatin; Cholesterin-Absorptionsinhibitoren wie Ezetimib; Gallensäurebindern wie Cholestyramin; Diuretika wie Hydrochlorthiaziden, Mannitol, Indapamid, Furosemid; Angiotensin-umwandelnden Enzymen (ACE)-Inhibitoren wie Captopril, Enalapril; Angiotensin-II-Rezeptor-Typ-I-Blockern (ARB) wie Losartan, Irbesartan; Rennin-Inhibitoren wie Aliskerin; β-adrenergen Rezeptorblockern wie Atenolol, Metoprolol; Calciumkanalblockern wie Amlodipin, Nifedipin; Aldosteron-Rezeptorantagonisten wie Spironolacton, Aldosteron-Synthaseinhibitoren wie FAD286.

19. Verwendung einer Verbindung der Formel (I) wie in einem der Ansprüche 1 bis 5 beansprucht, deren Polymorphe, Stereoisomere, pharmazeutisch verträglichen Salze oder Solvate davon, zur Herstellung eines Medikaments zur Glukokinase-Aktivierung.

20. Verwendung einer Verbindung der Formel (I) wie in einem der Ansprüche 1 bis 5 beansprucht, deren Polymorphe, Stereoisomere, pharmazeutisch verträglichen Salze oder Solvate davon, zur Herstellung eines Medikaments zur vorbeugenden oder therapeutischen Behandlung von Adipositas.

21. Verwendung einer Verbindung der Formel (I) wie in einem der Ansprüche 1 bis 5 beansprucht, deren Polymorphe, Stereoisomere, pharmazeutisch verträglichen Salze oder Solvate davon, zur Herstellung eines Medikaments zur prophylaktischen oder therapeutischen Behandlung von Hyperglykämie oder Diabetes, insbesondere Diabetes Typ II.

22. Verwendung einer Verbindung der Formel (I) wie in einem der Ansprüche 1 bis 5 beansprucht, deren Polymorphe, Stereoisomere, pharmazeutisch verträglichen Salze oder Solvate davon, zur Herstellung eines Medikaments zur Vorbeugung von Diabetes, insbesondere Diabetes Typ II, bei einem Menschen, welcher prädiabetische Hyperglykämie oder verminderte Glukosetoleranz aufweist.

23. Ein Verfahren zur Herstellung einer Verbindung der Formel (I) wie in einem der Ansprüche 1 bis 5 beansprucht oder deren Stereoisomere, Tautomere, pharmazeutisch verträglichen Salze, Polymorphe und Solvate davon, wobei das Verfahren umfasst:
Umsetzen einer Säure der Formel (II) 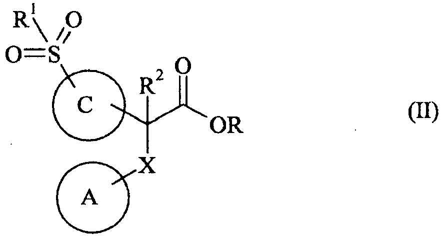
wobei R Wasserstoff, Alkyl oder Arylalkyl darstellt, mit einer Verbindung der Formel (III) 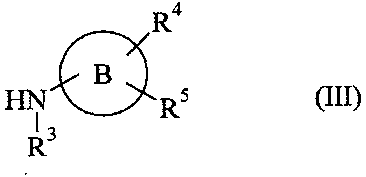
in Gegenwart eines geeigneten Amidkopplungs-Reagens, gegebenenfalls Hydrolysieren und gegebenenfalls weiteres Koppeln mit einem Amin der Formel NHR⁶R⁷, um die Verbindung der Formel (I) zu erhalten.

## Revendications

1. Composé de formule (I) 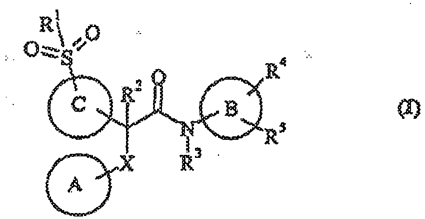 ou ses stéréoisomères, tautomères, sels pharmaceutiquement acceptables, polymorphes, solvates et formulations dans laquelle
R¹ représente un groupe cycloalkyle ; R² et R³ représentent un atome d'hydrogène ;
R⁴ et R⁵ sont choisis indépendamment dans un groupe constitué d'atomes d'hydrogène, d'halogène, de groupes alkyle, cycloalkyle, hétérocyclyle, aryle, hétéroaryle, halogénoalkyle mono-, di- ou tri-substitué, nitrile, -NR⁶C(O)OR⁷, -(CR⁸R⁹)ₙC(O)OR⁶, -(CR⁸R⁹)ₙC(O)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -C(R⁸R⁹)ₙNR⁶R⁷, -C(R⁸R⁹)ₙCO(R⁶) et -S(O)ₚC(R⁸R⁹)ₙC(O)OR⁶ ; dans lequel chacun de R⁴ et R⁵ est éventuellement substitué par un ou plusieurs substituants choisis parmi des groupes halogéno, alkyle, alcényle, alcynyle, cycloalkyle, aryle, hétéroaryle, hétérocyclyle, alkylsulfonyle, oxo, nitro, cyano, -(CR⁸R⁹)ₙR⁶, -(CR⁸R⁹)ₙCOOR⁶, -(CR⁸R⁹)ₙC(O)NR⁶R⁷, -(CR⁸R⁹)ₙOR⁶, -SR⁶ ou -NR⁶R⁷ ; dans lesquels n = 0 à 4 ;
X représente O ;
le cycle A représente un cycle hétérocyclyle monocyclique ;
le cycle B représente un cycle hétéroaryle et le cycle C représente un cycle phényle ;
les cycles A, B et C sont éventuellement substitués par 1 à 4 substituants choisis indépendamment parmi des atomes d'halogène, des groupes monohalogénoalkyle, dihalogénoalkyle ou perhalogénoalkyle, monohalogénoalcoxy, dihalogénoalcoxy ou perhalogénoalcoxy, cyano, nitro, alkyle, alcényle, alcynyle, méthylènedioxy, amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, cycloalkyle, cycloalkylalkyle, hétérocyclyle, hétérocycloalkyle, aryle, arylalkyle, hétéroaryle, hétéroarylalkyle, cycloalcényle, aryloxy, hétéroaryloxy ;
p = 0 à 2 ; n = 0 à 4 ;
R⁶ et R⁷ sont choisis indépendamment dans un groupe constitué d'un atome d'hydrogène, de groupes alkyle, alcényle, alcynyle, aryle, arylalkyle, hétéroaryle, hétéroarylalkyle, cycloalkyle, cycloalkylalkyle, hétérocyclyle et hétérocyclylalkyle dans lequel chacun de R⁶ et R⁷ est éventuellement substitué par un ou plusieurs substituants choisis parmi des groupes halogéno, alkyle, cycloalkyle, aryle, hétéroaryle, hétérocyclyle, alkylsulfonyle, cyano, -(CR⁸R⁹)ₙOR⁶, -(CR⁸R⁹)ₙCOOR⁶ ou -(CR⁸R⁹)ₙC(O)NR⁶R⁷ ; dans lesquels n = 0 à 4 ;
ou
R⁶ et R⁷ pris ensemble forment un système de cycle monocyclique ou bicyclique qui peut être saturé ou partiellement insaturé et comporter éventuellement des hétéroatomes supplémentaires choisis parmi O, N ou S, ledit système de cycle peut être en outre éventuellement substitué par 1 à 4 substituants choisis indépendamment parmi des groupes halogéno, alkyle, alcényle, alcynyle, nitro, cyano, -OR⁶, -SR⁶, -NR⁶R⁷, oxo, alkylsulfonyle, -COOR⁶, -C(O)NR⁶R⁷, cycloalkyle, cycloalkylalkyle, aryle, arylalkyle, hétérocyclyle, hétérocyclylalkyle, hétéroaryle, hétéroarylalkyle ; dans lequel R⁶ et R⁷ sont tels que décrits ci-dessus ;
R⁸ et R⁹ sont choisis indépendamment dans un groupe constitué d'atomes d'hydrogène, de fluor, de groupes OR⁶, alkyle, et perfluoroalkyle, ou
R⁸ et R⁹ pris ensemble forment un système de cycle monocyclique ou bicyclique qui est saturé ou partiellement insaturé et comporte éventuellement des hétéroatomes supplémentaires choisis parmi O, N ou S, ledit système de cycle peut être en outre éventuellement substitué par 1 à 4 substituants choisis indépendamment parmi des groupes halogéno, alkyle, alcényle, alcynyle, nitro, cyano, oxo, -OR⁶, -SR⁶, -NR⁶R⁷, alkylsulfonyle, -COOR⁶, -C(O)NR⁶R⁷, cycloalkyle, cycloalkylalkyle, aryle, arylalkyle, hétérocyclyle, hétérocyclylalkyle, hétéroaryle, hétéroarylalkyle.

2. Composé tel que revendiqué dans la revendication 1, ou ses stéréoisomères, tautomères, sels pharmaceutiquement acceptables, polymorphes, solvates et formulations dans lequel le cycle A est choisi parmi 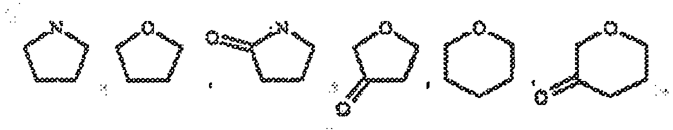 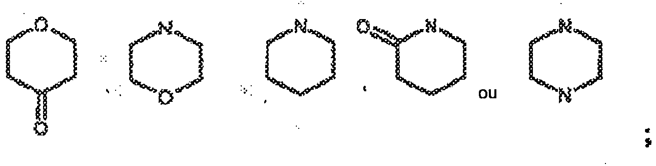 le cycle B est choisi parmi 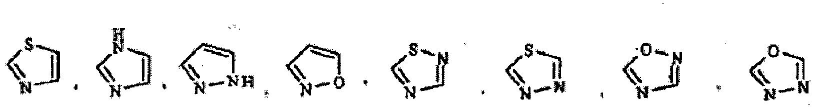 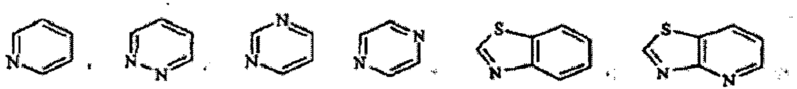 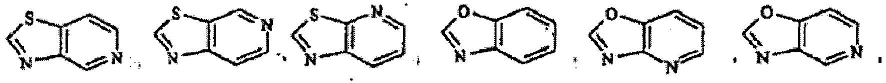 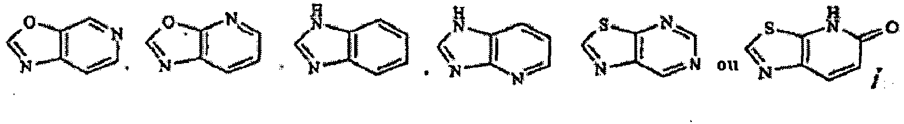
les cycles A, B et C sont éventuellement substitués par 1 à 4 substituants choisis indépendamment parmi des atomes d'halogène, des groupes monohalogénoalkyle, dihalogénoalkyle ou perhalogénoalkyle, monohalogénoalcoxy, dihalogénoalcoxy ou perhalogénoalcoxy, cyano, nitro, alkyle, alcényle, alcynyle, méthylènedioxy, amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, cycloalkyle, cycloalkylalkyle, hétérocyclyle, hétérocycloalkyle, aryle, arylalkyle, hétéroaryle, hétéroarylalkyle, cycloalcényle, aryloxy, hétéroaryloxy ;
p = 0 à 2 ; n = 0 à 4 ;
R¹ est choisi parmi des groupes cycloalkyle en C₃ à C₆ ;
R⁴ et R⁵ sont choisis indépendamment dans un groupe constitué d'atomes d'hydrogène, d'halogène, de groupes alkyle, cycloalkyle, hétérocyclyle, aryle, hétéroaryle, halogénoalkyle mono-, di- ou tri-substitué, nitrile, nitro, -NR⁶R⁷, -OR⁶, -NR⁶C(O)R⁷, -NR⁶C(O)OR⁷, -(CR⁸R⁹)ₙC(O)OR⁶, -(CR⁸R⁹)ₙC(O)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -C(R⁸R⁹)ₙNR⁶R⁷, -C(R⁸R⁹)ₙCO(R⁶) et -S(O)ₚC(R⁸R⁹)ₙC(O)OR⁶ ; dans lequel chacun de R⁴ et R⁵ est éventuellement substitué par un ou plusieurs substituants choisis parmi des groupes halogéno, alkyle à chaîne linéaire ou à chaîne ramifiée, alcényle, alcynyle, cycloalkyle, aryle, hétéroaryle, hétérocyclyle, alkylsulfonyle, oxo, nitro, cyano, -C(R⁸R⁹)ₙCOOR⁶, -C(O)NR⁶R⁷, -OR⁶, -SR⁶ ou -NR⁶R⁷ ;
dans lesquels n = 0 à 4 ;
R⁶ et R⁷ sont choisis indépendamment dans un groupe constitué d'un atome d'hydrogène, de groupes alkyle, aryle, arylalkyle, hétéroaryle, hétéroarylalkyle, cycloalkyle, cycloalkylalkyle, hétérocyclyle et hétérocyclylalkyle ; dans lequel chacun de R⁶ et R⁷ est éventuellement substitué par un ou plusieurs substituants choisis parmi des groupes halogéno, alkyle, cycloalkyle, aryle, hétéroaryle, hétérocyclyle, alkylsulfonyle, cyano, -(CR⁸R⁹)ₙOR⁶, -(CR⁸R⁹)ₙCOOR⁶ ou -(CR⁸R⁹)ₙC(O)NR⁶R⁷ ;
dans lesquels n = 0 à 4 ; ou
R⁶ et R⁷ pris ensemble forment un système de cycle monocyclique ou bicyclique qui peut être saturé ou partiellement insaturé et comporter éventuellement des hétéroatomes supplémentaires choisis parmi O, N ou S, ledit système de cycle peut être en outre éventuellement substitué par 1 à 4 substituants choisis indépendamment parmi des groupes halogéno, alkyle, alcényle, alcynyle, nitro, cyano, -OR⁶, -SR⁶, -NR⁶R⁷, oxo, alkylsulfonyle, -COOR⁶, -C(O)NR⁶R⁷, cycloalkyle, cycloalkylalkyle, aryle, arylalkyle, hétérocyclyle, hétérocyclylalkyle, hétéroaryle, hétéroarylalkyle ;
dans lequel R⁶ et R⁷ sont tels que décrits ci-dessus ;
R⁸ et R⁹ sont choisis indépendamment dans un groupe constitué d'atomes d'hydrogène, de fluor, de groupes alkyle, et perfluoroalkyle.

3. Composé tel que revendiqué dans la revendication 1, ou ses stéréoisomères, tautomères, sels pharmaceutiquement acceptables, polymorphes, solvates et formulations, dans lequel le cycle B est choisi parmi 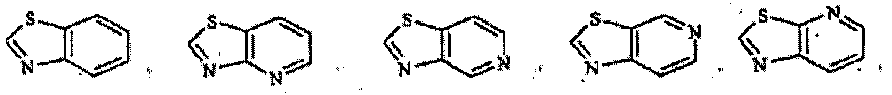 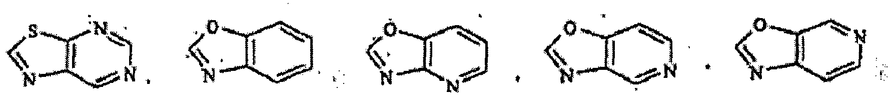 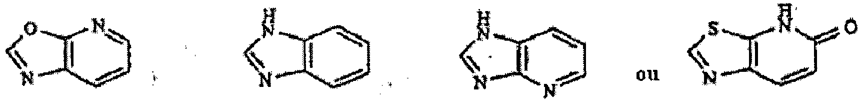 le cycle A est choisi parmi 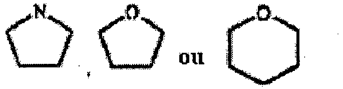
les cycles A, B et C sont éventuellement substitués par 1 à 4 substituants choisis indépendamment parmi des atomes d'halogène, des groupes monohalogénoalkyle, dihalogénoalkyle ou perhalogénoalkyle, monohalogénoalcoxy, dihalogénoalcoxy ou perhalogénoalcoxy, cyano, nitro, alkyle, alcényle, alcynyle, méthylènedioxy, amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, cycloalkyle, cycloalkylalkyle, hétérocyclyle, hétérocycloalkyle, aryle, arylalkyle, hétéroaryle, hétéroarylalkyle, cycloalcényle, aryloxy, hétéroaryloxy ;
p = 0 à 2 ; n = 0 à 4 ;
R¹ est choisi parmi des groupes cycloalkyle en C₃ à C₆ ;
R², R³ et R⁵ représentent un atome d'hydrogène ;
R⁴ est choisi dans un groupe constitué d'atomes d'hydrogène, d'halogène, de groupes alkyle, cycloalkyle, hétérocyclyle, aryle, hétéroaryle, halogénoalkyle mono-, di- ou tri-substitué, nitrile, nitro, -NR⁶R⁷, -OR⁶, -NR⁶C(O)R⁷, -NR⁶C(O)OR⁷, -(CR⁸R⁹)ₙC(O)OR⁶, -(CR⁸R⁹)ₙC(O)NR⁶R⁷, -(CR⁸R⁹)ₙS(O)ₚNR⁶R⁷, -(CR⁸R⁹)ₙN(R⁶)C(O)R⁷, -(CR⁸R⁹)ₙOR⁶, -C(R⁸R⁹)ₙNR⁶R⁷, -C(R⁸R⁹)ₙCO(R⁶) et -S(O)ₚC(R⁸R⁹)ₙC(O)OR⁶ ; dans lequel R⁴ est éventuellement substitué par un ou plusieurs substituants choisis parmi des groupes halogéno, alkyle à chaîne linéaire ou à chaîne ramifiée, alcényle, alcynyle, cycloalkyle, aryle, hétéroaryle, hétérocyclyle, alkylsulfonyle, oxo, nitro, cyano, -C(R⁸R⁹)ₙCOOR⁶, -C(O)NR⁶R⁷, -OR⁶, -SR⁶ ou -NR⁶R⁷ ;
dans lesquels n = 0 à 4 ;
R⁶ et R⁷ sont choisis indépendamment dans un groupe constitué d'un atome d'hydrogène, de groupes alkyle, aryle, hétéroaryle, cycloalkyle, hétérocyclyle ; dans lequel chacun de R⁶ et R⁷ est éventuellement substitué par un ou plusieurs substituants choisis parmi des groupes halogéno, alkyle, cycloalkyle, aryle, hétéroaryle, hétérocyclyle, alkylsulfonyle, cyano, (CR⁸R⁹)ₙOR⁶, -(CR⁸R⁹)ₙCOOR⁶ ou -(CR⁸R⁹)ₙC(O)NR⁶R⁷ ;
dans lesquels n = 0 à 4 ; ou
R⁶ et R⁷ pris ensemble forment un système de cycle monocyclique ou bicyclique qui peut être saturé ou partiellement insaturé et comporter éventuellement des hétéroatomes supplémentaires choisis parmi O, N ou S, ledit système de cycle peut être en outre éventuellement substitué par 1 à 4 substituants choisis indépendamment parmi des groupes halogéno, alkyle, alcényle, alcynyle, nitro, cyano, -OR⁶, -SR⁶, -NR⁶R⁷, oxo, alkylsulfonyle, -COOR⁶, -C(O)NR⁶R⁷, cycloalkyle, cycloalkylalkyle, aryle, arylalkyle, hétérocyclyle, hétérocyclylalkyle, hétéroaryle, hétéroarylalkyle ;
dans lequel R⁶ et R⁷ sont tels que décrits ci-dessus ;
R⁸ et R⁹ sont choisis indépendamment dans un groupe constitué d'atomes d'hydrogène, de fluor, de groupes alkyle, et perfluoroalkyle.

4. Composé tel que revendiqué dans la revendication 1, ou ses stéréoisomères, tautomères, sels pharmaceutiquement acceptables, polymorphes, solvates et formulations, dans lequel le cycle B est choisi parmi 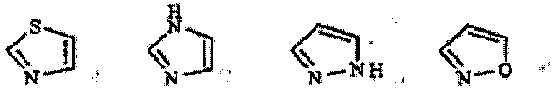 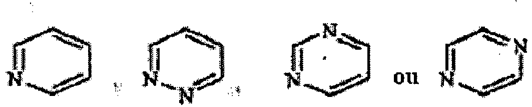
les cycles A, B et C sont éventuellement substitués par 1 à 4 substituants choisis indépendamment parmi des atomes d'halogène, des groupes monohalogénoalkyle, dihalogénoalkyle ou perhalogénoalkyle, monohalogénoalcoxy, dihalogénoalcoxy ou perhalogénoalcoxy, cyano, nitro, alkyle, alcényle, alcynyle, méthylènedioxy, amidino, -NR⁶R⁷, -OR⁶, -S(O)ₚR⁶, -S(O)ₚNR⁶R⁷, -NR⁶S(O)ₚR⁷, -NR⁶C(O)R⁷, -OS(O)ₚR⁷, -NR⁶C(O)OR⁷, -NR⁶C(O)NR⁶R⁷, -(CR⁸R⁹)ₙC(O)OR⁶, -(CR⁸R⁹)ₙC(O)NR⁶R⁷, -(CR⁸R⁹)ₙC(O)R⁶, cycloalkyle, cycloalkylalkyle, hétérocyclyle, hétérocycloalkyle, aryle, arylalkyle, hétéroaryle, hétéroarylalkyle, cycloalcényle, aryloxy, hétéroaryloxy ;
p = 0 à 2 ; n = 0 à 4 ;
R¹ est choisi parmi des groupes cycloalkyle en C₃ à C₆ ;
R⁴ représente -OR^{6'}, dans lequel R^{6'} représente un groupe phényle ou pyridyle qui est éventuellement substitué par un ou plusieurs substituants choisis parmi des groupes halogéno, alkyle, cycloalkyle, alkylsulfonyle, cyano, -(CR⁸R⁹)ₙOR⁶, -(CR⁸R⁹)ₙCOOR⁶, -(CR⁸R⁹)ₙC(O)NR⁶R⁷ ;
dans lesquels n = 0 à 4 ;
R⁶ et R⁷ sont choisis indépendamment dans un groupe constitué d'un atome d'hydrogène, de groupes alkyle, aryle, arylalkyle, hétéroaryle, hétéroarylalkyle, cycloalkyle, cycloalkylalkyle, hétérocyclyle et hétérocyclylalkyle ; dans lequel chacun de R⁶ et R⁷ est éventuellement substitué par un ou plusieurs substituants choisis parmi des groupes halogéno, alkyle, cycloalkyle, aryle, hétéroaryle, hétérocyclyle, alkylsulfonyle, cyano, -(CR⁸R⁹)ₙOR⁶, -(CR⁸R⁹)ₙCOOR⁶ ou -(CR⁸R⁹)ₙC(O)NR⁶R⁷ ;
dans lesquels n = 0 à 4 ;
ou
R⁶ et R⁷ pris ensemble forment un système de cycle monocyclique ou bicyclique qui peut être saturé ou partiellement insaturé et comporter éventuellement des hétéroatomes supplémentaires choisis parmi O, N ou S, ledit système de cycle peut être en outre éventuellement substitué par 1 à 4 substituants choisis indépendamment parmi des groupes halogéno, alkyle, alcényle, alcynyle, nitro, cyano, -OR⁶, -SR⁶, -NR⁶R⁷, oxo, alkylsulfonyle, -COOR⁶, -C(O)NR⁶R⁷, cycloalkyle, cycloalkylalkyle, aryle, arylalkyle, hétérocyclyle, hétérocyclylalkyle, hétéroaryle, hétéroarylalkyle ;
dans lequel R⁶ et R⁷ sont tels que décrits ci-dessus ;
R⁸ et R⁹ sont choisis indépendamment dans un groupe constitué d'atomes d'hydrogène, de fluor, de groupes alkyle, et perfluoroalkyle.

5. Composé tel que revendiqué dans la revendication 1 qui est
l'ester méthylique de l'acide 4-{2-[2-(4-cyclopropanesulfonyl-phényl)-2-(tétrahydro-pyran-4-yloxy)-acétylamino]-thiazol-5-yloxy}-benzoïque et ses énantiomères (+) et (-) ;
l'ester méthylique de l'acide 4-(2-{2-(4-cyclopropanesulfonyl-phényl)-2-[(R)-(tétrahydro-furan-3-yl)oxy]-acétylamino}-thiazol-5-yloxy)-benzoïque et ses énantiomères (+) et (-) ;
le 2-(4-cyclopropanesulfonyl-phényl)-2-(tétrahydro-pyran-4-yloxy)-N-thiazolo-[5,4-b]pyridin-2-yl-acétamide et ses énantiomères (+) et (-) ;
le 2-(4-cyclopropanesulfonyl-phényl)-N-(5-méthoxy-thiazolo[5,4-b]pyridin-2-yl)-2-(tétrahydro-pyran-4-yloxy)-acétamide et ses énantiomères (+) et (-) ;
le 2-(4-cyclopropanesulfonyl-phényl)-N-(5-éthoxy-thiazolo[5,4-b]pyridin-2-yl)-2-(tétrahydro-pyran-4-yloxy)-acétamide et ses énantiomères (+) et (-) ;
le 2-(4-cyclopropanesulfonyl-phényl)-N-(5-isopropoxy-thiazolo[5,4-b]pyridin-2-yl)-2-(tétrahydro-pyran-4-yloxy)-acétamide et ses énantiomères (+) et (-) ;
le 2-(4-cyclopropanesulfonyl-phényl)-N-(5-pyrazol-1-yl-thiazol-2-yl)-2-(tétrahydro-pyran-4-yloxy)-acétamide et ses énantiomères (+) et (-) ;
le 2-(4-cyclopropanesulfonyl-phényl)-N-(5-morpholin-4-yl-thiazol-2-yl)-2-(tétrahydro-pyran-4-yloxy)-acétamide et ses énantiomères (+) et (-) ;
le 2-(4-cyclopropanesulfonyl-phényl)-N-(5-fluoro-thiazol-2-yl)-2-(tétrahydro-pyran-4-yloxy)-acétamide et ses énantiomères (+) et (-) ;
le 2-(4-cyclopropanesulfonyl-phényl)-N-(1-méthyl-1H-pyrazol-3-yl)-2-(tétrahydro-pyran-4-yloxy)-acétamide et ses énantiomères (+) et (-) ;
le 2-(4-cyclopropanesulfonyl-phényl)-N-pyrazin-2-yl-2-(tétrahydro-pyran-4-yloxy)-acétamide et ses énantiomères (+) et (-) ;
le N-[5-(4-cyano-phénoxy)-thiazol-2-yl]-2-(4-cyclopropanesulfonyl-phényl)-2-(tétrahydro-pyran-4-yloxy)-acétamide et ses énantiomères (+) et (-) ;
le 2-(4-cyclopropanesulfonyl-phényl)-N-(5-méthoxy-thiazol-2-yl)-2-(tétrahydro-pyran-4-yloxy)-acétamide et ses énantiomères (+) et (-) ;
l'ester éthylique de l'acide {5-chloro-2-[2-(4-cyclopropanesulfonyl-phényl)-2-tétrahydro-furan-3-yloxy)-acétylamino]-thiazol-4-yl}-acétique et ses énantiomères (+) et (-) ;
l'ester méthylique de l'acide 4-{2-[2-(4-cyclopropanesulfonyl-phényl)-2-(tétrahydro-furan-3-yloxy)-acétylamino]-thiazol-5-yloxy}-benzoïque et ses énantiomères (+) et (-) ;
l'ester éthylique de l'acide (5-chloro-2-{2-(4-cyclopropanesulfonyl-phényl)-2-[(R)-(tétrahydro-furan-3-yl)oxy]-acétylamino}-thiazol-4-yl)-acétique et ses énantiomères (+) et (-) ;
l'ester méthylique de l'acide 4-(2-{2-(4-cyclopropanesulfonyl-phényl)-2-[(S)-(tétrahydro-furan-3-yl)oxy]-acétylamino}-thiazol-5-yloxy)-benzoïque et ses énantiomères (+) et (-) ;
l'ester éthylique de l'acide (5-chloro-2-{2-(4-cyclopropanesulfonyl-phényl)-2-[(S)-(tétrahydro-furan-3-yl)oxy]-acétylamino}-thiazol-4-yl)-acétique et ses énantiomères (+) et (-) ;
l'ester méthylique de l'acide 4-{3-[2-(4-cyclopropanesulfonyl-phényl)-2-(tétrahydro-pyran-4-yloxy)-acétylamino]-pyrazol-1-ylméthyl}-benzoïque et ses énantiomères (+) et (-) ;
l'ester éthylique de l'acide {5-chloro-2-[2-(4-cyclopropanesulfonyl-phényl)-2-(tétrahydro-pyran-4-yloxy)-acétylamino]-thiazol-4-yl}-acétique et ses énantiomères (+) et (-) ;
l'ester méthylique de l'acide 3-{2-[2-(4-cyclopropanesulfonyl-phényl)-2-(tétrahydro-pyran-4-yloxy)-acétylamino]-thiazol-5-yloxy}-bénzoïque et ses énantiomères (+) et (-) ;
l'ester méthylique de l'acide 4-{2-[2-(4-cyclopropanesulfonyl-phényl)-2-(tétrahydro-pyran-4-yloxy)-acétylamino]-thiazol-5-yloxy}-2-fluoro-benzoïque et ses énantiomères (+) et (-) ;
l'ester méthylique de l'acide 4-{2-[2-(4-cyclopropanesulfonyl-phényl)-2-(tétrahydro-pyran-4-yloxy)-acétylamino]-thiazol-5-yloxy}-3-fluoro-benzoïque et ses énantiomères (+) et (-) ;
l'ester éthylique de l'acide {2-[2-(4-cyclopropanesulfonyl-phényl)-2-(tétrahydro-pyran-4-yloxy)-acétylamino]-thiazolo[5,4-b]pyridin-5-yloxy}-acétique et ses énantiomères (+) et (-) ;
l'ester méthylique de l'acide 4-{2-[2-(4-cyclopropanesulfonyl-phényl)-2-(tétrahydro-pyran-4-yloxy)-acétylamino]-thiazolo[5,4-b]pyridin-5-yloxy}-benzoïque et ses énantiomères (+) et (-) ;
le 2-(4-cyclopropanesulfonyl-phényl)-N-(6-méthoxy-benzothiazol-2-yl)-2-(tétrahydro-pyran-4-yloxy)-acétamide et ses énantiomères (+) et (-) ;
le 2-(4-cyclopropanesulfonyl-phényl)-N-(5-méthoxy-thiazolo[5,4-b]pyridin-2-yl)-2-[(R)-(tétrahydro-furan-3-yl)oxy]-acétamide et ses énantiomères (+) et (-) ;
l'ester tert-butylique de l'acide 4-[(4-cyclopropanesulfonyl-phényl)-(5-fluoro-thiazol-2-ylcarbamoyl)-méthoxy]-pipéridine-1-carboxylique et ses énantiomères (+) et (-) ;
le 2-(4-cyclopropanesulfonyl-phényl)-N-(5-fluoro-thiazol-2-yl)-2-(pipéridin-4-yloxy)-acétamide et ses énantiomères (+) et (-) ;
l'ester méthylique de l'acide (4-{2-[2-(4-cyclopropanesulfonyl-phényl)-2-(tétrahydro-pyran-4-yloxy)-acétylamino]-thiazol-5-yloxy}-phényl)-acétique et ses énantiomères (+) et (-) ;
l'ester méthylique de l'acide 2-[2-(4-cyclopropanesulfonyl-phényl)-2-(tétrahydro-pyran-4-yloxy)-acétylamino]-benzothiazole-6-carboxylique et ses énantiomères (+) et (-) ;
l'ester méthylique de l'acide 2-{2-(4-cyclopropanesulfonyl-phényl)-2-[(R)-(tétrahydro-furan-3-yl)oxy]-acétylamino}-benzothiazole-6-carboxylique et ses énantiomères (+) et (-) ;
le 2-(4-cyclopropanesulfonyl-phényl)-N-(5-oxo-4,5-dihydro-thiazolo[5,4-b]-pyridin-2-yl)-2-(tétrahydro-pyran-4-yloxy)-acétamide et ses énantiomères (+) et (-) ;
le 2-(4-cyclopropanesulfonyl-phényl)-N-(5-isopropoxy-thiazolo[5,4-b]pyridin-2-yl)-2-[(R)-(tétrahydro-furan-3-yl)oxy]-acétamide et ses énantiomères (+) et (-) ;
l'ester méthylique de l'acide 4-{2-[2-(4-cyclopropanesulfonyl-phényl)-2-(tétrahydro-pyran-4-yloxy)-acétylamino]-thiazol-5-yloxy}-2-méthyl-benzoïque et ses énantiomères (+) et (-) ;
l'ester méthylique de l'acide 2-[2-(4-cyclopropanesulfonyl-phényl)-2-(tétrahydro-pyran-4-yloxy)-acétylamino]-5-méthyl-thiazole-4-carboxylique et ses énantiomères (+) et (-) ;
le N-(5-bromo-thiazolo[5,4-b]pyridin-2-yl)-2-(4-cyclopropanesulfonyl-phényl)-2-(tétrahydro-pyran-4-yloxy)-acétamide et ses énantiomères (+) et (-) ;
le 2-(4-cyclopropanesulfonyl-phényl)-N-[5-(4-formyl-phénoxy)-thiazol-2-yl]-2-(tétrahydro-pyran-4-yloxy)-acétamide et ses énantiomères (+) et (-) ;
le 2-(4-cyclopropanesulfonyl-phényl)-2-(tétrahydro-pyran-4-yloxy)-N-(5-vinyl-thiazol-2-yl)-acétamide et ses énantiomères (+) et (-) ;
le 2-(4-cyclopropanesulfonyl-phényl)-2-(tétrahydro-pyran-4-yloxy)-N-(4-vinyl-thiazol-2-yl)-acétamide et ses énantiomères (+) et (-) ;
l'acide 4-{2-[2-(4-cyclopropanesulfonyl-phényl)-2-(tétrahydro-pyran-4-yloxy)-acétylamino]-thiazol-5-yloxy}-benzoïque et ses énantiomères (+) et (-) ;
l'acide 4-(2- {2-(4-cyclopropanesulfonyl-phényl)-2-[(R)-(tétrahydro-furan-3-yl)-oxy]-acétylamino}-thiazol-5-yloxy)-benzoïque et ses énantiomères (+) et (-) ;
l'acide {5-chloro-2-[2-(4-cyclopropanesulfonyl-phényl)-2-(tétrahydro-furan-3-yloxy)-acétylamino}-thiazol-4-yl)-acétique et ses énantiomères (+) et (-) ;
l'acide 4-{2-[2-(4-cyclopropanesulfonyl-phényl)-2-(tétrahydro-furan-3-yloxy)-acétylamino]-thiazol-5-yloxy}-benzoïque et ses énantiomères (+) et (-) ;
l'acide (5-chloro-2-{2-(4-cyclopropanesulfonyl-phényl)-2-[(R)-(tétrahydro-furan-3-yl)oxy]-acétylamino}-thiazol-4-yl)-acétique et ses énantiomères (+) et (-) ;
l'acide 4-(2-{2-(4-cyclopropanesulfonyl-phényl)-2-[(S)-(tétrahydro-furan-3-yl)-oxy]-acétylamino}-thiazol-5-yloxy)-benzoïque et ses énantiomères (+) et (-) ;
l'acide (5-chloro-2-{2-(4-cyclopropanesulfonyl-phényl)-2-[(S)-(tétrahydro-furan-3-yl)oxy]-acétylamino}-thiazol-4-yl)-acétique et ses énantiomères (+) et (-) ;
l'acide 4-{3-[2-(4-cyclopropanesulfonyl-phényl)-2-(tétrahydro-pyran-4-yloxy)-acétylamino]-pyrazol-1-ylméthyl}-benzoïque et ses énantiomères (+) et (-) ;
l'acide {5-chloro-2-[2-(4-cyclopropanesulfonyl-phényl)-2-(tétrahydro-pyran-4-yloxy)-acétylamino]-thiazol-4-yl}-acétique et ses énantiomères (+) et (-) ;
l'acide 3-{2-[2-(4-cyclopropanesulfonyl-phényl)-2-(tétrahydro-pyran-4-yloxy)-acétylamino]-thiazol-5-yloxy}-benzoïque et ses énantiomères (+) et (-) ;
l'acide 4-{2-[2-(4-cyclopropanesulfonyl-phényl)-2-(tétrahydro-pyran-4-yloxy)-acétylamino]-thiazol-5-yloxy}-2-fluoro-benzoïque et ses énantiomères (+) et (-) ;
l'acide 4-{2-[2-(4-cyclopropanesulfonyl-phényl)-2-(tétrahydro-pyran-4-yloxy)-acétylamino]-thiazol-5-yloxy}-3-fluoro-benzoïque et ses énantiomères (+) et (-) ;
l'acide {2-[2-(4-cyclopropanesulfonyl-phényl)-2-(tétrahydro-pyran-4-yloxy)-acétylamino]-thiazolo[5,4-b]pyridin-5-yloxy}-acétique et ses énantiomères (+) et (-) ;
l'acide 4-{2-[2-(4-cyclopropanesulfonyl-phényl)-2-(tétrahydro-pyran-4-yloxy)-acétylamino]-thiazolo[5,4-b]pyridin-5-yloxy}-benzoïque et ses énantiomères (+) et (-) ;
l'ester tert-butylique de l'acide 4-[(4-carboxyméthyl-5-chloro-thiazol-2-ylcarbamoyl)-(4-cyclopropanesulfonyl-phényl)-méthoxy]-pipéridine-1-carboxylique et ses énantiomères (+) et (-) ;
l'acide {5-chloro-2-[2-(4-cyclopropanesulfonyl-phényl)-2-(pipéridin-4-yloxy)-acétylamino]-thiazol-4-yl}-acétique et ses énantiomères (+) et (-) ;
l'acide (4-{2-[2-(4-cyclopropanesulfonyl-phényl)-2-(tétrahydro-pyran-4-yloxy)-acétylamino]-thiazol-5-yloxy}-phényl)-acétique et ses énantiomères (+) et (-) ;
l'acide 2-[2-(4-cyclopropanesulfonyl-phényl)-2-(tétrahydro-pyran-4-yloxy)-acétylamino]-benzothiazole-6-carboxylique et ses énantiomères (+) et (-) ;
l'acide 2-{2-(4-cyclopropanesulfonyl-phényl)-2-[(R)-(tétrahydro-furan-3-yl)oxy]-acétylamino}-benzothiazole-6-carboxylique et ses énantiomères (+) et (-) ;
l'acide 4-{2-[2-(4-cyclopropanesulfonyl-phényl)-2-(tétrahydro-pyran-4-yloxy)-acétylamino]-thiazol-5-yloxy}-2-méthyl-benzoïque et ses énantiomères (+) et (-) ;
l'acide 2-[2-(4-cyclopropanesulfonyl-phényl)-2-(tétrahydro-pyran-4-yloxy)-acétylamino]-5-méthyl-thiazole-4-carboxylique et ses énantiomères (+) et (-) ;
le N-{5-[4-(azétidine-1-carbonyl)-phénoxy]-thiazol-2-yl}-2-(4-cyclopropane-sulfonyl-phényl)-2-(tétrahydro-pyran-4-yloxy)-acétamide et ses énantiomères (+) et (-) ;
le 4-{2-[2-(4-cyclopropanesulfonyl-phényl)-2-(tétrahydro-furan-3-yloxy)-acétylamino]-thiazol-5-yloxy}-benzamide et ses énantiomères (+) et (-) ;
le N-[4-(2-azétidin-1-yl-2-oxo-éthyl)-5-chloro-thiazol-2-yl]-2-(4-cyclopropane-sulfonyl-phényl)-2-(tétrahydro-pyran-4-yloxy)-acétamide et ses énantiomères (+) et (-) ;
le 2-(4-cyclopropanesulfonyl-phényl)-2-(tétrahydro-pyran-4-yloxy)-N-{5-[4-(2H-tétrazol-5-yl)-phénoxy]-thiazol-2-yl}-acétamide et ses énantiomères (+) et (-) ;
le 2-(4-cyclopropanesulfonyl-phényl)-N-[5-(4-hydroxyméthyl-phénoxy)-thiazol-2-yl]-2-(tétrahydro-pyran-4-yloxy)-acétamide et ses énantiomères (+) et (-) ;
le 2-(4-cyclopropanesulfonyl-phényl)-N-(4-hydroxyméthyl-5-méthyl-thiazol-2-yl)-2-(tétrahydro-pyran-4-yloxy)-acétamide et ses énantiomères (+) et (-) ;
le 2-(4-cyclopropanesulfonyl-phényl)-N-[5-(1,2-dihydroxy-éthyl)-thiazol-2-yl]-2-(tétrahydro-pyran-4-yloxy)-acétamide et ses énantiomères (+) et (-) ;
le 2-(4-cyclopropanesulfonyl-phényl)-N-[5-(1,2-dihydroxy-éthyl)-thiazolo[5,4-b]-pyridin-2-yl]-2-(tétrahydro-pyran-4-yloxy)-acétamide et ses énantiomères (+) et (-) ;
le 2-(4-cyclopropanesulfonyl-phényl)-N-[4-(1,2-dihydroxy-éthyl)-thiazol-2-yl]-2-(tétrahydro-pyran-4-yloxy)-acétamide et ses énantiomères (+) et (-) ;
le 2-(4-cyclopropanesulfonyl-phényl)-N-[3-(1,2-dihydroxy-éthyl)-[1,2,4]-thiadiazol-5-yl]-2-(tétrahydro-pyran-4-yloxy)-acétamide et ses énantiomères (+) et (-) ;
l'acide {5-chloro-2-[2-(4-cyclopropanesulfonyl-phényl)-2-(2-méthyl-tétrahydro-pyran-3-yloxy)-acétylamino]-thiazol-4-yl}-acétique et ses énantiomères (+) et (-) ;
l'acide 2-[2-[4-(3-oxo-cyclopentanesulfonyl)-phényl]-2-(tétrahydro-furan-3-yloxy)-acétylamino]-thiazole-4-carboxylique et ses énantiomères (+) et (-) ;
le N- 4-(1-acétyl-pipéridin-4-yl)-thiazol-2-yl]-2-(4-cyclopropanesulfonyl-phényl)-2-(tétrahydro-pyran-4-yloxy)-acétamide et ses énantiomères (+) et (-) ;
le 2-(4-cyclopropanesulfonyl-phényl)-N-{5-[4-(1,2-dihydroxy-éthyl)-phénoxy]-thiazol-2-yl}-2-(tétrahydro-pyran-4-yloxy)-acétamide et ses énantiomères (+) et (-) ;
le 2-(4-cyclopropanesulfonyl-phényl)-N-(4,5,6,7-tétrahydro-benzothiazol-2-yl)-2-(tétrahydro-pyran-4-yloxy)-acétamide et ses énantiomères (+) et (-) ;
le 2-(4-cyclopropanesulfonyl-phényl)-2-(tétrahydro-pyran-4-yloxy)-N-thiazolo-[5,4-d]pyrimidin-2-yl-acétamide et ses énantiomères (+) et (-) ;
le 2-(4-cyclopropanesulfonyl-phényl)-N-[5-(4-hydroxyméthyl-phénoxy)-thiazol-2-yl]-2-(tétrahydro-pyran-4-yloxy)-acétamide et ses énantiomères (+) et (-) ;
le 2-(4-cyclopropanesulfonyl-phényl)-N-[4-(1,2-dihydroxy-éthyl)-thiazol-2-yl]-2-(tétrahydro-pyran-4-yloxy)-acétamide et ses énantiomères (+) et (-) ;
le 2-(4-cyclopropanesulfonyl-phényl)-N-[4-(1,2-dihydroxy-éthyl)-5-fluoro-thiazol-2-yl]-2-(tétrahydro-pyran-4-yloxy)-acétamide et ses énantiomères (+) et (-) ;
le 2-(4-cyclopropanesulfonyl-phényl)-N-[5-(1,2-dihydroxy-éthyl)-thiazol-2-yl]-2-(tétrahydro-pyran-4-yloxy)-acétamide et ses énantiomères (+) et (-) ;
le 2-(4-cyclopropanesulfonyl-phényl)-N-[5-(1,2-dihydroxy-éthyl)-thiazolo[5,4-b]-pyridin-2-yl]-2-(tétrahydro-pyran-4-yloxy)-acétamide et ses énantiomères (+) et (-) ;
l'acide 2-(3-{2-(4-cyclopropanesulfonyl-phényl)-2-[(R)-(tétrahydro-furan-3-yl)-oxy]-acétylamino}-pyrazol-1-yl)-2-méthyl-propionique et ses énantiomères (+) et (-).

6. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans le traitement d'une maladie par l'intermédiaire de l'activation de la glucokinase.

7. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans le traitement d'une maladie par l'intermédiaire de la désinhibition de la glucokinase.

8. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans le traitement prophylactique ou thérapeutique de l'hyperglycémie ou du diabète, particulièrement du diabète de type II.

9. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans la prévention du diabète, particulièrement du diabète de type II, chez un être humain démontrant une hyperglycémie prédiabétique ou une altération de la tolérance au glucose.

10. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans un traitement combiné ou la prévention du diabète et de l'obésité.

11. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans le traitement ou la prévention de l'obésité.

12. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans le traitement ou la prévention d'une dyslipidémie.

13. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans le traitement de l'hyperglycémie, du TAG, du syndrome X, du diabète de type 2, du diabète de type 1, d'une dyslipidémie ou d'une hyperlipidémie, de l'hypertension, pour une utilisation dans le traitement ou la prophylaxie de l'obésité, pour une utilisation dans la diminution de la prise d'aliments, pour une utilisation dans la régulation de l'appétit ; et pour une utilisation dans la régulation du comportement alimentaire.

14. Composé de formule (I), tel que revendiqué dans l'une quelconque des revendications 1 à 5, ou ses stéréoisomères, tautomères, sels pharmaceutiquement acceptables, polymorphes, solvates et formulations, pour une utilisation dans l'amplification de la sécrétion des entéro-incrétines, comme le GLP-1 et le GIP, gérant de cette façon des maladies ou des troubles associés à la modulation des sécrétions des entéro-incrétines, comme l'hyperglycémie, la résistance à l'insuline, l'altération de la tolérance au glucose, l'obésité, la vidange gastrique, la gastroparésie, la satiété, la résistance aux leptines, une dyslipidémie, la cicatrisation, les complications diabétiques, telles que la néphropathie, la rétinopathie, la neuropathie et la cataracte.

15. Composition pharmaceutique comprenant, en tant que principe actif, au moins un composé de formule (I), tel que revendiqué dans l'une quelconque des revendications 1 à 5, ou ses stéréoisomères, tautomères, sels pharmaceutiquement acceptables, polymorphes, et solvates, conjointement avec un ou plusieurs supports ou excipients pharmaceutiquement acceptables.

16. Composition pharmaceutique comprenant, en tant que principe actif, au moins un composé de formule (I), tel que revendiqué dans l'une quelconque des revendications 1 à 5, ou ses stéréoisomères, tautomères, sels pharmaceutiquement acceptables, polymorphes, et solvates, en combinaison avec un ou plusieurs agents thérapeutiquement actifs pharmaceutiquement acceptables.

17. Composition pharmaceutique telle que revendiquée dans la revendication 16 dans laquelle, l'agent thérapeutiquement actif pharmaceutiquement acceptable est choisi parmi des agents antidiabétiques, des agents antihyperglycémiques, des agents anti-obésité, des agents antihypertenseurs ou des agents antidyslipidémiques.

18. Composition pharmaceutique telle que revendiquée dans la revendication 16 ou la revendication 17 dans laquelle l'agent thérapeutiquement actif pharmaceutiquement acceptable est choisi parmi des sécrétagogues de l'insuline comme les sulfonylurées choisies parmi Amaryl, le glyburide, le glimépiride, le glipyride, le glipizide ; des ligands des récepteurs de sulfonylurées insulinotropes comme les méglitinides choisis parmi le natéglinide, le répaglinide ; des biguanides comme la metformine, la phenformine, la buformine ; des antagonistes du glucagon comme un antagoniste peptidique ou non peptidique du glucagon ; des inhibiteurs de la glucosidase comme l'acarbose, le miglitol ; des agents insulinotropes sensibles au glucose comme le GLP-1, des mimétiques du GLP-1 comme l'exendine 4 ; les sensibilisateurs à l'insuline comme la troglitazone, la rosiglitazone, la pioglitazone ; les inhibiteurs de la dipeptidyl-peptidase-IV comme la sitagliptine, la vildagliptine ; la sibutramine, l'orlistat, le rimonabant ; des fibrates comme le gemfibrozil, le fénofibrate ; la niacine ; des statines comme la rosuvastatine, l'atorvastatine, la simvastatine ; des inhibiteurs de l'absorption de cholestérol comme l'ézétimibe ; des séquestrants d'acides biliaires comme la cholestyramine ; des diurétiques comme les hydrochlorothiazides, le mannitol, l'indapamide, le furosémide ; des inhibiteurs de l'enzyme de conversion de l'angiotensine (ECA) comme le captopril, l'énalapril ; des antagonistes du récepteur de l'angiotensine II de type I (ARA) comme le losartan, l'irbésartan ; des inhibiteurs de la rénine comme l'aliskiren ; des inhibiteurs des récepteurs β-adrénergiques comme l'aténolol, le métoprolol ; des inhibiteurs des canaux calciques comme l'amlodipine, la nifédipine ; des antagonistes du récepteur de l'aldostérone comme la spironolactone, des inhibiteurs de l'aldostérone synthase comme FAD286.

19. Utilisation d'un composé de formule (I) tel que revendiqué dans l'une quelconque des revendications 1 à 5, de ses polymorphes, stéréoisomères, sels pharmaceutiquement acceptables, ou solvates, pour la fabrication d'un médicament destiné à l'activation de la glucokinase.

20. Utilisation d'un composé de formule (I) tel que revendiqué dans l'une quelconque des revendications 1 à 5, de ses polymorphes, stéréoisomères, sels pharmaceutiquement acceptables, ou solvates, pour la fabrication d'un médicament destiné au traitement prophylactique ou thérapeutique de l'obésité.

21. Utilisation d'un composé de formule (I) tel que revendiqué dans l'une quelconque des revendications 1 à 5, de ses polymorphes, stéréoisomères, sels pharmaceutiquement acceptables, ou solvates, pour la fabrication d'un médicament destiné au traitement prophylactique ou thérapeutique de l'hyperglycémie ou du diabète, particulièrement du diabète de type II.

22. Utilisation d'un composé de formule (I) tel que revendiqué dans l'une quelconque des revendications 1 à 5, de ses polymorphes, stéréoisomères, sels pharmaceutiquement acceptables, ou solvates, pour la fabrication d'un médicament destiné à la prévention du diabète, particulièrement du diabète de type II, chez un être humain démontrant une hyperglycémie prédiabétique ou une altération de la tolérance au glucose.

23. Procédé de préparation d'un composé de formule (I) tel que revendiqué dans l'une quelconque des revendications 1 à 5 ou de ses stéréoisomères, tautomères, sels pharmaceutiquement acceptables, polymorphes, et solvates, ledit procédé comprenant :
la réaction d'un acide de formule (II) 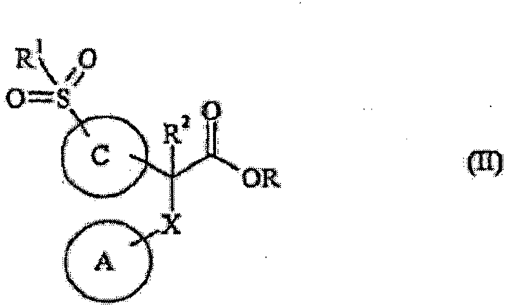
dans laquelle R représente un atome d'hydrogène, un groupe alkyle ou arylalkyle, avec un composé de formule (III) 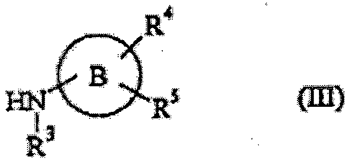
en présence d'un réactif de couplage d'amides approprié, éventuellement une hydrolyse et éventuellement un autre couplage avec une amine de formule NHR⁶R⁷ pour obtenir le composé de formule (I).
